# FASCICULE DE BREVET EUROPEEN

(11) **EP 0 454 511 B1**
(45) Date de publication et mention de la délivrance du brevet: **17.06.1998**
(21) Numéro de dépôt: 91400745.5
(22) Date de dépôt: 20.03.1991
(51) Int. Cl.: C07D 235/02, A61K 31/415, C07D 233/70, C07D 233/96, C07D 239/70, C07D 403/10, C07D 417/12, C07D 471/10, C07D 491/10, A61K 31/505

(54) **Dérivés hétérocycliques N-substitués leur préparation, les compositions pharmaceutiques en contenant**
N-substituierte heterozyklische Derivate, Verfahren zu deren Herstellung, diese enthaltende Zusammensetzungen
N-substituted heterocycle derivatives, their preparation, compositions containing them

(30) Priorité: 20.03.1990 FR 9003563; 08.08.1990 FR 9010144
(43) Date de publication de la demande: 30.10.1991
(73) Titulaire: SANOFI, 75008 Paris (FR)
(72) Inventeur: Bernhart, Claude, F-34980 Saint Gely du Fesc (FR); Breliere, Jean-Claude, F-34090 Montpellier (FR); Clement, Jacques, F-34570 Saussan (FR); Nisato, Dino, F-34680 Saint Georges d'Orques (FR); Perreaut, Pierre, "Les collines d'Estanove", F-34100 - Montpellier (FR)
(74) Mandataire: Gillard, Marie-Louise

(56) Documents cités:
- EP-A- 144 748
- EP-A- 226 947
- EP-A- 253 310
- EP-A- 291 969
- EP-A- 323 841
- EP-A- 324 377
- EP-A- 407 342
- EP-A- 411 507
- EP-A- 412 594
- EP-A- 419 048

## Description

La présente invention concerne des dérivés hétérocycliques N-subtitués, leur préparation et les compositions pharmaceutiques en contenant.

Les composés selon l'invention antagonisent l'action de l'angiotensine II qui est une hormone peptidique de formule :

H-Asp-Arg-Val-Tyr-Ile-His-Pro-Phe-OH

L'angiotensine II est un puissant agent vasopresseur qui est le produit biologiquement actif du système rénine-angiotensine : la rénine agit sur l'angiotensinogène du plasma pour produire l'angiotensine I, celle-ci est convertie en angiotensine II par action de l'enzyme de conversion de l'angiotensine I.

Les composés de la présente invention sont des composés non peptidiques, antagonistes de l'angiotensine II. En inhibant l'action de l'angiotensine II sur ses récepteurs, les composés selon l'invention empêchent notamment l'augmentation de la pression sanguine produite par l'intéraction hormone-récepteur, ils ont également d'autres actions physiologiques au niveau du système nerveux central.

Ainsi les composés selon l'invention sont utiles dans le traitement d'affections cardiovasculaires comme l'hypertension, la défaillance cardiaque ainsi que dans le traitement d'affections du système nerveux central et dans le traitement du glaucome et de la rétinopathie diabétique.

La présente invention a pour objet des composés de formule : 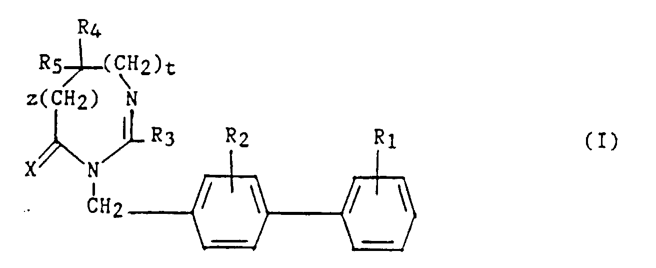 dans laquelle :
- R₁ et R₂ sont semblables ou différents et représentent chacun indépendamment l'hydrogène ou un groupe choisi parmi un alkyle en C₁-C₆, un alcoxy en C₁-C₄, un amino, un aminométhyle, un carboxy, un alcoxycarbonyle dans lequel l'alcoxy est en C₁-C₄, un cyano, un tétrazolyle, un méthyltétrazolyle, un méthylsulfonylamino, un trifluorométhylsulfonylamino, un trifluorométhylsulfonylaminométhyle, un N-cyanocarbamoyl, un N-hydroxycarbamoyl, un N-((carboxy-4) thiazol-1,3-yl-2)-carbamoyl, un uréido, un cyano-2 guanidinocarbonyle, un cyano-2 guanidinométhyle, un imidazol-1-yl-carbonyle, un cyano-3 méthyl-2 isothiouréidométhyle, à la condition qu'au moins l'un des substituants R₁ ou R₂ soit différent de l'hydrogène ;
- R₃ représente un hydrogène, un alkyle en C₁-C₆, non substitué ou substitué par un ou plusieurs atomes d'halogène, un alcényle en C₂-C₆, un cycloalkyle en C₃-C₇, un phényle, un phénylalkyle dans lequel l'alkyle est en C₁-C₃, un phénylalcényle dans lequel l'alcényle est en C₂-C₃, lesdits groupes phényles étant non substitués ou substitués une ou plusieurs fois par un atome d'halogène, un alkyle en C₁-C₄, un halogénoalkyle en C₁-C₄, un polyhalogénoalkyle en C₁-C₄, un hydroxyle ou un alcoxy en C₁-C₄ ;
- R₄ et R₅ représentent chacun indépendamment un alkyle en C₁-C₆, un phényle, un phénylalkyle dans lequel l'alkyle est en C₁-C₃, lesdits groupes alkyle, phényle et phénylalkyle étant non substitués ou substitués par un ou plusieurs atomes d'halogène ou par un groupe choisi parmi un perfluoroalkyle en C₁-C₄, un hydroxyle, un alcoxy en C₁-C₄ ;
- ou R₄ et R₅ ensemble forment un groupe de formule =CR₇R₈, dans laquelle R₇ représente l'hydrogène, un alkyle en C₁-C₄ ou un phényle, et R₈ représente un alkyle en C₁-C₄ ou un phényle ;
- ou encore R₄ et R₅ liés ensemble représentent, soit un groupe de formule (CH₂)ₙ, soit un groupe de formule (CH₂)ₚY(CH₂)_{q}, dans lequel Y est, soit un atome d'oxygéné, soit un atome de soufre, soit un atome de carbone substitué par un groupe alkyle en C₁-C₄, un phényle ou un phénylalkyle dans lequel l'alkyle est en C₁-C₃, soit un groupe N-R₆ dans lequel R₆ représente un hydrogène, un alkyle en C₁-C₄, un phénylalkyle dans lequel l'alkyle est en C₁-C₃, un alkylcarbonyle en C₁-C₄, un halogénoalkylcarbonyle en C₁-C₄, un polyhalogénoalkylcarbonyle en C₁-C₄, un benzoyle, un alpha aminoacyle ou un groupe N-protecteur, ou R₄ et R₅ liés ensemble avec l'atome de carbone auquel ils sont liés constituent un indane ou un adamantane ;
- p + q = m ;
- n est un nombre entier compris entre 2 et 11 ;
- m est un nombre entier compris entre 2 et 5 ;
- X représente un atome d'oxygène ou atome de soufre ;
- z et t sont nuls ou l'un est nul et l'autre représente un ;
et leurs sels.

Lorsqu'un composé selon l'invention présente un carbone asymétrique, l'invention comprend les 2 isomères optiques de ce composé.

Les sels des composés de formule (I) selon la présente invention comprennent ceux avec des acides minéraux ou organiques qui permettent une séparation ou une cristallisation convenable des composés de formule (I), tels que l'acide picrique, l'acide oxalique ou un acide optiquement actif, par exemple un acide mandélique ou un acide camphosulfonique, et ceux qui forment des sels pharmaceutiquement acceptables tels que le chlorhydrate, le bromhydrate, le sulfate, l'hydrogénosulfate, le dihydrogénophosphate, le méthanesulfonate, le méthylsulfate, le maléate, le fumarate, le 2-naphtalènesulfonate.

Les sels des composés de formule (I) comprennent également les sels avec des bases organiques ou minérales, par exemple les sels des métaux alcalins ou alcalino-terreux comme les sels de sodium, de potassium, de calcium, les sels de sodium et de potassium étant préférés, ou avec une amine tertiaire, telle que le trométamol, ou bien les sels d'arginine, de lysine, ou de toute amine physiologiquement acceptable.

Selon la présente description et dans les revendications qui vont suivre, par atome d'halogène on entend un atome de brome, de chlore ou de fluor ; par groupe N-protecteur (également désigné par Pr) on entend un groupe utilisé classiquement dans la chimie des peptides pour permettre une protection temporaire de la fonction amine, par exemple un groupe Boc, Z, Fmoc ou un groupe benzyle; par groupe carboxy estérifié on entend un ester labile dans des conditions appropriées, comme par exemple un ester méthylique, éthylique, benzylique ou tertiobutylique. Par "alkyle" on désigne les restes d'hydrocarbures aliphatiques saturés, droits ou ramifiés.

Les composés de formule I dans laquelle R₁ est en ortho et représente un groupe carboxy ou tétrazolyle et R₂ est l'hydrogène sont des composés préférés.

Les composés de formule (I) dans lesquels R₄ et R₅ liés ensemble constituent avec le carbone auquel ils sont liés un cyclopentane ou un cyclohexane sont des composés préférés.

De même, les composés de formule (I) dans lesquels R₃ représente un groupe alkyle droit en C₁-C₆ sont des composés préférés.

Les composés de formule (I) dans lesquels X représente un atome d'oxygène sont également des composés préférés.

Enfin les composés de formule (I) dans lesquels z = t = 0 sont des composés préférés.

Les abréviations suivantes sont utilisées dans la description et dans les exemples :
- Et :: Ethyl
- nBu, tBu :: n-butyl, *tert*-butyl
- DMF :: diméthylformamide
- THF :: tétrahydrofuranne
- DCM :: dichlorométhane
- NBS :: N-bromo-succinimide
- DCC :: dicyclohexylcarbodiimide
- DIPEA :: diisopropyléthylamine
- Ether :: éther éthylique
- TFA :: acide trifluoroacétique
- Z :: benzyloxycarbonyle
- Boc :: *tert*-butoxycarbonyle
- BOP :: hexafluorophosphate de benzotriazolyloxy trisdiméthylamino phosphonium
- Fmoc :: fluorénylméthyloxycarbonyle

La présente invention a également pour objet le procédé de préparation des composés (I). Ledit procédé est caractérisé en ce que :
a1) on fait réagir un dérivé hétérocyclique de formule : 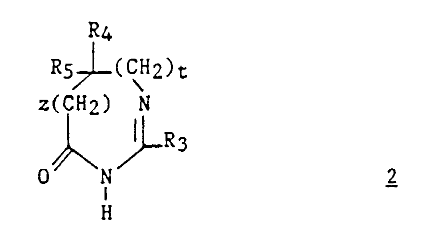 dans laquelle z, t, R₃, R₄ et R₅ ont les significations indiquées ci-dessus pour (I), sur un dérivé du (biphényl-4-yl) méthyl de formule : 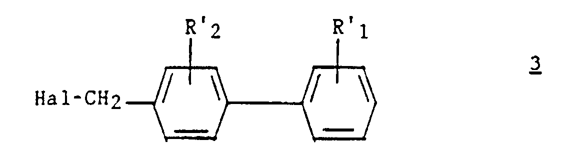 dans laquelle Hal représente un atome d'halogène et R'₁ et R'₂ représentent respectivement soit R₁ et R₂, soit un groupement précurseur de R₁ et R_{2 ;}
b1) éventuellement, le composé ainsi obtenu de formule : 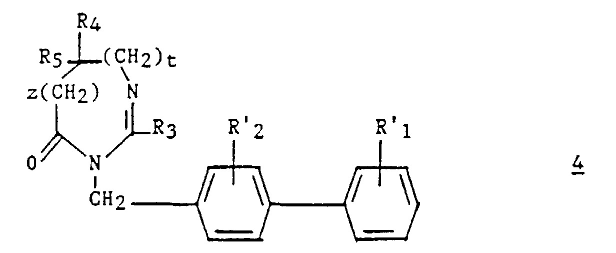 est traité par le réactif de Lawesson [bis(méthoxy-4 phényl)-2,4 dithia-1,3 diphosphétane-2,4 disulfure 2,4] ;
c1) le composé obtenu en a1) ou en b1), de formule : 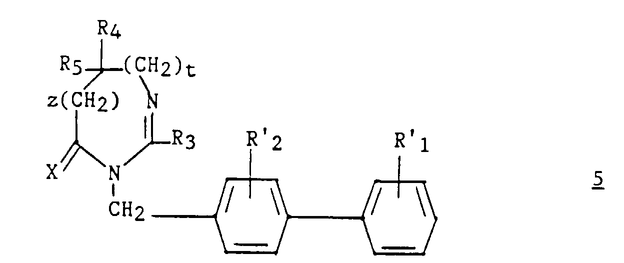 dans laquelle X représente un atome d'oxygène ou un atome de soufre, est traité pour préparer le composé (I) par transformation des groupes R'₁ et/ou R'₂ en respectivement, les groupes R₁ et/ou R₂.

Parmi les composés 2, les composés (II) tels que définis ci-dessous sont nouveaux.

Ainsi la présente invention a également pour objet les composés (II) de formule : 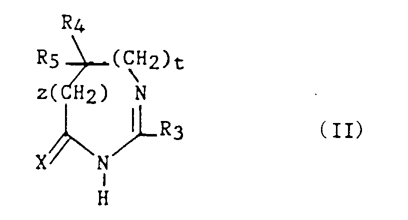 dans laquelle :
- R₃ représente un hydrogène, un alkyle en C₁-C₆, non substitué ou substitué par un ou plusieurs atomes d'halogène, un alcényle en C₂-C₆, un cycloalkyle en C₃-C₇, un phényle, un phénylalkyle dans lequel l'alkyle est en C₁-C₃, un phénylalcényle dans lequel l'alcényle est en C₂-C₃, lesdits groupes phényles étant non substitués ou substitués une ou plusieurs fois par un atome d'halogène, un alkyle en C₁-C₄, un halogénoalkyle en C₁-C₄, un polyhalogénoalkyle en C₁-C₄, un hydroxyle ou un alcoxy en C₁-C₄ ;
- R₄ et R₅ représentent chacun indépendamment un alkyle en C₁-C₆, un phényle, un phénylalkyle dans lequel l'alkyle est en C₁-C₃, lesdits groupes alkyle, phényle et phénylalkyle étant non substitués ou substitués par un ou plusieurs atomes d'halogène ou par un groupe choisi parmi un perfluoroalkyle en C₁-C₄, un hydroxyle, un alcoxy en C₁-C₄ ;
- ou R₄ et R₅ ensemble forment un groupe de formule =CR₇R₈, dans laquelle R₇ représente l'hydrogène, un alkyle en C₁-C₄ ou un phényle, et R₈ représente un alkyle en C₁-C₄ ou un phényle ;
- ou encore R₄ et R₅ liés ensemble représentent, soit un groupe de formule (CH₂)ₙ, soit un groupe de formule (CH₂)ₚY(CH₂)_{q}, dans lequel Y est, soit un atome d'oxygène, soit un atome de soufre, soit un atome de carbone substitué par un groupe alkyle en C₁-C₄, un phényle ou un phénylalkyle dans lequel l'alkyle est en C₁-C₃, soit un groupe N-R₆ dans lequel R₆ représente un hydrogène, un alkyle en C₁-C₄, un phénylalkyle dans lequel l'alkyle est en C₁-C₃, un alkylcarbonyle en C₁-C₄, un halogénoalkylcarbonyle en C₁-C₄, un polyhalogénoalkylcarbonyle en C₁-C₄, un benzoyle, un alpha aminoacyle ou un groupe N-protecteur, ou R₄ et R₅ liés ensemble avec l'atome de carbone auquel ils sont liés constituent un indane ou un adamantane ;
- p + q = m ;
- n est un nombre entier compris entre 2 et 11 ;
- m est un nombre entier compris entre 2 et 5 ;
- X représente un atome d'oxygène ou atome de soufre ;
- z et t sont nuls ou l'un est nul et l'autre représente un ;
   avec la limitation que
   A/ lorsque z et t sont nuls et X représente l'oxygène :
      1) R₄ et R₅ sont autres qu'un alkyle en C₁-C₆, un phényle, un phénylalkyle dans lequel l'alkyle est en C₁-C₃, lesdits groupes alkyle, phényle et phénylalkyle étant non substitués ou substitués par un ou plusieurs atomes d'halogène ou par un groupe choisi parmi un perfluoroalkyle en C₁-C₄, un hydroxyle, un alcoxy en C₁-C₄ ; ou
      2) R₄ et R₅ liés ensemble sont autres qu'un groupe de formule (CH₂)ₚY(CH₂)_{q} dans lequel Y est un groupe N-R₆ dans lequel R₆ est un hydrogène, un alkyle en C₁-C₄, un phénylalkyle dans lequel l'alkyle est en C₁-C₃ ; ou
      3) R₄ et R₅ ensemble sont différents du groupe = CR₇R₈ lorsque R₃ est un hydrogène, un phényle, un méthylphényle ou un benzyle ;
      4) n est différent de 6 ; ou bien
      5) R₄ et R₅ liés ensemble sont autres qu'un groupe (CH₂)ₙ dans lequel n est compris entre 3 et 5, lorsque R₃ représente un groupe phényle substitué ;
   B/ lorsque z = 1 et R₃ est un phényle, R₄ et R₅ sont chacun différent d'un méthyle ;
   C/ lorsque z et t sont nuls et X est le soufre :
      1) R₄ et R₅ sont autres qu'un phényle lorsque R₃ est un méthyle, un phényle, un chlorophényle, un méthoxyphényle ou un méthylphényle ; ou
      2) R₄ et R₅ sont autres qu'un alkyle en C₁-C₃ lorsque R₃ est un méthyle ou un phényle; ou
      3) R₄ et R₅ sont autres qu'un méthoxyphényle ou chlorophényle lorsque R₃ est phényle ; ou
      4) R₄ et R₅ sont autres qu'un chlorophényle lorsque R₃ est un méthyle.

Parmi les dérivés (II), les composés dans lesquels z = t = 0 et R₄ et R₅, ensemble avec le carbone auquel ils sont liés, constituent un cyclopentane, sont des composés préférés. Ces composés répondent à la formule : 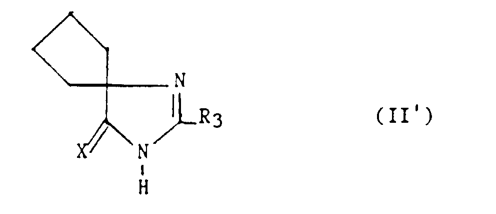 dans laquelle X représente un atome d'oxygène ou un atome de soufre et R₃ représente un hydrogène, un alkyle en C₁-C₆, non substitué ou substitué par un ou plusieurs atomes d'halogène, un alcényle en C₂-C₆, un cycloalkyle en C₃-C₇, un phényle, un phénylalkyle dans lequel l'alkyle est en C₁-C₃, un phénylalcényle dans lequel l'alcényle est en C₂-C₃, lesdits groupes phényles étant non substitués ou substitués une ou plusieurs fois par un atome d'halogène, un alkyle en C₁-C₄, un halogénoalkyle en C₁-C₄, un polyhalogénoalkyle en C₁-C₄, un hydroxyle ou un alcoxy en C₁-C₄.

Les composés (II) dans lesquels z = 0 et t = 1 de formule : 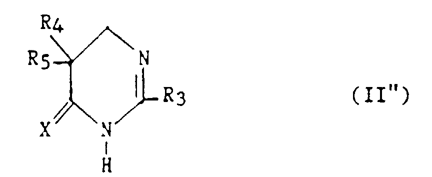 dans laquelle R₃, R₄, R₅ et X ont les définitions données ci-dessus pour (II) sont des composés préférés.

Enfin, les composés (II) dans lesquels z = 1 et t = 0 de formule : 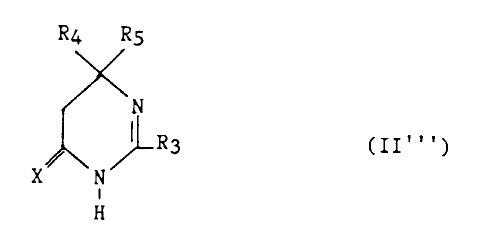 dans laquelle :
- R₃ représente un hydrogène, un alkyle en C₁-C₆, non substitué ou substitué par un ou plusieurs atomes d'halogène, un alcényle en C₂-C₆, un cycloalkyle en C₃-C₇, un phényle, un phénylalkyle dans lequel l'alkyle en C₁-C₃, un phénylalcényle dans lequel l'alcényle est en C₂-C₃, lesdits groupes phényles étant non substitués ou substitués une ou plusieurs fois par un atome d'halogène, un alkyle en C₁-C₄, un halogénoalkyle en C₁-C₄, un polyhalogénoalkyle en C₁-C₄, un hydroxyle ou un alcoxy en C₁-C₄ ;
- R₄ et R₅ représentent chacun indépendemment un alkyle en C₁-C₆, un phényle, un phénylalkyle dans lequel l'alkyle est C₁-C₃, lesdits groupes alkyle, phényle et phénylalkyle étant non substitués ou substitués par un ou plusieurs atomes d'halogène ou par un groupe choisi parmi un perfluoroalkyle en C₁-C₄, un hydroxyle, un alcoxy en C₁-C₄ ;
- ou R₄ et R₅ ensemble forment un groupe de formule =CR₇R₈, dans laquelle R₇ représente l'hydrogène, un alkyle en C₁-C₄ ou un phényle, et R₈ représente un alkyle en C₁-C₄ ou un phényle ;
- ou R₄ et R₅ liés ensemble représentent, soit un groupe de formule (CH₂)ₙ, soit un groupe de formule (CH₂)ₚY(CH₂)q, dans lequel Y est, soit un atome d'oxygène, soit un atome de soufre, soit un atome de carbone substitué par un groupe alkyle en C₁-C₄, un phényle ou phénylalkyle dans lequel l'alkyle est en C₁-C₃, soit un groupe N-R₆ dans lequel R₆ représente un hydrogène, un alkyle en C₁-C₄, un phénylalkyle dans lequel l'alkyle est en C₁-C₄, un alkylcarbonyle en C₁-C₄, un halogénoalkylcarbonyle en C₁-C₄, un polyhalogénoalkylcarbonyle en C₁-C₄, un benzoyle, un alpha aminoacyle ou un groupe N-protecteur, ou R₄ et R₅ liés ensemble avec l'atome de carbone auquel ils sont liés constituent un indane ou un adamantane ;
- p + q = m ;
- n est un nombre entier compris entre 2 et 11 ;
- m est un nombre entier compris entre 2 et 5 ;
- X représente un atome d'oxygène ou un atome de soufre ; avec la limitation que R₃ est autre qu'un phényle lorsque R₄ et R₅ représentent chacun un méthyle ;
sont des composés préférés.

Les dérivés 2 sont préparés par des méthodes connues. Par exemple, on peut utiliser la méthode décrite par Jacquier et al.(Bull. Soc. Chim. France, 1971, 3, 1040-1051) et par Brunken et Bach (Chem. Ber., 1956, 89, 1363-1373) et faire agir un imidate d'alkyle sur un acide aminé ou son ester, selon le schéma réactionnel suivant : 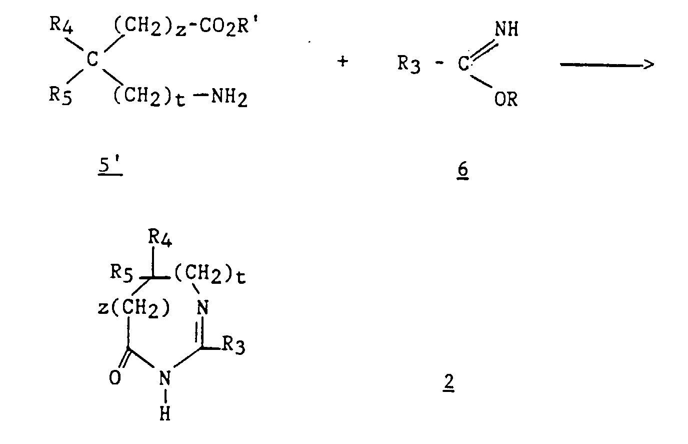 dans lequel R représente un alkyle en C₁-C₄, R' représente l'hydrogène ou un alkyle en C₁-C₄ et R₃, R₄, R₅, z et t sont tels que définis précédemment pour (I).

Cette réaction est effectuée en milieu acide, par chauffage dans un solvant inerte tel que le xylène ou le toluène.

Selon un autre mode opératoire, le composé 2 peut être préparé par action en milieu acide d'un aminoalkylamide (5") sur un ortho-ester d'alkyle (10) selon le schéma réactionnel suivant : 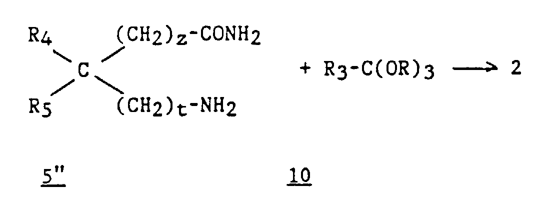 dans lequel R représente un alkyle en C₁-C₄.

En utilisant un mode opératoire décrit par H. Takenaka et al. (Heterocycles, 1989, 29 (6), 1185-89), on peut également préparer le composé 2, en faisant agir sur le dérivé 5" un halogénure d'acide de formule :

R₃ - CO - Hal 12

dans laquelle Hal représente un halogène, de préférence le chlore.

La réaction est réalisée en milieu basique.

Plus particulièrement, le composé 2, selon un autre objet de la présente invention, est préparé par un procédé caractérisé en ce que : on fait agir sur un composé de formule : 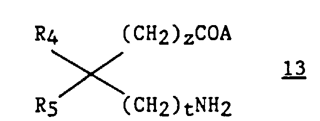 dans laquelle A représente un groupe OH, un groupe NH₂ ou un groupe OR', R' étant l'hydrogène ou un alkyle en C₁-C₄, un composé de formule :

R₃ - B 14

dans laquelle B représente :
- un groupement C (OR)₃
- un groupement 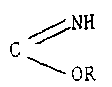 ou
- un groupement COHal
R étant un alkyle en C₁-C₄ et Hal désignant un atome d'halogène, de préférence le chlore ;
puis éventuellement, le composé ainsi obtenu est traité par le réactif de Lawesson (bis/méthoxy-4 phényl)-2,4 dithia-1,3 diphosphétane-2,4 disulfure.

Le dérivé du (biphényl-4-yl) méthyl (3) est préparé selon un procédé décrit dans la demande de brevet EP 324 377.

La transformation d'un groupe R'₁ et/ou R'₂ en un groupe R₁ et/ou R₂ est effectuée par des méthodes bien connues de l'homme de l'art. Ainsi, lorsque le composé (I) à préparer possède un groupe R₁ et/ou R₂=carboxy, R'₁ et/ou R'₂ représente un groupe carboxy estérifié. Lorsque le composé (I) à préparer possède un groupe R₁ et/ou R₂=tétrazolyle, R'₁ et/ou R' ₂ peut représenter soit un tétrazolyle protégé par exemple par un groupe trityle, soit un groupe cyano qui sera ensuite remplacé par un groupe tétrazolyle éventuellement protégé par un trityle. La transformation du groupe cyano en un tétrazolyle peut être effectuée par un azide, par exemple l'azide de tributyl étain ou par l'azide de sodium.

On peut également utiliser des groupes R'₁ et/ou R'₂ tels que les groupes nitro, carboxy, cyano ou chlorure d'acide et les transformer ensuite par des réactions bien connues de l'homme de l'art pour obtenir des groupes R₁ et/ou R₂ tels que définis pour le composé (I).

Ainsi, lorsque R'₁ et/ou R'₂ représente un carboxy, il peut être transformé en R₁ et/ou R₂ représentant un imidazol-1-yl carbonyle, ou bien en N-[(carboxy-4) thiazol-1,3 yl-2)]carbamoyle.

Le groupe R'₁ et/ou R'₂ représentant un chlorure d'acide peut être transformé en R₁ et/ou R₂ représentant N-hydroxycarbamoyl, N-cyanocarbamoyl, uréido ou cyano-2 guanidinocarbonyle.

Le groupe R'₁ et/ou R'₂ représentant un nitro peut être transformé en amino à partir duquel on prépare R₁ et/ou R₂, tel que méthylsulfonylamino, trifluorométhylsulfonylamino et trifluorométhylsulfonylaminométhyle.

Le groupe R'₁ et/ou R'₂ représentant un cyano peut être transformé en aminométhyle à partir duquel on prépare un cyano-3 méthyl-2 isothiouréidométhyle (selon C. Gordon et Al., J. Org. Chem., 1970, 35 (6), 2067-2069), un cyano-2 guanidinométhyl (selon R.W. Turner, Synthesis, 1975, 322).

L'étape a1) est réalisée dans un solvant inerte tel que le DMF, le DMSO ou le THF, en milieu basique, par exemple en présence de potasse, d'un alcoolate métallique, d'un hydrure métallique, de carbonate de calcium, ou de triéthylamine.

L'étape b1) est réalisée pas chauffage sous azote dans un solvant tel que le toluène, selon la méthode décrite par M.P. Cava et al., Tetrahedron, 1985, 41, 22, 5061.

Dans la description ci-après, le procédé comprenant les étapes a1, b1 et c1, est appelé procédé 1.

Alternativement, les composés (I) peuvent être préparés selon un autre procédé qui est également un objet de la présente invention. Ce procédé est caractérisé en ce que :
a2) on fait réagir un aminoacide de formule : 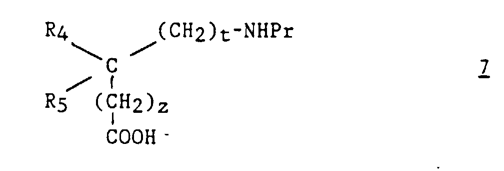 dans laquelle z, t, R₄ et R₅ ont les significations indiquées ci-dessus pour (I) et dont la fonction amine est protégée par le groupe Pr, sur un dérivé (biphényl-4-yl) méthylamine de formule : 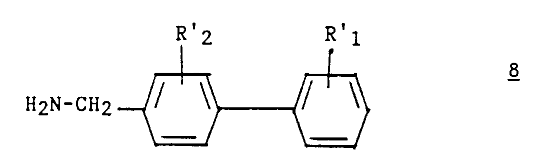 dans laquelle R'₁ et R'₂ représentent respectivement soit R₁ et R₂, soit un groupement précurseur de R₁ et R₂ ;
b2) après déprotection de l'amine, le composé ainsi obtenu de formule: 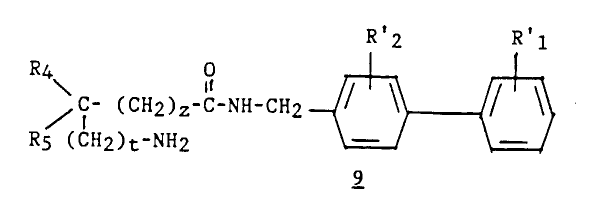 est ensuite traité par un ortho-ester d'alkyle de formule R₃C(OR)₃ (10) dans laquelle R₃ a la signification indiquée ci-dessus pour (I) et R est un alkyle en C₁-C₄ ;
c2) éventuellement, le composé ainsi obtenu de formule : 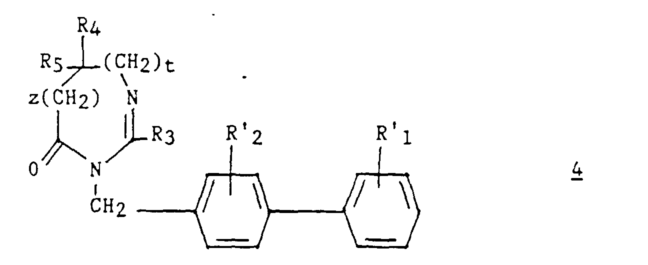 est traité par le réactif de Lawesson [bis(méthoxy-4 phényl)-2,4 dithia-1,3 diphosphétane-2,4 disulfure 2,4] ;
d2) le composé ainsi obtenu en b2 ou en c2 de formule : 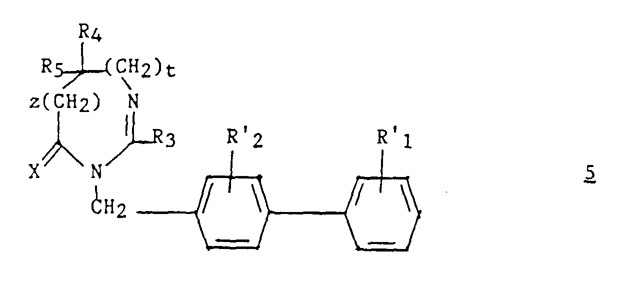 est ensuite traité dans des conditions convenables pour préparer le composé (I) par transformation des groupes R'₂ et/ou R'₁ en, respectivement, les groupes R₂ et/ou R₁.

Les composés 7 sont connus ou préparés par des méthodes connues (Chemistry of the Amino Acids, Greenstein and Winitz, John Wiley ed., 1961, vol. I, p. 697).

Les composés 8 sont préparés selon la demande de brevet européen 324 377. L'étape a2) est réalisée dans les conditions habituelles du couplage d'un acide sur une amine, par exemple en présence de BOP et de DIPEA.

L'étape b2) qui est la cyclisation du composé 9 en présence de 10 est effectuée selon Jacquier et al. (Bull. Soc. Chim. France, 1971, (3), 1040-1051) et selon Brunken et Bach (Chem. Ber., 1956, 89, 1363-1373).

Dans la description ci-après, le procédé comprenant les étapes a2 à d2 est appelé procédé 2.

Selon une variante du procédé 2, à l'étape b2, on peut éventuellement isoler un intermédiaire 9' de formule : 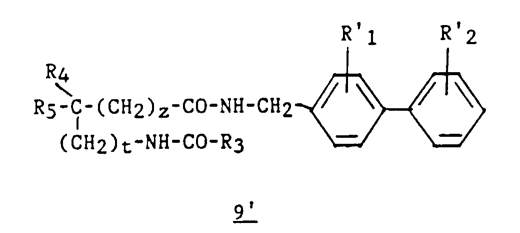 puis préparer la composé 4 par cyclisation en milieu acide.

Selon une autre variante du procédé 2 et pour préparer un composé I dans lequel R₄R₅ représente un groupe =CR₇R₈ on peut faire réagir en milieu acide,
un aminoacide de formule : 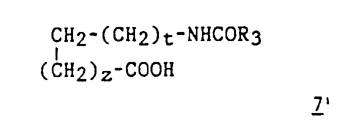 sur un aldéhyde ou une cétone de formule :

R₇COR₈

dans laquelle R₇ et R₈ ont les significations données ci-dessus pour (I), puis par action du composé 8, on obtient un composé de formule :

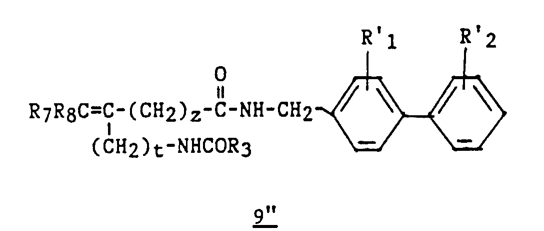

La cyclisation de ce composé en milieu acide conduit au composé 4,

Dans ce procédé, pour préparer un composé (I) dans lequel R₁ et/ou R₂ est un groupe carboxy, le substituant R'₁ et/ou R'₂ représente préférentiellement un groupe *tert*-butoxycarbonyle.

Enfin, une autre alternative pour la préparation des composés (I) selon l'invention dans lequel z et t sont égals à zéro, est le procédé par photooxydation qui est également un objet de la présente invention.

Ce dernier procédé est caractérisé en ce que :
a3) on fait agir sur un dérivé de l'imidazole de formule : 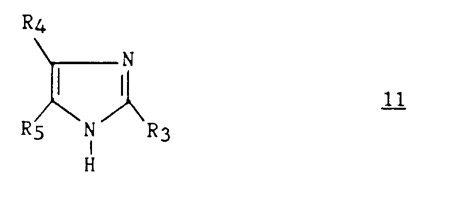 dans laquelle R₃, R₄, R₅ ont les significations indiquées ci-dessus pour (I),
   un dérivé du (biphényl-4-yl) méthyl de formule : 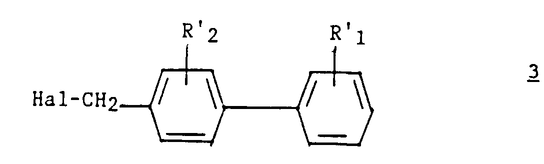 dans laquelle Hal représente un atome d'halogène et R'₁ et R'₂ représentent respectivement soit R₁ et R₂, soit un groupement précurseur de R₁ et R₂, en présence d'oxygène et d'une irradiation UV, en milieu basique ;
b3) éventuellement, le composé ainsi obtenu de formule : 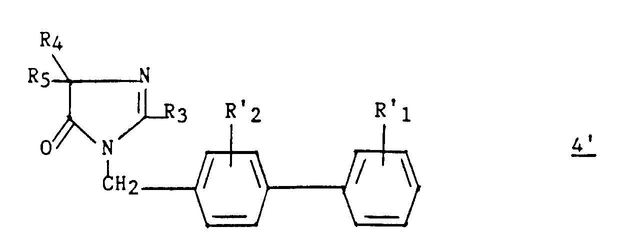 est traité par le réactif de Lawesson [bis(méthoxy-4 phényl)-2,4 dithia-1,3 diphosphétane-2,4 disulfure-2,4] ;
c3) le composé ainsi obtenu en b3 ou en c3 de formule : 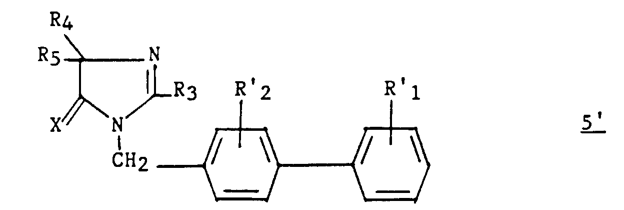 est ensuite traité dans des conditions convenables pour préparer le composé (I) par transformation des groupes R'₁ et/ou R'₂ en respectivement les groupes R₁ et/ou R₂.

Le dérivé de l'imidazole 11 est soit commercial, soit connu, soit préparé par des méthodes connues, indiquées ci-dessus pour la préparation des composés 2.

L'étape a3) est effectuée dans un solvant inerte tel que le DMF par exemple ; pour faciliter la réaction, on peut adjoindre un produit photosensibilisant comme le bleu de méthylène.

Dans la description ci-après, le procédé comprenant les étapes a3) à c3) est appelé procédé 3.

Les composés (I) selon l'invention, dans lesquels R₄ et R₅ liés ensemble représentent un groupe de formule (CH₂)ₚY (CH₂)_{q} dans lequel Y est un groupe NH, peuvent être préparés par hydrogénolyse catalytique d'un composé (I) correspondant dans lequel Y est un groupe N-R₆, R₆ étant un benzyle.

L'affinité des produits selon l'invention pour les récepteurs de l'angiotensine II a été étudiée sur un test de liaison de l'angiotensine II marquée à l'iode 125 à des récepteurs membranaires de foie de rats. La méthode utilisée est celle décrite par S. KEPPENS et al. dans Biochem. J., 1982, 208, 809-817.

On mesure la CI₅₀ : concentration qui donne 50 % de déplacement de l'angiotensine II marquée, liée spécifiquement au récepteur. La CI₅₀ des composés selon l'invention est inférieure à 10⁻⁶ M.

De plus l'effet antagoniste de l'angiotensine II des produits selon l'invention a été constaté sur différentes espèces animales dans lesquelles le système rénine-angiotensine a été préalablement activé (C. LACOUR et al., J. Hypertension, 1989, 7 (suppl.2), S33-S35).

Les composés selon l'invention sont actifs après administration par différentes voies, notamment par voie orale.

Aucun signe de toxicité n'est observé avec ces composés aux doses pharmacologiquement actives.

Ainsi les composés selon l'invention peuvent être utilisés dans le traitement de différentes affections cardiovasculaires, notamment l'hypertension, la défaillance cardiaque, l'insuffisance veineuse, ainsi que dans le traitement du glaucome, des rétinopathies diabétiques, et de différentes affections du système nerveux central, l'anxiété, la dépression, les déficits mnésiques ou la maladie d'Alzheimer par exemple.

La présente invention a également pour objet des compositions pharmaceutiques contenant une dose efficace d'un composé selon l'invention ou d'un sel pharmaceutiquement acceptable et des excipients convenables. Lesdits excipients sont choisis selon la forme pharmaceutique et le mode d'administration souhaité.

Dans les compositions pharmaceutiques de la présente invention pour l'administration orale, sublinguale, sous-cutanée, intramusculaire, intraveineuse, topique, intratrachéale, intranasale, transdermique ou rectale, les principes actifs de formule I ci-dessus, ou leurs sels éventuels, peuvent être administrés sous formes unitaires d'administration, en mélange avec des supports pharmaceutiques classiques, aux animaux et aux êtres humains pour la prophylaxie ou le traitement des troubles ou des maladies ci-dessus. Les formes unitaires d'administration appropriées comprennent les formes par voie orale telles que les comprimés, les gélules, les poudres, les granules et les solutions ou suspensions orales, les formes d'administration sublinguale, buccale, intratrachéale, intranasale, les formes d'administration sous-cutanée, intramusculaire ou intraveineuse et les formes d'administration rectale. Pour l'application topique, on peut utiliser les composés selon l'invention dans des crèmes, pommades ou lotions.

Afin d'obtenir l'effet prophylactique ou thérapeutique désiré, la dose de principe actif peut varier entre 0,01 et 50 mg par kg de poids du corps et par jour.

Chaque dose unitaire peut contenir de 0,1 à 1000 mg, de préférence de 1 à 500 mg, d'ingrédients actifs en combinaison avec un support pharmaceutique. Cette dose unitaire peut être administrée 1 à 5 fois par jour de façon à administrer un dosage journalier de 0,5 à 5000 mg, de préférence de 1 à 2500 mg.

Lorsqu'on prépare une composition solide sous forme de comprimés, on mélange l'ingrédient actif principal avec un véhicule pharmaceutique tel que la gélatine, l'amidon, le lactose, le stéarate de magnésium, le talc, la gomme arabique ou analogues. On peut enrober les comprimés de saccharose, d'un dérivé cellulosique, ou d'autres matières appropriées ou encore on peut les traiter de telle sorte qu'ils aient une activité prolongée ou retardée et qu'ils libèrent d'une façon continue une quantité prédéterminée de principe actif.

On obtient une préparation en gélules en mélangeant l'ingrédient actif avec un diluant et en versant le mélange obtenu dans des gélules molles ou dures.

Une préparation sous forme de sirop ou d'elixir ou pour l'administration sous forme de gouttes peut contenir l'ingrédient actif conjointement avec un édulcorant, acalorique de préférence, du méthylparaben et du propylparaben comme antiseptiques, ainsi qu'un agent donnant du goût et un colorant approprié.

Les poudres ou les granules dispersibles dans l'eau peuvent contenir l'ingrédient actif en mélange avec des agents de dispersion ou des agents mouillants, ou des agents de mise en suspension, comme la polyvinylpyrrolidone, de même qu'avec des édulcorants ou des correcteurs du goût.

Pour une administration rectale, on recourt à des suppositoires qui sont préparés avec des liants fondant à la température rectale, par exemple du beurre de cacao ou des polyéthylèneglycols.

Pour une administration parentérale, on utilise des suspensions aqueuses, des solutions salines isotoniques ou des solutions stériles et injectables qui contiennent des agents de dispersion et/ou des mouillants pharmacologiquement compatibles, par exemple le propylèneglycol ou le butylèneglycol.

Le principe actif peut être formulé également sous forme de microcapsules, éventuellement avec un ou plusieurs supports ou additifs.

Les compositions de la présente invention peuvent contenir, à côté des produits de formule I ci-dessus ou d'un des sels pharmaceutiquement acceptables, d'autres principes actifs tels que, par exemple, des tranquillisants ou d'autres médicaments qui peuvent être utiles dans le traitement des troubles ou maladies indiquées ci-dessus.

Ainsi, la présente invention a pour objet des compositions pharmaceutiques contenant plusieurs principes actifs en association dont l'un est un composé selon l'invention et le ou les autres peuvent être un composé béta-bloquant, un antagoniste calcique, un diurétique, un antiinflammatoire non stéroïdien ou un tranquillisant.

Les exemples suivants illustrent l'invention sans toutefois la limiter. Dans ces exemples, on utilise les abréviations suivantes : d signifie densité, TA signifie température ambiante, KHSO₄-K₂SO₄ signifie une solution aqueuse contenant 16,6 g de bisulfate de potassium et 33,3 g de sulfate de potassium pour 1 litre.

Les points de fusion (Fc) sont donnés en degrés Celsius ; sauf indication contraire, ils ont été mesurés sans recristallisation du produit.

La pureté des produits est vérifiée en chromatographie sur couche mince (CCM) ou par HPLC. Les produits sont caractérisés par leur spectre RMN enregistré à 200 MHz dans le DMSO deutéré, la référence interne étant le tétraméthylsilane.

Pour l'interprétation des spectres de RMN, on emploie :
- s: pour singulet
- s.e.: pour singulet élargi
- d: pour doublet
- t: pour triplet
- q: pour quadruplet
- quint: pour quintuplet
- sext: pour sextuplet
- m: pour massif ou multiplet.

Par ailleurs, im signifie imidazole.

De façon classique, les atomes d'hydrogène sont numérotés sur le biphénylyle comme représenté dans la formule suivante :

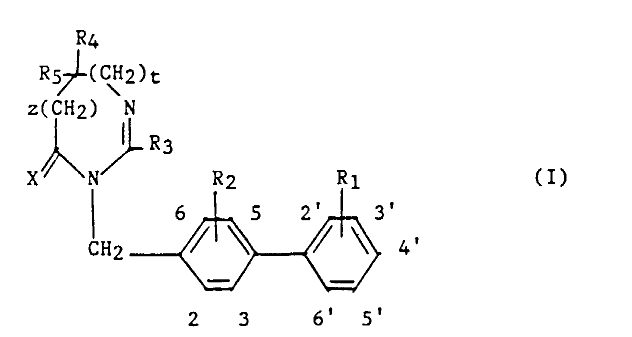

Dans les composés suivants, z et t sont nuls, sauf lorsque le composé préparé est une pyrimidinone.

### EXEMPLE 1

n-butyl-2 spirocyclopentane-4 [(*tert*-butoxycarbonyl-2' biphényl-4-yl) méthyl]-1 imidazoline-2 one-5.

et trifluoroacétate de n-butyl-2 [(carboxy-2' biphényl-4-yl) méthyl]-1 spirocyclopentane-4 imidazoline-2 one-5 - Procédé 2.
A) L'acide N-Fmoc amino-1 cyclopentane carboxylique est préparé selon le procédé décrit par CHI-DEU CHANG et al. (Int. J. Peptide Protein Res., 1980, 15, 59-66). Fc = 89-91°C.
B) N-(*tert*-butoxycarbonyl-2' biphényl-4-yl méthyl)-(N-Fmoc amino)-1 cyclopentanecarboxamide-1.
   700 mg du produit préparé à l'étape précédente sont dissous dans 8 ml de DMF et on ajoute successivement 576 mg d'aminométhyl-4 (*tert*-butoxycarbonyl-2') biphényle, 970 mg de BOP et une quantité suffisante de DIPEA pour amener à pH = 6.
   Après 1 heure d'agitation, le milieu réactionnel est dilué par 100 ml d'acétate d'éthyle et 20 ml d'eau ; la phase organique est lavée successivement par une solution saturée de bicarbonate de sodium puis par une solution KHSO₄-K₂SO₄, enfin par une solution saturée de chlorure de sodium. Après séchage sur sulfate de sodium, la solution est évaporée à sec. On obtient une huile m = 1,2 g.
C) N-(*tert*-butoxycarbonyl-2' biphényl-4-yl méthyl)-(amino-1) cyclopentanecarboxamide-1.
   Le produit obtenu à l'étape précédente est dissous dans 10 ml de DMF, puis on ajoute 1 ml de diéthylamine et on agite pendant 1 heure 15 à TA. Le milieu réactionnel est repris par 100 ml d'acétate d'éthyle et 20 ml d'eau puis la phase organique est lavée 1 fois à l'eau, 1 fois par une solution saturée de chlorure de sodium, puis séchée sur sulfate de sodium et évaporée à sec.
   Le résidu est chromatographié sur gel de silice en éluant par le mélange acétate d'éthyle/méthanol/ammoniaque à 30% (99/1/0,5 ; v/v/v). On obtient 600 mg du produit attendu.
   - IR (CHCl₃)
      3350 cm⁻¹ : H (amide et amine)
      1700 cm⁻¹ : C = O (CO₂ tBu)
      1650 cm⁻¹ : C = O (CONH)

   Spectre RMN :
   1,25 ppm : s : 9 H : tBu
   2,15 - 1,40 ppm : m : 10 H : (C₅H₈, NH₂)
   4,40 ppm : d : 2 H : CH₂-NH
   7,15-7,75 ppm : m : 8 H : biphényle
   8,60 : t : 1 H : NH - CH₂
D) n-butyl-2 spirocyclopentane-4 [(*tert*-butoxycarbonyl-2' biphényl-4-yl) méthyl]-1 imidazoline-2 one-5.
   394 mg du produit préparé à l'étape précédente et 250 mg d'orthovalérate d'éthyle sont mélangés dans 2 ml de DCM. On ajoute 1 goutte d'acide acétique puis on chauffe à 90°C en laissant évaporer le DCM. Après 1 heure 15, le milieu réactionnel est repris dans 50 ml d'acétate d'éthyle, 10 ml d'eau et 1 ml de solution saturée de bicarbonate de sodium. La phase organique est ensuite lavée par une solution saturée de chlorure de sodium puis séchée sur sulfate de sodium et évaporée à sec. Le résidu est chromatographié sur gel de silice en éluant par un mélange acétate d'éthyle/toluène (1/2, v/v). On obtient 390 mg du produit attendu qui cristallise. Fc = 63-65°C.
   - IR (CHCl₃) :
      1710-1720 cm⁻¹ : C = O, C = O (ester et imidazoline)
      1625 cm⁻¹: C = N

   Spectre RMN :
   0,88 ppm : t : 3 H : CH₃ (nBu)
   1,20 ppm : s : 9 H : tBu
   1,35 ppm : sext : 2 H : CH₃-CH₂-
   1,58 ppm : quint : 2 H : CH₃-CH₂-CH₂-
   1,95-1,65 ppm : m : 8 H : cyclopentane
   2,42 ppm : t : 2 H : CH₃-CH₂-CH₂-CH₂-
   4,78 ppm : s : 2 H : CH₂-C₆H₄-
   7,20-7,80 ppm : m : 8 H : H aromatiques
      - spectre de masse : MH⁺ : 461.
E) trifluoroacétate de n-butyl-2 [(carboxy-2' biphényl-4-yl) méthyl]-1 spirocyclopentane-4 imidazoline-2 one-5.
   180 mg du produit préparé à l'étape précédente sont traités par 3 ml de DCM et 4 ml de TFA pendant 45 minutes. Après évaporation sous vide, le résidu est repris dans l'éther. On obtient un solide blanc qui est filtré, lavé à l'éther puis séché sous vide. m = 155 mg. Fc = 176-178°C ,
   Spectre RMN :
   0,78 ppm : t : 3 H : CH₃ (nBu)
   1,25 ppm : sext : 2 H : CH₃-CH₂
   1,50 ppm : quint : 2 H : CH₃-CH₂-CH₂
   1,75-2,00 : m, 8 H : cyclopentane
   2,65 ppm : t : 2 H : CH₃-CH₂-CH₂-CH₂-
   4,83 ppm : s : 2 H : CH₂-C₆H₄-
   7,20-7,75 ppm : m : 8 H : aromatiques
      - spectre de masse : MH⁺ : 405

### EXEMPLE 2

Trifluoroacétate de n-butyl-2 [(carboxy-2' biphényl-4-yl) méthyl]-1 spirocyclopentane-4 imidazoline-2 one-5 - Procédé 1.
A) n-butyl-2 spirocyclopentane-4 imidazoline-2 one-5.
   L'ester éthylique de l'acide amino-1 cyclopentane carboxylique est préparé selon ADKINS et BILLICA (J. Amer. Chem. Soc., 1948, 70, 3121).
   Le valérimidate d'éthyle est préparé selon Mac ELVAIN (J. Amer. Chem. Soc., 1942, 64 1825-1827) puis libéré de son chlorhydrate par action du carbonate de potassium et extraction par le DCM.
   L'ester éthylique de l'acide amino-1 cyclopentane carboxylique (1,57g) et le valérimidate d'éthyle (1,56 g) sont dissous dans 12 ml de xylène contenant 6 gouttes d'acide acétique. Après 6 heures et demi de chauffage à reflux, le milieu réactionnel est concentré sous vide puis le résidu est chromatographié sur gel de silice en éluant par un mélange chloroforme/méthanol/acide acétique (94/4/2 ; v/v/v). La fraction contenant le produit attendu est évaporée plusieurs fois en présence de xylène puis de benzène pour éliminer l'acide acétique. On obtient 1,91 g de produit sous forme d'une huile épaisse.
   - IR (CHCl₃) :
      1720 cm⁻¹ : C = O
      1635 cm⁻¹ : C = N
      Remarque : le fait de ne pas voir de bande entre 1500 et 1600 cm⁻¹ indique que, dans la solution de chloroforme, le produit est une imidazolinone-5.
      Spectre RMN :
      0,92 ppm : t : 3 H : CH₃ (nBu)
      1,35 ppm : sext : 2 H : CH₃-CH₂-
      1,50-1,93 ppm : m : 10 H : CH₃-CH₂-CH₂ et cyclopentane
      2,33 ppm : t : 2 H : CH₃-CH₂-CH₂-CH₂-
      10,7 ppm : m : NH
   - spectre de masse : MH⁺ : 195

La n-butyl-2 spirocyclopentane-4 imidazoline-2 one-5 préparé à l'étape A, peut également être obtenue selon un autre mode opératoire décrit ci-dessous, en utilisant comme produit de départ la cyclopentanone.
a) amino-1 cyclopentanenitrile.
   Cette étape est réalisée selon A. Strecker (Org. Synth., 1955, 3)
   Dans un ballon on dissout 1,97 g du cyanure de sodium dans 3,9 ml d'eau et on ajoute une solution contenant 2,33 g de chlorure d'ammonium dans 5,9 ml d'eau et 3,5 ml d'ammoniaque à 20 %, enfin on ajoute dans le ballon 3 g de cyclopentanone dans 3,8 ml de méthanol. Après 1 heure et demie sous agitation, on porte à 60°C pendant 45 minutes puis on arrête le chauffage, maintient l'agitation pendant 45 minutes puis refroidit à 25°C. On extrait plusieurs fois par du chlorure de méthylène. On sèche sur sulfate de sodium, filtre et concentre sous vide. On obtient 4 g du produit attendu sous forme huileuse.
   L'amino-1 cyclopentane nitrile obtenu est mis en solution dans 300 ml d'acétone et l'on ajoute, sous agitation, une solution de 2,25 g d'acide oxalique dihydraté dans 200 ml d'acétone. Le précipité formé est essoré, lavé avec de l'acétone puis séché.
   m = 4,71.g
   Fc = 220°C.

   Ce composé est l'hemioxalate d'amino-1 cyclopentane nitrile.
b) amino-1 cyclopentane carboxamide.
   Cette étape est réalisée selon J. Zabicky, (The Chemistry of Amides, Intersciences, New-York, 1970, 119).
   5,1 g de l'oxalate obtenu à l'étape précédente sont traités en 45 minutes et sous agitation par 7,65 ml d'acide sulfurique concentré (d = 1,84). On observe un dégagement gazeux, la température augmente jusqu'à 100°C. On refroidit vers 35°C et verse sur un mélange glace-ammoniaque concentrée, (10 g/2,8 ml). La suspension formée est extraite 6 fois de suite avec du chloroforme contenant 5 % de méthanol. On ajoute 3 ml d'ammoniaque (d = 0,92) à la phase aqueuse et extrait à nouveau par du chloroforme contenant du méthanol (1/0,5 ; v/v). Les phases organiques réunies sont séchées sur sulfate de sodium, on filtre et concentre. Le produit attendu est obtenu sous forme d'un solide blanc.
   m = 3,79 g
   Fc = 95°C.

   Les résultats de l'analyse et le spectre IR permettent de confirmer la structure.
c) n-butyl-2 spirocyclopentane-4 imidazoline-2 one-5
   Cette étape est réalisée selon H. Takenaka et al., Heterocycles, 1989, 29 (6), 1185-89.
   3 g du composé préparé à l'étape précédente sont placés dans 70 ml de THF anhydre et 3,3 ml de triéthylamine et l'on ajoute, sous agitation, 3,ml de chlorure de valéryle dans 10 ml de THF anhydre. Il se forme une suspension blanche. Le composé intermédiaire formé, mais non isolé, est le (N-valéryl) amino-1 cyclopentane carboxamide. On ajoute 6 g de potasse en pastille, 7 ml d'eau et 16 ml de méthanol. On chauffe à reflux pendant 2 heures et demie puis l'on ajoute 9 g de chlorure d'ammonium. Après 15 minutes d'agitation, on concentre sous vide. Le résidu obtenu est repris par 40 ml d'eau et extrait par 10 ml d'acétate d'éthyle puis 2 fois par 5 ml d'acétate d'éthyle. Les phases organiques réunies sont séchées sur sulfate de sodium et filtrées. Le filtrat est concentré à sec. On obtient 4,85 g du produit attendu. Le spectre de RMN est semblable à celui décrit précédemment. On peut préparer le chlorhydrate de ce composé par addition d'acide chlorhydrique concentré. Le chlorhydrate fond à 240°C en se sublimant.
B) n-butyl-2 spirocyclopentane-4 [(*tert*-butoxycarbonyl-2' biphényl-4-yl) méthyl]-1 imidazoline-2 one-5.
   970 mg du produit obtenu à l'étape A) sont dissous dans 10 ml de DMF. On ajoute 270 mg de méthylate de sodium et laisse sous agitation pendant 15 minutes à TA. On additionne 2,08 g de bromométhyl-4 (*tert*-butoxycarbonyl-2') biphényle à la suspension puis, après 30 minutes, on chauffe à 40°C sous azote pendant 3 heures et demi. Le milieu réactionnel est repris par un mélange contenant 100 ml d'acétate d'éthyle, 10 ml d'eau et 1 ml de solution saturée de bicarbonate de sodium. La phase organique est lavée par une solution saturée de chlorure de sodium puis séchée sur sulfate de sodium et évaporée à sec. Le résidu est chromatographié sur gel de silice en éluant par un mélange acétate d'éthyle/toluène (1/2 ; v/v). On obtient 1,25 g du produit attendu qui cristallise. Fc = 63-66°C.
   Les spectres IR, RMN et le spectre de masse ainsi que le Rf sont identiques à ceux obtenus à l'étape D) de l'exemple 1.
C) Trifluoroacétate de n-butyl-2 [(carboxy-2' biphényl-4-yl) méthyl]-1 spirocyclopentane-4 imidazoline-2 one-5.
   1,22 g de produit obtenu à l'étape précédente est agité pendant 40 minutes dans une solution contenant 6 ml de DCM et 8 ml de TFA. Après concentration sous vide, le résidu est repris dans de l'éther éthylique ; le précipité blanc formé est filtré, lavé à l'éther, puis séché sous vide. On obtient 1,15 g du produit attendu. Fc = 176-178°C.
   Les spectres IR, RMN et spectre de masse sont identiques à ceux obtenus à l'exemple 1E, de même le Rf observé en CCM est identique.

### EXEMPLE 3

Trifluoroacétate de n-butyl-2 [(carboxy-2' biphényl-4-yl) méthyl]-1 spirocyclopentane-4 imidazoline-2 one-5. - Procédé 3.
A) On prépare le n-butyl-2 benzimidazole selon W.O. POOL (J. Amer. Chem. Soc., 1937, 59, 178), puis on prépare le n-butyl-2 tétrahydro-4,5,6,7, benzimidazole selon M. HARTMANN et L. PANIZZON (Hel. Chim. Acta, 1938, 21, 1692-1694). Fc = 145°C.
   Spectre RMN :
   0,82 ppm : t : 3 H : CH₃ (nBu)
   1,23 ppm : sext : 2 H : CH₃-CH₂-
   1,50 ppm : quint : 2 H : CH₃-CH₂-CH₂-
   1,65 ppm : s : 4 H : H₅, H₆ (tétrahydrobenzimidazole)
   2,35 ppm : s; 4 H : H₄, H₇ (tétrahydrobenzimidazole)
   2,45 ppm : t : 2 H : CH₃-CH₂-CH₂-CH₂-
   11,1 ppm : m : NH
      - spectre de masse : M⁺ : 178
B) n-butyl-2 spirocyclopentane-4 [(*tert*-butoxycarbonyl-2' biphényl-4-yl) méthyl]-1 imidazoline-2 one-5.
   1 g du produit préparé à l'étape précédente est dissous dans 45 ml de DMF avec 303 mg de méthylate de sodium et quelques mg de bleu de méthylène. On fait barboter de l'oxygène dans le milieu réactionnel qui est éclairé avec une lampe UV. Après 15 minutes, on ajoute 2,14 g de bromométhyl-4 (*tert*-butoxycarbonyl-2') biphényle puis, après 1 heure, le milieu réactionnel est repris dans 300 ml d'acétate d'éthyle additionné de 50 ml d'eau et de 5 ml de solution saturée de bicarbonate de sodium. La phase organique est ensuite lavée par une solution saturée de chlorure de sodium puis séchée sur sulfate de sodium et évaporée à sec. Le résidu est chromatographié sur gel de silice en éluant par un mélange acétate d'éthyle/toluène (1/2, v/v). On obtient 610 mg du produit attendu qui cristallise. Fc = 62-65°C.
   Les spectres IR, RMN, le spectre de masse de même que le Rf sont identiques à ceux obtenus précédemment pour le même composé.
C) Trifluoroacétate de n-butyl-2 [(carboxy-2' biphényl-4-yl) méthyl]-1 spirocyclopentane-4 imidazoline-2 one-5.
   Ce composé est obtenu par traitement en milieu acide comme décrit à la dernière étape de l'exemple 1 et de l'exemple 2. Les données physicochimiques sont identiques à celles obtenues pour le même composé préparé par les procédés 1 ou 2.

### EXEMPLE 4

n-butyl-2 diméthyl-4,4 [(*tert*-butoxycarbonyl-2' biphényl-4-yl) méthyl]-1 imidazoline-2 one-5.

et trifluoroacétate de n-butyl-2 [(carboxy-2' biphényl-4-yl) méthyl]-1 diméthyl-4,4 imidazoline-2 one-5 - Procédé 1.
A) n-butyl-2 diméthyl-4,4 imidazoline-2 one-5.
   On prépare l'ester éthylique de l'acide alpha amino isobutyrique selon R. Jacquier et al. (Bull. Soc. Chim., France, 1971, (3), 1040-1051). 650 mg de ce composé et 780 mg de valérimidate d'éthyle sont dissous dans 8 ml de xylène contenant 4 gouttes d'acide acétique et on chauffe à reflux pendant 7 heures. Le milieu réactionnel est alors concentré sous vide et le résidu est chromatographié sur gel de silice en éluant par un mélange chloroforme/méthanol/acide acétique (95/5/2, v/v/v). Après plusieurs évaporations avec du xylène, puis du benzène pour éliminer l'acide acétique, on obtient 560 mg du produit attendu qui cristallise. Fc = 35-38°C.
   - IR (CHCl₃)
      1725 cm⁻¹ : C = O
      1635 cm⁻¹ : C = N
      Remarque : l'absence de signal entre 1500 et 1600 cm⁻¹ confirme que le composé présent dans la solution de chloroforme est une imidazoline-2 one-5.
      Spectre RMN :
      0,92 ppm : t : 3H : CH₃(nBu)
      1,20 ppm : s : 6H : C (CH₃)₂
      1,38 ppm : sext : 2H : CH₃-CH₂
      1,63 ppm : quint : 2 H : CH₃-CH₂-CH₂-
      2,38 ppm : t : 2 H : CH₃-CH₂-CH₂-CH₂-
      10,7 ppm : m : 1 H : N-H
   - spectre de masse : MH⁺ : 169
B) n-butyl-2 diméthyl-4,4 [(*tert*-butoxycarbonyl-2' biphényl-4-yl) méthyl]-1 imidazoline-2 one-5.
   520 mg du produit préparé à l'étape précédente sont dissous dans 10 ml de DMF. On ajoute 167 mg de méthylate de sodium et on agite sous azote pendant 15 minutes. On ajoute ensuite 1,25 g de bromométhyl-4 (*tert*-butoxycarbonyl-2') biphényle et on laisse sous agitation à 40°C pendant 3 heures et demie. Le milieu réactionnel est repris dans 150 ml d'acétate d'éthyle puis 20 ml d'eau et 2 ml de solution de bicarbonate de sodium saturée. La phase organique est lavée par une solution saturée de chlorure de sodium, séchée sur sulfate de sodium et évaporée à sec. Le résidu est chromatographié sur gel de silice en éluant par le mélange acétate d'éthyle/toluène (1,2/2 ; v/v). On obtient 570 mg du produit attendu qui cristallise. Fc = 98-100°C.
   - IR ( CHCl₃)
      1710-1720 cm⁻¹ : C = O, C = O (imidazolinone, ester)
      1625 cm⁻¹ : C = N

      Spectre RMN :
      0,78 ppm : t : 3 H : CH₃ (nBu)
      1,08 ppm : s : 9 H, C(CH₃)₃
      1,15 ppm : s : C (CH₃)₂ et 1,20 ppm : sext : CH₃-CH₂- ) 8 H
      1,45 ppm : quint : 2 H : CH₃-CH₂-CH₂-
      2,30 ppm : t : 2 H : CH₃-CH₂-CH₂-CH₂-
      4,65 ppm : s : 2 H : CH₂-C₆H₄-
      7,15-7,65 ppm : m : 8 H : H aromatiques

      L'étude N.O.E. (Nuclear Overhauser Effect) confirme la position des substitutions 5-one et 4,4-diméthyl sur l'imidazolinone.
   - spectre de masse : MH⁺ : 435
C) trifluoroacétate de n-butyl-2 [(carboxy-2' biphényl-4-yl) méthyl]-1 diméthyl-4,4 imidazoline-2 one-5.
   460 mg du produit préparé à l'étape précédente sont traités pas 3 ml de DCM et 4 ml de TFA pendant 45 minutes. Après concentration sous vide, le résidu est repris dans l'éther et le précipité formé est filtré, lavé à l'éther puis séché sous vide. On obtient 450 mg de produit attendu sous forme d'un solide blanc. Fc = 168-171°C.
   Spectre RMN :
   0,82 ppm : t : 3 H : CH₃ (nBu) 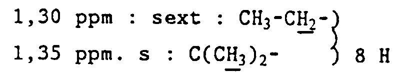
   1,55 ppm : quint : 2 H : CH₃-CH₂-CH₂-
   2,62 ppm : t : 2 H : CH₃-CH₂-CH₂-CH₂-
   4,82 ppm : s : 2 H : CH₂-C₆H₄-
   7,20-7,75 : m : 8 H aromatiques

   - spectre de masse : MH⁺ : 379

### EXEMPLE 5

[(cyano-2' biphényl-4-yl) méthyle]-1 n-butyl-2 spirocyclopentane-4 imidazoline-2 one-5.

et n-butyl-2 spirocyclopentane-4 [((tétrazolyl-5)-2' biphényl-4-yl) méthyl]-1 imidazoline-2 one-5 - Procédé 1.
A) [(cyano-2' biphényl-4-yl) méthyl]-1 n-butyl-2 spirocyclopentane-4 imidazoline-2 one-5.
On prépare sous atmosphère d'azote un mélange contenant 250 mg d'hydrure de sodium (dispersé à 80 % dans l'huile minérale) et 5 ml de DMF et on ajoute goutte à goutte une solution contenant 0,97 g de n-butyl-2 spirocyclopentane-4 imidazoline-2 one-5 (préparé à l'exemple 2, étape A) dans 10 ml de DMF. On agite 30 minutes à TA puis on ajoute une solution de 1,5 g de bromométhyl-4 cyano-2' biphényle dans 10 ml de DMF. Après 1 heure d'agitation à TA, on évapore le DMF sous pression réduite puis on reprend le résidu par de l'acétate d'éthyle, lave la phase organique avec de l'eau puis sèche sur sulfate de sodium, filtre et évapore. Le résidu est chromatographié sur gel de silice en éluant par un mélange DCM/acétate d'éthyle (9/1, v/v). On récupère 1,68 g du produit attendu. Fc = 92-93°C.
B) n-butyl-2 spirocyclopentane-4 [(triphénylméthyltétrazolyl-5)-2' biphényl-4-yl méthyl]-1 imidazoline-2 one-5.
1,56 g du produit précédent,2,6 g d'azide de tributylétain et 30 ml de xylène sont chauffés à reflux pendant 66 heures. Le xylène est ensuite évaporé et le résidu est dissous dans 20 ml de DCM et 5 ml de THF, en ajoutant 0,8 ml de soude 10 N et, après 30 minutes d'agitation, 2,5 g de chlorure de trityle et on laisse sous agitation pendant 26 heures. Après évaporation des solvants, le résidu est repris par de l'acétate d'éthyle et lavé à l'eau par une solution de sulfate acide potassium à 3 % et de l'eau. On sèche et évapore. Le résidu est chromatographié sur alumine en éluant sur le mélange hexane/acétate d'éthyle (9/1 ; v/v). On obtient 1,97 g du produit attendu. Fc = 150-152°C.
C) n-butyl-2 spirocyclopentane-4 [((tétrazolyl-5)-2' biphényl-4-yl) méthyl]-1 imidazoline-2 one-5.
1,96 g du produit préparé à l'étape précédente sont dissous dans 10 ml de méthanol et 10 ml de THF. Après refroidissement du milieu réactionnel à 5°C, on ajoute 1,5 ml d'acide chlorhydrique 4N et on agite pendant 3 heures à TA et 1 heure à 30°C. Après évaporation des solvants, le résidu est repris par de l'eau et l'on porte à pH 12 par addition de soude 10 N. La phase aqueuse est extraite par de l'éther, du toluène et à nouveau de l'éther. On acidifie la phase aqueuse jusqu'à pH 2 par addition d'acide chlorhydrique 1 N, puis on extrait à l'acétate d'éthyle, sèche et évapore. Le solide blanc obtenu est séché à 50°C sous 0,05 mm de mercure. On obtient 840 mg du produit attendu. Fc = 180-181°C.
Spectre RMN :
0,75 ppm : t : 3 H : CH₃ (nBu)
1,10 ppm : sext : 2 H : CH₃-CH₂-
1,20 ppm : quint : 2 H : CH₃-CH₂-CH₂-
1,5-2 ppm : m : 8 H : -C₅H₈
2,2 ppm : t : 2 H : CH₃-CH₂-CH₂-CH₂-
4,6 ppm : s : 2H : CH₂-C₆H₄-
7 ppm : s : 4 H : CH₂-C₆H₄-
7,35-7,7 ppm : m : 4 H : H_{3',4', 5', 6'} aromatiques

L'étude N.O.E. confirme la position de la substitution 5-one sur l'imidazole.
D) Sel de potassium du n-butyl-2 spirocyclopentane-4 [((tétrazolyl-5)-2' biphényl-4-yl) méthyl]-1 imidazoline-2 one-5.
On dissout 970 mg du composé obtenu à l'étape précédente dans 40 ml d'un mélange isopropanol-méthanol (1/1, v/v), on ajuste à pH 12 par addition d'une solution de potasse à 85% dans un mélange méthanol-eau (20/1, v/v). On évapore, reprend le résidu par de l'isopropanol et évapore à nouveau. Le résidu est dissous dans 20 ml d'isopropanol en chauffant légèrement, puis on laisse revenir à température ambiante. On laisse décanter, évapore le filtrat puis reprend le résidu par de l'heptane. Après trituration, le produit concrétise ; on le filtre puis lave à nouveau avec de l'heptane et sèche sous vide. On obtient 945 mg du sel de potassium attendu. Fc = 142-144°C

| - Analyse élémentaire : C₂₅H₂₇KN₆O, H₂O | | | |
|---|---|---|---|
| calc. : | C : 61,95 | H : 6,03 | N : 17,34 |
| trouvé % | 62,02 | 6,13 | 17,14 |

### EXEMPLE 6

Trifluoroacétate de n-butyl-2 [(carboxy-2' biphényl-4-yl) méthyl]-1 (spirotétrahydropyranne-4)-4 imidazoline-2 one-5.

et n-butyl-2 (spirotétrahydropyranne-4)-4 [(*tert*-butoxycarbonyl-2' biphényl-4-yl) méthyl]-1 imidazoline-2 one-5 - Procédé 2.
A) L'acide amino-4 tétrahydropyrannecarboxylique-4 est préparé à partir de la tétrahydropyrannone-4 selon la méthode décrite dans le brevet allemand DE - 2 215 721.
B) (N-benzyloxycarbonylamino)-4 carboxy-4 tétrahydropyranne.
   1,015 g du composé de l'étape A est placé dans 12 ml d'eau et traité à 10°C par 1,22 ml de diisopropyléthylamine puis 3,33 g de N-(benzyloxycarbonyloxy) succinimide dissous dans 12 ml d'acétonitrile. Après 1 heure 15 minutes, le milieu réactionnel est dilué par 70 ml d'acétate d'éthyle et 10 ml d'eau et on ramène à pH2 par une solution saturée de bisulfate de potassium.
   Après décantation, la phase organique est lavée par une solution saturée de chlorure de sodium, séchée sur sulfate de sodium puis évaporée sous vide. Le résidu est dilué dans 60 ml d'éther puis on ajoute 7 mmoles de dicyclohexylamine. Le précipité formé est filtré et lavé à l'éther ; on le dissout ensuite dans un mélange acétate d'éthyle-eau et on l'amène à pH 1,5 par une solution saturée de bisulfate de potassium. La phase organique est décantée, lavée par une solution saturée de chlorure de sodium, évaporée sous vide et l'on obtient 1,9 g d'un solide blanc. Fc = 110-115°C.
C) N-(*tert*-butoxycarbonyl-2' biphényl-4-yl méthyl) (N-benzyloxycarbonylamino)-4 tétrahydropyrannecarboxamide-4.
   850 mg du composé préparé à l'étape B sont dissous dans 15 ml de DMF et on ajoute des quantités équimolaires d'aminométhyl-4 (*tert*-butoxycarbonyl-2') biphényle, de DIPEA puis du BOP (10% d'excès). Après 40 minutes, le milieu est repris par 200 ml d'acétate d'éthyle et 200 ml d'eau. La phase organique est décantée puis lavée 2 fois par une solution saturée de bicarbonate de sodium, 2 fois par une solution à 5% de bisulfate de sodium puis 1 fois par une solution saturée de chlorure de sodium. Après séchage sur sulfate de sodium, la phase organique est évaporée sous vide. On obtient 1,8 g du produit attendu.
D) N-(*tert*-butoxycarbonyl-2' biphényl-4-yl méthyl) amino-4 tétrahydropyrannecarboxamide-4.
   Le produit obtenu à l'étape C est dissous dans 30 ml de méthanol. On ajoute 400 mg de Palladium à 10% sur charbon et hydrogène à pression atmosphérique. Après 1 heure, le catalyseur est filtré, puis le filtrat est concentré sous vide. Le résidu est chromatographié sur silice en éluant par un mélange acétate d'éthyle-méthanol-ammoniaque à 33% (99/1/0,5, v/v/v). On obtient 0,93 g du produit attendu sous forme d'un solide blanc. Fc = 125-127°C.
   Spectre RMN :
   8,50 ppm : t : 1 H : H amide
   7,60-7,05 ppm : m : 8 H : H aromatiques
   4,25 ppm : d : 2 H : CH₂ - C₆H₄-
   3,70-3,50 ppm : m : 4 H : CH₂ en 2 et 6 du tétrahydropyranne
   2,00-1,80 ppm : m : 4 H : CH₂ en 3 et 5 du tétrahydropyranne
   1,05 ppm : s : 9 H : tBu
E) n-butyl-2 (spirotetrahydropyranne-4)-4 [(*tert*-butoxycarbonyl-2' biphényl-4-yl) méthyl]-1 imidazoline-2 one-5
   On chauffe pendant 3 heures à 110°C un mélange contenant 0,9 g du composé obtenu à l'étape D, 327 mg d'orthovalérate de méthyle et 2 gouttes d'acide acétique. Le milieu réactionnel est repris par 100 ml d'acétate d'éthyle puis on lave par une solution saturée de bicarbonate de sodium, une solution saturée de chlorure de sodium puis on sèche sur sulfate de sodium et évapore l'acétate d'éthyle. Le résidu obtenu est chromatographié sur silice en éluant par un mélange acétate d'éthyle/toluène (2/1, v/v). On obtient 550 mg du produit attendu sous forme de cire.
   Spectre RMN :
   7,05-7,60 ppm : m : 8 H : H aromatiques
   4,63 ppm : s : 2 H : CH₂ - C₆H₄-
   3, 85 - 3, 55 ppm : m : 4 H, CH₂ en 2 et 6 du tétrahydropyranne
   2,30 ppm : t : 2 H : CH₂ - C₃H₇
   1,05-1,80 ppm : m : 8 H : CH₂-CH₂-CH₂-CH3. et CH₂ en 3 et 5 du tétrahydropyranne
   1,03 ppm : s : 9 H : tBu
   0,75 ppm : t : 3 H : (CH₂)₃-CH₃
      - IR ( CHCl₃)
         1710-1720 cm⁻¹ : C = 0, C = 0
         1625 cm⁻¹ : C = N
F) n-butyl-2 (spirotétrahydropyranne-4)-4 [(*tert*-butoxycarbonyl-2' biphényl-4-yl) méthyl]-1 imidazoline-2 one-5.
   530 mg du produit obtenu à l'étape précédente sont traités par 4 ml de dichlorométhane et 5 ml de TFA pendant 45 minutes. Après évaporation sous vide, le résidu est repris dans l'éther, le précipité formé est filtré, lavé à l'éther puis séché sous vide. On obtient 510 mg du produit attendu. Fc = 159-162°C.
   Spectre RMN :
   7,80-7,10 ppm : m : 8 H : H aromatiques
   4,80 ppm : s : 2 H : CH₂ - C₆H₄-
   4,00-3,75 ppm : m : 4 H, CH₂ en 2 et 6 du tétrahydropyranne
   2,60 ppm : t : 2 H : CH₂ - C₃H₇
   1,45-2,00 ppm : m : 6 H : CH₂-CH₂-CH₂-CH3. et CH₂
      en 3 et 5 du tétrahydropyranne
   1,30 ppm : sext : 2 H : CH₂-CH₂-CH₂-CH₃
   0,80 ppm : t : 3 H : (CH₂)₃-CH₃

### EXEMPLE 7

Trifluoroacétate de n-butyl-2 [(carboxy-2' biphényl-4-yl) méthyl]-1 [spiro(benzyl-1 piperidine-4)]-4 imidazoline-2 one-5.

et n-butyl-2 [spiro (benzyl-1 piperidine-4)]-4 [(*tert*-butoxycarbonyl-2' biphényl-4-yl) méthyl]-1 imidazoline-2 one-5 - Procédé 1
A) L'acide amino-4 benzyl-1 pipéridinecarboxylique-4 est préparé à partir de la N-benzyl pipéridone-4 selon la méthode décrite dans le brevet allemand DE- 2 215 721.
B) Amino-4 benzyl-1 pipéridinecarboxylate d'éthyle-4
   3,80 g du composé préparé à l'étape A sont ajoutés à une solution de 13 g d'acide chlorhydrique dans 50 ml d'éthanol à 0°C puis on porte au reflux pendant 5 heures. Après concentration sous vide, le résidu est lavé à l'éther puis dissous dans un mélange éther-eau auquel on ajoute une solution saturée de carbonate de potassium pour atteindre pH 9. La phase éthérée est décantée, lavée par une solution saturée de chlorure de sodium, séchée sur sulfate de sodium puis évaporée à sec. On obtient 3,50 g du produit attendu sous forme d'huile.
   Spectre RMN :
   7,20-7,40 ppm : m : 5 H : H aromatiques
   4,10 ppm : q : 2 H : CH₂ -CH₃
   3,45 ppm : s : 2 H : CH₂ du benzyle
   2,25-2,60 ppm : m : 4 H : CH₂ en 2 et 6 de pipéridine 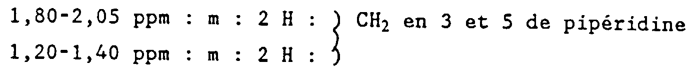
   1,12 ppm : t : 3 H : CH₃-CH₂-
C) n-butyl-2 [spiro(benzyl-1 piperidine-4)]-4 imidazoline-2 one-5
   Le valérimidate d'éthyle est préparé comme à l'exemple 2, étape A. On mélange 2,06 g de valérimidate d'éthyle, 3,40 g du composé préparé à l'étape B et 8 gouttes d'acide acétique, dans 15 ml de xylène et l'on chauffe à reflux pendant 6 heures. Après concentration sous vide, le résidu est chromatographié sur gel de silice en éluant par un mélange chloroforme/méthanol/acide acétique (82/15/3, v/v/v). On obtient 2,80 g du produit attendu après extraction par le chloroforme à pH 9 pour éliminer l'acide acétique. Fc = 170-172°C
   - IR (chloroforme)
      1725 cm⁻¹ C = 0
      1640 cm⁻¹ C = N

      Spectre RMN :
      7,10-7,30 ppm : m : 5 H : H aromatiques
      3,45 ppm : s : 2 H : -CH₂-C₆H₅
      1,10-2,75 ppm : 5 m, 14 H : CH₂ en 2,3,5,6 de pipéridine et (CH₂)₃-CH₃
      0,80 ppm : t : 3 H : (CH₂)₃-CH₃
D) n-butyl-2 [spiro (benzyl-1 piperidine-4)]-4 [(*tert*-butoxycarbonyl-2' biphényl-4-yl) méthyl]-1 imidazoline-2 one-5
   A 2,78 g du composé obtenu à l'étape C, dissous dans 25 ml de DMF, on ajoute 513 mg de méthylate de sodium et, après 15 minutes, 4,16 g de bromométhyl-4 (*tert*-butoxycarbonyl-2') biphényle. On chauffe à 40°C pendant 5 heures puis le milieu réactionnel est repris par 300 ml d'acétate d'éthyle, 50 ml d'eau et 5 ml de solution saturée de bicarbonate de sodium. La phase organique est décantée, relavée une fois par une solution saturée de chlorure de sodium, séchée sur sulfate de sodium et évaporée sous vide. Le résidu est chromatographié sur silice en éluant par un mélange acétate d'éthyle/méthanol (95/5, v/v). On obtient-0,98 g du produit attendu. Fc : 103-106°C
      - IR (CHCl₃)
         1710-1725 cm⁻¹ C = O, C = O (imidazoline, ester)
         1630 cm⁻¹ C = N

         Spectre RMN :
         7,70-7,10 ppm : m : 13 H : H aromatiques
         4,70 ppm : s : 2 H : CH₂-C₆H₄-
         3,55 ppm : s : 2 H : CH₂-C₆H₅
         1,20-2,75 ppm : 5 m : 14 H : CH₂ en 2,3,5,6, de la pipéridine et (CH₂)₃-CH₃
         1,15 ppm : s : 9 H : tBu
         0,85 ppm : t : 3 H : (CH₂)₃-CH₃
E) Trifluoroacétate de n-butyl-2 [(carboxy-2' biphényl-4-yl) méthyl]-1 [spiro(benzyl-1 pipéridine-4)]-4 imidazoline-2 one-5.
   350 mg du composé obtenu à l'étape D sont dissous dans 4 ml de dichlorométhane et 5 ml de TFA. Après 45 minutes, le milieu est concentré sous vide puis le résidu est repris dans un mélange éther-hexane, le précipité formé est filtré, lavé à l'éther et séché sous vide. On obtient 350 mg du produit attendu. Fc = 198-200°C
   Spectre RMN :
   7,05-7,75 ppm : m : 13 H : H aromatiques
   4,75 ppm : s : 2 H : CH₂-C₆H₄-
   4,40 ppm : s : 2 H : CH₂-C₆H₅
   3,20-3,60 ppm : m : 4 H : CH₂ en 2 et 6, de la pipéridine
   2,35 ppm : t : 2 H : CH₂-CH₂-CH₂-CH₃
   2,20-1,40 ppm : 3 massifs : CH₂ en 3 et 5 de la pipéridine et CH₂-CH₂-CH₂- CH₃
   1,25 ppm : sext : 2 H : CH₂-CH₂-CH₂-CH₃
   0,80 ppm : t : 3 H : (CH₂)₃-CH₃

### EXEMPLE 8

Ditrifluoroacétate de n-butyl-2 [(carboxy-2' biphényl-4-yl méthyl]-1 (spiropipéridine-4)-4 imidazoline-2 one-5.

et butyl-2 [spiropipéridine-4]-4 [(*tert*-butoxycarbonyl-2' biphényl-4-yl) méthyl]-1 imidazoline-2 one-5
A) n-butyl-2 (spiropipéridine-4-)-4 [(*tert*-butoxycarbonyl-2' biphényl-4-yl) méthyl]-1 imidazoline-2 one-5
   300 mg du composé de l'exemple 7, étape D sont dissous dans 10 ml de méthanol. On ajoute 180 mg de Palladium à 10% sur charbon et on hydrogène pendant 3 heures à pression atmosphérique. On filtre la catalyseur et le filtrat est concentré sous vide. On obtient 200 mg du produit attendu.
   Spectre RMN :
   7,20-7,75 ppm : m : 8 H : H aromatiques
   4,75 ppm : s : 2 H : CH₂-C₆H₄-
   3,00-1,70 ppm : 3 massifs pour les 4 CH₂ de la pipéridine
   2,40 ppm : t : 2 H : CH₂-CH₂-CH₂-CH₃
   1,60 ppm : quint : 2 H : CH₂-CH₂-CH₂-CH₃
   1,35 ppm : sext : 2 H : CH₂-CH₂-CH₂-CH₃
   1,20 ppm : s : 9 H : tBu
   0,90 ppm : t : 3 H : (CH₂)₃-CH₃
B) Ditrifluoroacétate de n-butyl-2 [(carboxy-2' biphényl-4-yl) méthyl]-1 (spiropipéridine-4)-4 imidazoline-2 one-5.
   160 mg du produit obtenu à l'étape A sont agités dans 3 ml de dichlorométhane et 4 ml d'acide trifluoroacétique, pendant 45 minutes. On concentre sous vide, le résidu est repris dans l'éther. On obtient une gomme puis une mousse après séchage sous vide (150 mg). Fc = 80-85°C
   Spectre RMN :
   7,15-7,80 ppm : m : 8 H : H aromatiques
   4,75 ppm : s : 2 H : CH₂-C₆H₄-
   3,20-1,60 ppm : 3 massifs : 4 CH₂ de la pipéridine
   2,40 ppm : t : 2 H : CH₂-CH₂-CH₂-CH₃
   1,50 ppm : quint : 2 H : CH₂-CH₂-CH₂-CH₃
   1,30 ppm : sext : 2 H : CH₂-CH₂-CH₂-CH₃
   0,80 ppm : t : 3 H : (CH₂)₃-CH₃

### EXEMPLE 9

Trifluoroacétate de n-butyl-2 [(carboxy-2' biphényl-4-yl) méthyl]-1 diphényl-4,4 imidazoline-2 one-5.

et n-butyl-2 diphényl-4,4 [(*tert*-butoxycarbonyl-2' biphényl-4-yl) méthyl]-1 imidazoline-2 one-5 - Procédé 1
A) Chlorhydrate de valéramidine
   6 g de chlorhydrate de valérimidate d'éthyle sont ajoutés à une solution de 6,75 g d'ammoniac dans 80 ml de méthanol à 0°C. Après 18 h. le milieu réactionnel est concentré sous vide et l'on obtient le produit attendu sous forme d'un solide blanc.
B) n-butyl-2 diphényl-4,4 imidazoline-2 one-5
   Ce composé est préparé selon le mode opératoire décrit pas J. NYITRAI et K. LEMPERT dans Tetrahedron, 1969, 25, 4265-4275, à partir du benzile et du chlorhydrate de valéramidine. Fc = 135°C
   - IR (CHCl₃)
      1725 cm⁻¹ C = 0
      1640 cm⁻¹ C = N

      Spectre RMN :
      7,20-7,50 ppm : m : 10 H : H aromatiques
      2,50 ppm : t : 2 H : CH₂-CH₂-CH₂-CH₃
      1,65 ppm : quint : 2 H : CH₂-CH₂-CH₂-CH₃
      1,35 ppm : sext : 2 H : CH₂-CH₂-CH₂-CH₃
      0,90 ppm : t : 3 H : CH₂-CH₂-CH₂-CH₃
      11 ppm : s.e. : NH
C) n-butyl-2 diphényl-4,4 [(*tert*-butoxycarbonyl-2' biphényl-4-yl) méthyl]-1 imidazoline-2 one-5.
   Ce composé est préparé selon le procédé habituel par action sur le composé préparé à l'étape B du bromométhyl-4 (*tert*-butoxycarbonyl-2') biphényle, en présence de méthylate de sodium dans le DMF.
   - IR (CHCl₃)
      1715-1725 cm⁻¹ C = 0, C = O (ester, imidazolinone)
      1635 cm⁻¹ C = N

      Spectre RMN :
      7,25-7,80 ppm : m : 18 H : H aromatiques
      4,85 ppm : s : 2 H : N-CH₂-C₆H₄-
      2,60 ppm : t : 2 H : CH₂-CH₂-CH₂-CH₃
      1,75 ppm : quint : 2 H : CH₂-CH₂-CH₂-CH₃
      1,40 ppm : sext : 2 H : CH₂-CH₂-CH₂-CH₃
      1,15 ppm : s : 9 H : tBu
      0,90 ppm : t : 3 H : CH₃ du n-butyle
D) Trifluoroacétate de n-butyl-2 [(carboxy-2' biphényl-4-yl) méthyl]-1 diphényl-4,4 imidazoline-2 one-5.
   500 mg du produit préparé à l'étape C sont traités par 2,5 ml de dichlorométhane et 2,5 ml d'acide trifluoroacétique à 20°C, pendant 40 minutes. Après concentration sous vide, le résidu est repris par un mélange éther-hexane, le précipité formé est filtré, lavé à l'hexane et séché. On obtient 440 mg du produit attendu. Fc = 55-60°C
   Spectre RMN :
   7,15-7,80 ppm : m : 18 H : H aromatiques
   4,85 ppm : s : 2 H : N-CH₂-C₆H₄-
   2,60 ppm : t : 2 H : CH₂-CH₂-CH₂-CH₃
   1,70 ppm : quint : 2 H : CH₂-CH₂-CH₂-CH₃
   1,40 ppm : sext : 2 H : CH₂-CH₂-CH₂-CH₃
   0,90 ppm : t : 3 H : CH₃ du butyle

### EXEMPLE 10

Trifluoroacétate de n-butyl-2 [(carboxy-2' biphényl-4-yl) méthyl]-3 spirocyclopentane-6 dihydro-5,6 1H pyrimidinone-4.
A) Acide (amino-1 cyclopentyl) acétique.
   L'acide cyclopentylidène acétique est préparé selon G.A.R. KON et R.P. LINSTEAD, J. Chem. Soc., 1925, 127, 616. Dans un autoclave, on place 740 mg de cet acide et 5 ml d'ammoniaque à 20% et on chauffe à 150°C pendant 24 heures. Après évaporation des solvants, on chromatographie le résidu sur une colonne de silice en éluant par un mélange DCM-méthanol-solution aqueuse d'ammoniaque à 20% (70/30/1, v/v/v). On obtient 330 mg de l'acide attendu.
B) (Amino-1 cyclopentyl) acétate d'éthyle
   On dissout 330 mg d'acide dans 10 ml d'éthanol. On refroidit dans un bain de glace et on sature par de l'acide chlorhydrique gazeux. Après 24 heures à reflux, on évapore le milieu réactionnel, reprend le résidu par une solution de carbonate de sodium et extrait à l'acétate d'éthyle puis on sèche sur sulfate de sodium, filtre et évapore. On obtient 312 mg de l'ester attendu.
C) n-butyl-2 spirocyclopentane-6 dihydro-5,6 1H pyrimidinone-4
   On porte à reflux un mélange contenant 310 mg du composé obtenu à l'étape B, 348 mg de valérimidate d'éthyle, 10 ml de xylène et 6 gouttes d'acide acétique. On ajoute à nouveau, après 2 heures et 18 heures, 348 mg de valérimidate d'éthyle et après 24 heures de reflux au total, on évapore le milieu réactionnel puis on chromatographie sur silice en éluant par un mélange DCM-méthanol (97/3, v/v). On obtient 153 mg du produit attendu.
D) n-butyl-2-spirocyclopentane-6-[(*tert*-butoxycarbonyl-2' biphényl-4-yl) méthyl]-3 dihydro-5,6 1 H pyrimidinone-4.
   On prépare sous atmosphère d'azote un mélange de 10 ml de DMF et de 40 mg d'hydrure de sodium à 80% dans l'huile. On ajoute goutte à goutte, à température ambiante, 144 mg du composé préparé à l'étape C, dissous dans 5 ml de DMF. Après 30 minutes sous agitation, on ajoute 288 mg de bromométhyl-4 *tert*-butoxycarbonyl-2' biphényle dissous dans 5 ml de DMF. On laisse 2 heures sous agitation puis on évapore, réprend le résidu par de l'eau et extrait à l'acétate d'éthyle. On sèche sur sulfate de sodium, filtre et évapore, puis on purifie par chromatographie sur colonne, en éluant par un mélange hexane-acétate d'éthyle (85/5, v/v). On obtient 174 mg du produit attendu.
E)
   On refroidit 10 ml d'acide trifluoroacétique par un bain d'eau glacée et l'on ajoute 161 mg du composé préparé à l'étape D. On laisse sous agitation pendant 30 minutes puis on évapore. On reprend le résidu par de l'éther éthylique puis on évapore à nouveau. Cette opération est répétée puis on sèche le résidu sous vide. On obtient 140 mg du composé attendu, sous forme de poudre amorphe. Fc = 108-115°C
   Spectre RMN :
   0,9 ppm : t : 3 H : (CH₂)₃-CH₃
   1,1 à 2,1 ppm : m : 12 H : cyclopentane et CH₂-CH₂-CH₂-CH₃
   2,7 ppm : t : 2 H : CH₂-CH₂-CH₂-CH₃
   3,1 ppm : s : 2 H : -CH₂-CO
   5,1 ppm : s : 2 H : N-CH₂-C₆H₅
   7,2 à 7,8 ppm : m : 8 H : H aromatiques

### EXEMPLE 11

n-butyl-2 spirocyclopentane-4 [(*tert*-butoxycarbonyl-2' biphényl-4-yl) méthyl]-1 imidazoline-2 thione-5

et trifluoroacétate n-butyl-2 [(carboxy-2' biphényl-4-yl) méthyl]-1 spirocyclopentane-4 imidazoline-2 thione-5.
A) n-butyl-2 spirocyclopentane-4 [(*tert*-butoxycarbonyl-2' biphényl-4-yl) méthyl]-1 imidazoline-2 thione-5.
   5,63 g du composé, préparé à l'exemple 1, étape D, sont mis en solution dans 40 ml de toluène anhydre et traité à 80°C sous azote par 3 g de réactif de Lawesson. Après 6 heures, la réaction est filtrée et concentrée. On chromatographie sur silice en éluant par un mélange DCM-acétate d'éthyle (95/5, v/v). On obtient le produit attendu sous forme d'une huile qui cristallise à froid. m = 4,5 g. Fc = 77-79°C.
   Spectre RMN :
   0,90 ppm : t : 3 H : CH₃ (n-Bu)
   1,20 ppm : s : 9 H : tBu
   1,35 ppm : sext : 2 H : CH₃-CH₂-
   1,60 ppm : quint : 2 H : CH₃-CH₂-CH₂-
   1,80-2,10 ppm : m : 8 H : cyclopentane
   2,60 ppm : t : 2 H : CH₃-CH₂-CH₂-CH₂
   5,35 ppm : s : 2 H : CH₂-C₆H₄-
   7,25-7,80 ppm : m : 8 H : H aromatiques
B) trifluoroacétate n-butyl-2 [(carboxy-2' biphényl-4-yl) méthyl]-1 spirocyclopentane-4 imidazoline-2 thione-5.
   225 mg du composé obtenu à l'étape A sont traités par 5 ml de DCM et 5 ml de TFA pendant 30 minutes. Après concentration, le résidu est repris dans l'éther. Le composé attendu est obtenu sous forme d'une poudre jaune qui est essorée puis rincée par de l'hexane. m = 160 mg. Fc = 185-190°C
   - Spectre de masse : MH⁺ : 421
      Spectre RMN :
      0,78 ppm : t : 3 H : CH₃ (n-Bu)
      1,20 ppm : sext : 2 H : CH₃-CH₂
      1,50 ppm : quint : 2 H : CH₃-CH₂-CH₂-
      1,75-2,00 ppm : m : 8 H : cyclopentane
      2,40 ppm : t : 2 H : CH₃-CH₂-CH₂-CH₂
      5,20 ppm : s : 2 H : CH₂-C₆H₄-
      7,00-7,65 ppm : m : 8H: H aromatiques

### EXEMPLE 12

n-butyl-2 (spiroindane-2)-4 [(*tert*-butoxycarbonyl-2' biphényl-4-yl) méthyl]-1 imidazoline-2 one-5.

et n-butyl-2 [(carboxy-2' biphényl-4-yl) méthyl]-1 (spiroindane-2)-4 imidazoline-2 one-5 - Procédé 1.
A) L'acide amino-2 indane-2 carboxylique est préparé selon R.M. Pinder, J. Med. Chem., 1971, 14, 9, 892, et l'ester éthylique correspondant est ensuite préparé selon Adkins (réf. citée à l'exemple 2A).
B) n-butyl-2 (spiroindane-2)-4 imidazoline-2 one-5.
   2,78 g d'ester éthylique, préparé à l'étape A et 2,5 g de valérimidate d'éthyle sont mis en solution dans 20 ml de xylène en présence de 60 µl d'acide acétique et portés à reflux pendant 3 heures. On rajoute 500 mg de valérimidate d'éthyle et maintient le reflux pendant 3 heures supplémentaires. Le milieu réactionnel est concentré puis chromatographié sur silice en éluant par un mélange hexane-acétate d'éthyle-acide acétique (3/8/0,3 ; v/v/v). Les fractions pures sont réunies et évaporées avec du toluène. On obtient 3,07 g du produit attendu sous forme d'un solide blanc. Fc = 148-150°C.
   Spectre RMN :
   0,90 ppm : t : 3 H : CH₃ (n-Bu)
   1,2-1,7 ppm : m : 4 H : CH₂-CH₂-CH₃
   2,4 ppm : t : 2 H : CH₂-(CH₂)₂-CH₃
   2,8-3,2 ppm : q : 4 H : 2CH₂ (indane)
   4,90 ppm : s, 2 H : CH₂-C₆H₄-
   7,2 ppm : m : 4 H : H aromatiques
C) n-butyl-2 (spiroindane-2)-4 [(*tert*-butoxycarbonyl-2' biphényl-4-yl) méthyl]-1 imidazoline-2 one-5.
   Le composé obtenu à l'étape précédente est mis en solution dans 20 ml de DMF anhydre et traité par 450 mg de méthylate de sodium sous azote. Après 20 minutes à température ambiante, on ajoute 3,6 g de bromométhyl-4 (*tert*-butoxycarbonyl-2') biphényle et on laisse sous agitation à 40°C pendant 6 heures. Le milieu réactionnel est concentré puis on effectue les lavages habituels et on chromatographie sur silice en éluant par dichlorométhane-acétate d'éthyle (95/5, v/v) le composé attendu sous forme d'une mousse (m = 1,84 g)
   Spectre RMN
   0,80 ppm : t : 3 H : CH₃(n-Bu)
   1,20 ppm : s : 9 H : tBu
   1,20-1,60 ppm : m : 4 H : CH₂-CH₂-CH₃
   2,40 ppm : t : 2 H : CH₂-(CH₂)₂-CH₃
   2,9-3,3 ppm : q : 4 H : 2CH₂ (indane)
   4,80 ppm : s : 2 H : N-CH₂-C₆H₄-
   7,20-7,80 ppm : m : 12 H : H aromatiques
D) n-butyl-2 [(carboxy-2' biphényl-4-yl) méthyl]-1 (spiroindane-2)-4 imidazoline-2 one-5.
   1,71g du composé obtenu à l'étape précédente est mis en solution dans 15 ml de DCM et traité par 20 ml de TFA. Après 30 minutes, le milieu réactionnel est concentré puis repris par de l'éther. Après trituration, le solide obtenu est essoré, rincé par de l'éther et séché. On obtient 1,42 g du produit attendu. Fc = 217-218°C
   Spectre RMN :
   0,70 ppm : t : 3 H : CH₃ (n-Bu)
   1,10-1,50 ppm m : 4 H : CH₂-CH₂-CH₃
   2,30 ppm : t : 2 H : CH₂-(CH₂)₂-CH₃
   2,8-3,3 ppm : q : 4 H : 2CH₂ (indane)
   4,70 ppm : s : 2 H : N-CH₂-C₆H₄-
   7,1-7,7 ppm : m : 12 H : H aromatiques

D'autres composés selon l'invention ont été préparés selon l'un des procédés décrit ci-dessus. Ils sont rassemblés dans le tableau 1. La structure de chacun de ces composés est conforme à l'analyse de leur spectre RMN.

### EXEMPLE 13

n-butyl-2 [((imidazolyl-1 carbonyl)-2' biphényl-4-yl) méthyl]-1 spirocyclopentane-4 imidazoline-2 one-5.
(I : 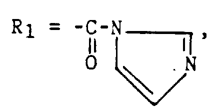 R₂ = H, R₃ = n-C₄H₉,
CR₄R₅ = cyclopentane, X = O)

On agite à température ambiante pendant 72 heures un mélange contenant 404 mg du composé préparé à l'exemple 1 étape E, 15 ml de THF et 260 mg de carbonyldiimidazole. Le milieu réactionnel est évaporé, repris par de l'acétate d'éthyle, lavé par de l'eau puis par une solution de chlorure de sodium, on obtient 420 mg de produit qui sont purifiés par chromatographie sur silice en éluant avec un mélange DCM-acétate d'éthyle (70/30, v/v) pour obtenir le composé attendu.
m = 230 mg
Fc = 120°C

### EXEMPLE 14

n-butyl-2 [((cyano-3 méthyl-2 isothiouréidométhyl)-2' biphényl-4-yl) méthyl]-1 spirocyclopentane-4 imidazoline-2 one-5.
(I : 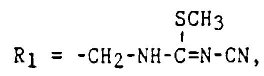 R₂ = H, R₃ = n-C₄H₉,
CR₄ R₅ = cyclopentane, X = O)
A) [(aminométhyl-2' biphényl-4-yl) méthyl]-1 n-butyl-2 spirocyclopentane-4 imidazoline-2 one-5.
   Ce composé est obtenu par hydrogénation du composé, préparé à l'exemple 5.
   1 g du composé, préparé à l'exemple 5 étape A, est placé dans 15 ml de méthanol absolu et 2,3 ml d'éthanol en présence de (0,5 g) Palladium sur charbon à 5 % et on hydrogène à température ambiante pendant 24 heures. Après traitement on obtient 730 mg du produit attendu sous forme huileuse.
B)
   On porte à reflux pendant 24 heures un mélange contenant 300 mg du composé préparé à l'étape précédente et 113 mg de N-cyanimido-S,S-diméthyl-dithiocarbonate dans 3 ml d'éthanol. Après le traitement habituel, le milieu réactionnel est purifié par chromatographie sur silice en éluant par un mélange DCM-acétate d'éthyle (50/50, v/v). Le produit attendu est isolé sous forme d'un solide blanc.

m = 307 mg
Fc = 83°C

### EXEMPLE 15

n-butyl-2 [((cyano-2 guanidinométhyl)-2' biphényl-4-yl) méthyl]-1 spirocyclopentane-4 imidazoline-2 one-5.
(I : 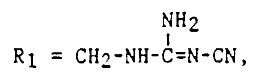 R₂ = H, R₃ = n-C₄H₉,
CR₄R₅ = cyclopentane, X = 0)

Ce composé est obtenu à partir du composé, préparé à l'exemple précédent. On met 200 mg du composé dans 10 ml d'éthanol absolu, on sature par de l'ammoniac vers 10°C, puis on chauffe à 80°C en autoclave pendant 1 nuit. Après concentration à sec du milieu réactionnel, on effectue une chromatographie sur silice en éluant par un mélange DCM-méthanol (95/5, v/v). On obtient 130 mg du produit attendu.
Fc = 100°C.

### EXEMPLE 16

trifluorométhylsulfonate de n-butyl-2 spirocyclopentane-4 [ (trifluorométhylsulfonylamino-2' biphényl-4-yl) méthyl]-1 imidazoline-2 one-5.
(I : R₁ = -NHSO₂CF₃, R₂ = H, R₃ = n-C₄H₉, CR₄R₅ = cyclopentane, X = O)
A) méthyl-4 nitro-2' biphényle.
   On mélange 11,2 g de nitro-2 bromobenzène et 15 g de iodo-4 toluène, on chauffe à 195°C et on laisse sous agitation à cette température pendant 3 heures et demie. Après retour à température ambiante, on reprend par du DCM, porte à reflux et filtre la solution chaude sur célite ^{R} puis on évapore le DCM.
   m = 6,5 g
   Eb = 80-120°C sous 0,2 mm Hg, n_{D}²⁴ = 1,6042.
B) bromométhyl-4 nitro-2' biphényle.
   On porte à reflux pendant 5 heures un mélange contenant 6,5 g de méthyl-4 nitro-2' biphényle, 5,42 g de NBS, 118 mg de azo-bis isobutyronitrile et 500 ml de tétrachlorocarbone. On refroidit à 0°C, essore et concentre le filtrat pour obtenir 9 g d'un produit huileux utilisé tel quel à l'étape suivante.
C) n-butyl-2 [(nitro-2' biphényl-4-yl) méthyl]-1 spirocyclopentane-4 imidazoline-2 one-5.
   On prépare un mélange contenant 260 mg d'hydrure de sodium à 80 % dans 5 ml de DMF et l'on ajoute à température ambiante sous azote 500 mg de n-butyl-2 spirocyclopentane-4 imidazoline-2 one-5, préparé à l'exemple 2 étape A. Après 15 minutes sous agitation, on ajoute 901 mg de bromométhyl-4 nitro-2' biphényle dans 5 ml de DMF et on maintient sous agitation pendant 24 heures. Le milieu réactionnel est concentré à sec, repris par un mélange eau-acétate d'éthyle. La phase organique est décantée, séchée sur sulfate de sodium et filtrée puis on évapore l'acétate d'éthyle. Le produit obtenu est chromatographié sur silice en éluant par un mélange DCM/acétate d'éthyle (9/1, v/v). On obtient 500 mg du produit attendu.
D) [(amino-2' biphényl-4-yl) méthyl]-1 n-butyl-2 spirocyclopentane-4 imidazoline-2 one-5.
   450 mg du produit obtenu à l'étape précédente sont placés dans 10 ml de méthanol, en présence de Palladium sur charbon à 5 %, à température ambiante pour être hydrogénés. Après filtration du catalyseur et évaporation, on obtient 240 mg du produit attendu.
E)
   Dans 4 ml de DCM, on mélange 225 mg du produit obtenu à l'étape précédente, 0,1 ml de triéthylamine et l'on ajoute, sous argon, à -78°C, 0,2 ml d'anhydride trifluorométhylsulfonique puis on laisse revenir à température ambiante. Le milieu réactionnel est lavé par de l'eau, une solution de carbonate acide de sodium, puis séché et concentré. On obtient 150 mg d'un solide blanc amorphe.
   Spectre RMN
   0,4-1,3 ppm : m, 7 H : CH₃-CH₂-CH₂-
   1,4-2,3 ppm : m, 10 H : CH₃-CH₂-CH₂-CH₂ et cyclopentane
   4-4,8 ppm : système AB, 2H : N-CH₂-C₆H₄-
   7-7,6 ppm : m, 8 H : aromatiques
   8,3 ppm : s, 1 H : -NH
   10 ppm : s.e., 1 H : CF₃SO₃H

### EXEMPLE 17

Trifluorométhylsulfonate de n-butyl-2 spirocyclopentane-4 [(trifluorométhylsulfonylaminométhyl-2' biphényl-4-yl) méthyl]-1 imidazoline-2 one-5.
(I : R₁ = CH₂NHSO₂CF₃, R₂ = H, R₃ = n-C₄H₉, CR₄R₅ = cyclopentane, X = O)

La préparation est réalisée à partir de l'[(aminométhyl-2' biphényl-4-yl) méthyl]-1 n-butyl-2 spirocyclopentane-4 imidazoline-2 one-5, préparé à l'exemple 14 étape A. 322 mg de ce composé et 0,122 ml de triéthylamine sont placés dans 3,4 ml de DCM à -70°C et l'on ajoute 0,294 ml d'anhydride trifluorométhylsulfonique. On laisse revenir à température ambiante, verse dans de l'acide acétique dilué, on extrait par DCM, on sèche sur sulfate de sodium, filtre et évapore le DCM. Le résidu est chromatographié 2 fois sur silice en éluant par DCM-acétate d'éthyle (95/5, v/v) puis 99,5/0,5 ; v/v).

On obtient m = 90 mg
Fc = 90°C

Spectre RMN
0,4-1,2 ppm : m, 7 H : -CH₂-CH₂-CH₃
1,3-2,45 ppm : m, 10 H : CH₂-CH₂-CH₂-CH₃ et cyclopentane
4,1-5 ppm : m 4 H : N-CH₂-C₆H₄- et NH-CH₂-C₆H₄-
7,1-7,7 ppm : m, 8 H : H aromatiques
8,4 ppm : s, 1 H : NH

### EXEMPLE 18

n-butyl-2 [((N-hydroxycarbamoyl)-2' biphényl-4-yl) méthyl]-1 spirocyclopentane-4 imidazoline-2 one-5.
(I : R₁ = -CO-NHOH, R₂ = H, R₃ = n-C₄H₉, CR₄R₅ = cyclopentane, X = O)

Le composé préparé à l'exemple 2, est libéré de son sel d'acide trifluoroacétique en reprenant ce composé dans un mélange acétate d'éthyle-eau et en amenant la solution à pH 6 par addition d'une solution saturée de carbonate acide de sodium. La phase organique est lavée par une solution de chlorure de sodium saturée, séchée sur sulfate de sodium, filtrée et concentrée pour donner la base libre sous forme de solide blanc.

450 mg de ce composé sont mis en solution dans du chloroforme, on ajoute 860 ml de chlorure de thionyle à 0°C et on laisse sous agitation à température ambiante pendant 2 heures. La solution est concentrée et les traces de chlorure de thionyle éliminées par distillation azéotropique avec du toluène. Le chlorure d'acide ainsi obtenu est ajouté goutte à goutte en solution dans le DMF à une solution contenant 200 mg de chlorhydrate d'hydroxylamine et 700 µl de DIPEA dans 10 ml de DMF. Après 2 heures à 0°C, le milieu réactionnel est concentré, repris par 100 ml de DCM et 50 ml d'eau. On amène à pH 7, extrait la phase organique puis on la sèche sur sulfate de sodium. Après filtration, la solution est concentrée. Le produit obtenu est recristallisé dans un mélange acétate d'éthyle-éther éthylique-hexane.
m = 360 mg
Fc = 85°C

### EXEMPLE 19

n-butyl-2 spirocyclopentane-4 [(ureido-2' biphényl-4-yl) méthyl]-1 imidazoline-2 one-5.
(I : R₁ = NHCONH₂, R₂ = H, R₃ = n-C₄H₉, CR₄R₅ = cyclopentane, X = O)

Ce composé est préparé en utilisant la méthode décrite par B.B. Kobu et al. dans Org. Synth., 1957, 37, 52 à partir de l'[(amino-2' biphényl-4-yl) méthyl]-1 n-butyl-2 spirocyclopentane-4 imidazoline-2 one-5, préparé à l'exemple 14 étape A.

1 g de ce dernier est mis en solution dans 50 ml d'acide chlorhydrique 6N et traité par de l'isocyanate de potassium pendant 1 heure à 5°C. Le milieu réactionnel est concentré, repris par de l'acétate d'éthyle, lavé par du carbonate acide de sodium puis par une solution saturée de chlorure de sodium. Après séchage sur sulfate de sodium et filtration, la solution est concentrée et l'huile obtenue est purifiée par chromatographie sur silice en éluant par un mélange DCM-méthanol (9/1, v/v).
m = 600 mg

Spectre RMN
0,85 ppm : t, 3 H : CH₂-CH₃
1,35 ppm : sext, 2 H : CH₂-CH₃
1,6 ppm : quint, 2 H : CH₂-CH₂-CH₃
1,7-2 ppm : m, 8 H : cyclopentane
2,45 ppm : t, 2 H : CH₂-CH₂-CH₂-CH₃
4,8 ppm : s, 2 H : -CH₂-C₆H₄-
6,05 ppm : s, 2 H : NH₂
7-8 ppm : m, 9 H : 8 H aromatiques + NHCO

### EXEMPLES 20 ET 21

[(carboxy-2' biphényl-4-yl) méthyl]-1 n-propyl-2 spirocyclohexane-4 imidazoline-2 one-5.

et [(N-cyanocarbamoyl -2' biphényl-4-yl) méthyl]-1 n-propyl-2 spirocyclohexane-4 imidazoline-2 one-5.
(I : R₁ = CO-NH-CN, R₂ = H, R₃ = n-C₃H₇, CR₄R₅ = cyclohexane, X = O).
A) Chlorhydrate de butyrimidate d'éthyle 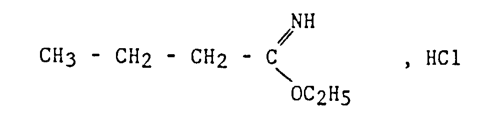
   Ce composé est préparé selon Mc Elvain (J. Amer. Chem. Soc., 1942, 64, 1825-1827).
   A une solution de 10,6 g de gaz chlorhydrique dans 20 ml d'éthanol anhydre, on ajoute à 0°C, 23 ml de butyronitrile puis, après abandon du milieu réactionnel 4 jours à 0°C, on le verse, sous agitation, sur 200 ml d'éther anhydre à 0°C ; le précipité formé est filtré, lavé à l'éther puis séché sous vide. On obtient 25,8 g du produit attendu.
B) Butyrimidate d'éthyle.
   16 g d'imidate obtenu à l'étape A sont dissous dans 100 ml de dichlorométhane et 50 ml d'eau et on ajoute 15 g de carbonate de potassium. Après décantation, le dichlorométhane est séché sur carbonate de potassium puis évaporé à sec sans chauffer.
C) Ester éthylique de l'acide amino-1 cyclohexane.
   L'acide amino-1 cyclohexane carboxylique est commercial. 15 g de cet aminoacide sont ajoutés à 0°C à une solution de 23 g de gaz chlorhydrique dans 150 ml d'éthanol anhydre. On chauffe au reflux pendant 5 heures, puis on concentre à sec le milieu réactionnel et on le reprend par de l'éther. Le solide blanc obtenu est filtré, lavé à l'éther puis dissous dans un mélange de 300 ml d'éther et 100 ml d'eau. On amène à pH 9 par addition d'une solution de carbonate de potassium. La phase organique est décantée, lavée par une solution saturée de chlorure de sodium, séchée sur sulfate de sodium puis évaporée à sec. On obtient 14 g du produit attendu sous forme d'une huile.
D) n-propyl-2 spirocyclohexane-4 imidazoline-2 one-5.
   14 g du produit obtenu à l'étape C sont dissous dans 200 ml de xylène contenant 0,6 ml d'acide acétique. On ajoute la moitié de l'imidate obtenu à l'étape B et on chauffe à reflux. Après 1 heure et demie, on ajoute la moitié de l'imidate restant puis le dernier quart après 4 heures. Après 7 heures de reflux au total, le milieu est évaporé à sec. Le solide obtenu est repris dans de l'hexane, filtré, lavé à l'éther puis séché.
   On obtient 10,3 g de l'imidazolinone attendue.
   Fc = 124-125°C.
      - IR (CHCl₃)
         1715 cm⁻¹ : C = O
         1635 cm⁻¹ : C = N

         Note : Le composé présent dans la solution est bien une imidazolinone-5, d'après les valeurs des bandes IR.
E) n-propyl-2 spirocyclohexane-4 [(*tert*-butoxycarbonyl-2' biphényl-4-yl) méthyl]-1 imidazoline-2 one-5.
   0,24 g d'hydrure de sodium à 80 % dans l'huile, en suspension dans 10 ml de diméthylformamide, on ajoute 970 mg d'imidazolinone obtenue à l'étape D. Après 20 minutes d'agitation sous azote, on ajoute en 5 minutes 1,91 g de bromométhyl-4 tertbutoxycarbonyl-2' biphényle, préparé selon la demande de brevet européen 324 377. Après 1 heure d'agitation, le milieu est concentré sous vide de moitié et repris par 100 ml d'acétate d'éthyle puis par 20 ml d'eau. La phase organique est décantée, lavée par une solution saturée de chlorure de sodium, séchée sur sulfate de sodium puis concentrée sous vide. Le résidu est chromatographié sur silice en éluant par un mélange acétate d'éthyle-toluène. On obtient 2,10 g du produit attendu sous forme de cire.
   - IR (CHCl₃)
      1705-1715 cm⁻¹ : C = O, C = O (ester, imidazolinone)
      1635 cm⁻¹ : C = N

   L'analyse du spectre RMN confirme la structure.
F) [(carboxy-2' biphényl-4-yl) méthyl]-1 n-propyl-2 spirocyclohexane-4 imidazoline-2 one-5. (Exemple 20).
   1,25 g de l'ester tertiobutylique obtenu à l'étape E sont agités 45 minutes dans un mélange de 11 ml de dichlorométhane et 15 ml d'acide trifluoroacétique. Après concentration sous vide, le résidu est repris dans de l'éther. Le solide formé est filtré, lavé à l'éther puis séché. On obtient 1,04 g de solide blanc.
   Fc = 170-172°C

   Spectre RMN
   7,10-7,80 ppm : m, 8 H : aromatiques
   4,90 ppm : s, 2 H : N-CH₂-C₆H₄-
   2,45 ppm : t, 2 H : CH₃-CH₂-CH₂-
   1,40-1,80 ppm : m, 12 H : spirocyclohexane + CH₃-CH₂-CH₂-
   0,90 ppm : t, 3 H : CH₃-CH₂-CH₂-

   1,60 g du trifluoroacétate obtenu précédemment sont dissous dans 150 ml d'acétate d'éthyle plus 20 ml d'eau. On ajoute de la soude 1N pour obtenir pH 5,0. La phase organique est décantée, lavée par une solution saturée de chlorure de sodium, séchée sur sulfate de sodium puis évaporée à sec. Le résidu solide est repris dans de l'éther éthylique filtré et séché.
   m = 1,14 g
   Fc = 208-210°C.
G) [(N-cyanocarbamoyl -2' biphényl-4-yl) méthyl]-1 propyl-2 spirocyclopentane-4 imidazoline-2 one-5.(Exemple 21).
   A 300 mg du composé préparé à l'étape précédente en suspension dans 5 ml de DCM, on ajoute 0,54 ml de chlorure de thionyle. Après 1 heure et demie, le milieu réactionnel est concentré sous vide, puis évaporé 2 fois avec du benzène. Le chlorure d'acide ainsi obtenu est dissous dans 2 ml de dioxanne et ajouté à 42 mg de cyanamide en solution dans 1 ml de dioxanne contenant 0,2 ml de soude 10 N. Après 1 heure et demie, le milieu réactionnel est dilué par 150 ml d'acétate d'éthyle, 20 ml d'eau et on amène à pH 5 par de l'acide acétique, la phase organique est décantée, lavée par une solution saturée de chlorure de sodium, séchée sur sulfate de sodium puis évaporée à sec. Le résidu est chromatographié sur silice en éluant par un mélange chloroforme/méthanol/acide acétique (90/8/2, v/v/v). On obtient 160 mg du produit attendu sous forme solide.
   - IR (KBr)
      2150 cm⁻¹ C ≡ N
   - spectre de masse
      MH⁺ : 429.

   Spectre RMN
   7,20-7,70 ppm : m, 8 H : aromatiques
   4,75 ppm : s, 2 H : N-CH₂-C₆H₄-
   2,40 ppm : t, 2 H : CH₃-CH₂-CH₂-
   1,30-1,80 ppm : m, 12 H : CH₃-CH₂-CH₂- et spirocyclohexane
   0,85 ppm : t, 3 H : CH₃-CH₂-CH₂

### EXEMPLE 22

[(N-(carboxy-4 thiazol-1,3 yl-2 carbamoyl)-2' biphényl-4-yl méthyl]-1 n-propyl-2 spirocyclohexane-4 imidazoline-2 one-5.
(I : 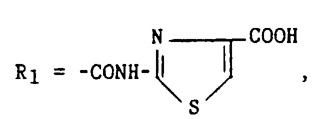 R₂ = H, R₃ = n-C₃H₇,
CR₄R₅ = cyclohexane, X = O

Ce composé est préparé à partir du composé, obtenu à l'exemple 20.

L'amino-2 éthoxycarbonyl-4 thiazole-1,3 est préparé selon B. Plouvier et al., J. Heterocycl. Chem., 1989, 26 (6), 1646.
A) [(N-(carbethoxy-4) thiazol-1,3-2-yl)- acétamido) méthyl]-1 n-propyl-2 spirocyclohexane-4 imidazoline-2 one-5.
   A une solution de 404 mg du composé préparé à l'exemple 20 et 190 mg de dérivé du thiazole dans 4 ml de DCM et 1 ml de DMF, on ajoute 500 mg de BOP et 0,14 ml de triéthylamine. On agite 40 heures à température ambiante puis 7 heures à 50°C. Le milieu réactionnel est repris dans 50 ml d'acétate d'éthyle et on lave 2 fois par une solution KHSO₄-K₂SO₄ puis 2 fois par une solution saturée de bicarbonate de sodium puis 1 fois par une solution saturée de chlorure de sodium. Après séchage sur sulfate de sodium, la phase organique est concentrée sous vide et le résidu est chromatographié sur silice en éluant par un mélange acétate d'éthyle-toluène. On obtient 120 mg du produit attendu.
   Fc = 96-98°C
B)
   A 110 mg du produit obtenu à l'étape précédente, dissous dans 1 ml de méthanol et 1 ml de dioxanne, on ajoute 0,5 ml de soude 2 N. Après 35 minutes d'agitation, le milieu réactionnel est dilué par 10 ml d'eau et 60 ml d'acétate d'éthyle et on amène à pH 5 par addition d'acide chlorhydrique 1 N. La phase organique est décantée, lavée par une solution saturée de chlorure de sodium, séchée sur sulfate de sodium puis concentrée. Le résidu est repris dans l'éther, filtré et séché.
   m = 100 mg
   Fc = 145-148°C

   Spectre RMN
   8,0 ppm : s, 1 H : H en 5 du thiazole
   7,1-7,7 ppm : m, 8 H : H aromatiques
   4,7 ppm : s, 2 H : N-CH₂-C₆H₄-
   2,25 ppm : t, 2 H : CH₂-CH₂-CH₃
   1,2-1,8 ppm : m, 12 H : cyclohexane et CH₂-CH₂-CH₃
   0,85 ppm : t, 3 H : CH₂-CH₂-CH₃

### EXEMPLE 23

n-butyl-2 [((cyano-2 guanidinocarbonyl)-2' biphényl-4-yl) méthyl]-1 spirocyclopentane-4 imidazoline-2 one-5.
(I : 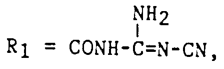 R₂ = H, R₃ = n-C₄H₉,
CR₄R₅ = cyclopentane, X = O).

On prépare le chlorure d'acide du composé obtenu à l'exemple 2 : 1 g de ce composé est placé dans 20 ml de DCM en présence de 1,8 ml de chlorure de thionyle et agité à température ambiante pendant 2 heures. Après concentration du milieu, on reprend par du benzène puis concentre à nouveau. Le produit brut isolé est ensuite utilisé. On le mélange à 417 mg de dicyanodiamide, 0,5 ml de soude 10 N, 0,5 ml d'eau et 10 ml de dioxanne puis on laisse sous agitation pendant 5 heures. Le milieu réactionnel est repris par de l'eau et de l'acétate d'éthyle, on ajoute du carbonate de potassium puis concentre. Le résidu obtenu est chromatographié sur silice en éluant par un mélange DCM-méthanol (95/5, v/v). On isole 100 mg du produit attendu.
Fc = 105°C.

### EXEMPLE 24

trifluoroacétate de benzylidène-4 n-butyl-2 [(carboxy-2') biphényl-4-yl méthyl]-1 imidazoline-2 one-5.
(I : R₁ = CO₂H, R₂ = H, R₃ = n-C₄H₉, R₄R₅ = =CH-C₆H₅, X = O).
A) (benzylidène-1 valérylamino-1 méthylamidométhyl)-4 biphényl-2 *tert-*-butylcarboxylate. 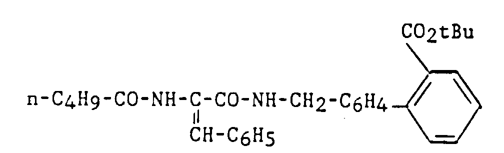
   A partir de la N-Boc α-dehydro (L) phénylalanine, on prépare la N-carboxyanhydride de α-dehydro (L) phénylalanine selon R. Jacquier et al., Tetrahedron Lett., 1984, 25 (26), 2775. p. A 430 mg de ce composé en solution dans 5 ml de THF on ajoute 644 mg de aminométhyl-4 biphényl-2' *tert-*-butyl carboxylate, on agite 2 heures à température ambiante puis on ajoute 1 ml d'orthovalérate de méthyle et évapore à sec, sous vide sans chauffer. le résidu est chauffé 3 heures à 100°C, concentré sous vide, puis chromatographié sur silice en éluant par un mélange hexane-acétate d'éthyle (4/1, v/v). On obtient 580 mg d'un solide blanc.
   Fc = 154°C.

   Spectre RMN
   1,3 ppm : s, 9 H : t-Bu
   0,65 ppm : t, 3 H : CH₃ (n-Bu)
   2 ppm : t, 2 H : CH₃-CH₂-CH₂-CH₂-CO
   4,4 ppm : d, 1 H : CH₂-NH
   6,8 ppm : s, 1 H : CH (=CH-C₆H₅)
B) benzylidène-4 n-butyl-2 [(*tert-*-bytoxycarbonyl-2' biphényl-4-yl) méthyl]-1 imidazoline-2 one-5.
   440 mg du composé obtenu à l'étape A sont dissous dans 1 ml d'acide acétique et chauffés pendant 30 minutes à 100°C.
   On évapore à sec sous vide et chromatographie le résidu sur silice en éluant par un mélange hexane-acétate d'éthyle (4/1, v/v). On obtient 130 mg du produit attendu sous forme huileuse.
   Spectre RMN
   4,9 ppm : s, 2 H : CH₂ ( N-CH₂-C₆H₄-)
C)
   On dissout 100 mg du composé obtenu à l'étape précédente dans 1 ml de DCM et on ajoute 1 ml d'acide trifluoroacétique puis on laisse sous agitation 40 minutes à température ambiante et évapore sous vide. On reprend plusieurs fois par DCM puis évapore. Par addition d'éther éthylique, un solide blanc précipite.
   m = 101 mg
   Fc = 85°C

   spectre de masse
   MH⁺ : 439

   Spectre RMN
   0,82 ppm : t, 3 H : CH₃ (n-Bu)
   1,3 ppm : sext, 2 H : CH₃-CH₂-
   1,6 ppm : m, 2 H : CH₃-CH₂-CH₂-
   2,6 ppm : t, 2 H : CH₃-CH₂-CH₂-CH₂-
   4,82 ppm : s, 2 H : CH₂-C₆H₄-
   7,05 ppm : s, 1 H, =CH-C₆H₅
   7,2-8,2 ppm : m, 13 H : aromatiques

### EXEMPLE 25

benzylidène-4 [(carboxy-2') biphényl-4-yl méthyl]-1 phényl-2 imidazoline-2 one-5.
(I : R₁ = CO₂H, R₂ = H, R₃ = C₆H₅, R₄R₅ = =CH-C₆H₅, X = O).
A) benzylidène-4 phényl-2 oxazolone-5.
   On dissout 1,8 g d'acide hippurique et 0,4 g de bicarbonate de potassium dans 4 ml d'anhydride acétique, on chauffe quelques minutes à 50°C puis on refroidit à température ambiante et l'on ajoute 1,49 g de benzaldehyde. Après 1 heure à température ambiante, on ajoute 20 ml d'eau distillée à 80°C. Le solide qui précipite est essoré, lavé à l'eau, à l'éthanol puis séché. On obtient 1,24 g du produit attendu sous forme d'un solide jaune.
   Fc = 215°C.
   Spectre RMN

   7,4 ppm : s, 1 H : =CH-C₆H₅
   8,1-8,4 ppm : m, 10 H : aromatiques
B) (benzoylamino-1 benzylidène-1 méthylamidométhyl)-4 biphényl-2' *tert-*-butyl carboxylate.
   On chauffe à 110°C pendant 3 heures un mélange contenant 500 mg du composé obtenu à l'étape précédente, 570 mg d'aminométhyl-4 biphényl-2' *tert-*-butyl carboxylate et 10 ml de pyridine. On évapore sous vide, reprend par du chloroforme puis évapore à nouveau. Le résidu est chromatographié sur silice en éluant par un mélange hexane-acétate d'éthyle (3/1 puis 2/1, v/v). On obtient 106 mg du produit attendu sous forme d'un solide jaune.
   Spectre RMN
   1,1 ppm : s, 9 H : t-Bu
   4,35 ppm : t, 2 H : -CH₂-NH
   7,05-7,06 ppm : m, 19 H : H aromatiques + C₆H₅-CH=
   8,65 ppm : t, 1 H : NH-CH₂
   9,9 ppm : s, 1 H : NH-CH=
C)
   On chauffe pendant 6 heures à reflux dans 5 ml d'acide acétique un mélange de 1,2 g du composé obtenu à l'étape précédente et 1,1 g d'acétate de sodium fraîchement fondu. On laisse refroidir, puis on précipite par addition de chloroforme un insoluble. Le filtrat est évaporé et le résidu est chromatographié sur silice en éluant par un mélange chloroforme-méthanol (98/2, v/v). Le solide obtenu est recristallisé dans l'éther éthylique.
   m = 692 mg
   Fc = 120°C

   Spectre RMN
   4,95 ppm : s, 2 H : CH₂-C₆H₄-
   7,1-8,3 ppm : m, 19 H : H aromatiques + =CH-C₆H₅

### EXEMPLES 26 ET 27

n-butyl-2 [((méthyl-2 tétrazol-5-yl)-2' biphényl-4-yl) méthyl]-1 spirocyclopentane-4 imidazoline-2 one-5. (Exemple 26).

et n-butyl-2 [((méthyl-1 tétrazol-5-yl)-2' biphényl-4-yl) méthyl]-1 spirocyclopentane-4 imidazoline-2 one-5. (Exemple 27).

Dans 10 ml de DMF, on mélange 500 mg du composé préparé à l'exemple 5, et 58 mg d'hydrure de sodium, on agite pendant 30 minutes puis on ajoute 179 mg d'iodure de méthyle et 2 ml de DMF et on laisse sous agitation à température ambiante pendant 4 heures. On concentre le milieu réactionnel, reprend à l'eau, puis extrait à l'acétate d'éthyle. On sèche sur sulfate de sodium, filtre et évapore le solvant. Le résidu est chromatographié sur silice en éluant par un mélange hexane-acétate d'éthyle (6/4, v/v). On isole 2 fractions :
90 mg du composé de l'exemple 26
et 184 mg du composé de l'exemple 27

Spectres RMN

Exemple 26
0,7 ppm : t, 3 H : CH₃- (n-Bu)
1,2 ppm : sext, 2 H : CH₃-CH₂-
1,4 ppm : quint, 2 H : CH₃-CH₂-CH₂-
1,5-1,9 ppm : m, 8 H : cyclopentane
2,25 ppm : t, 2 H : CH₃-CH₂-CH₂-CH₂-
4,15 ppm : s, 3 H : N-CH₃
4,6 ppm : s, 2 H : -N-CH₂-C₆H₄-
7 ppm : système AA', BB', 4 H : CH₂-C₆H₄-
7,3-7,75 ppm : m, 4 H : CH₂-C₆H₄-C₆H₄-

Exemple 27
0,7 ppm : t, 3 H : CH₃ (n-Bu)
1,15 ppm : sext, 2 H : CH₃-CH₂-
1,38 ppm : quint, 2 H : CH₃-CH₂-CH₂-
1,5-1,9 ppm : m, 8 H : cyclopentane
2,2 ppm : t, 2 H : CH₃-CH₂-CH₂-CH₂-
3,35 ppm : s, 3 H : N-CH₃
4,6 ppm : s, 2 H : N-CH₂-C₆H₄
7 ppm : système AA', BB', 4 H : N-CH₂-C₆H₄-
7,4-7,8 ppm : m, 4 H : CH₂-C₆H₄-C₆H₄-

### EXEMPLE 28

n-butyl-2 spirocyclopentane-6 [((tétrazolyl-5)-2' biphényl-4-yl) méthyl] -3 4(1H)-dihydro-5,6 pyrimidinone-4.
A) cyclopentylidène acétate d'éthyle.
   Dans 40 ml de benzène, on met 6 g d'hydrure de sodium à 80 % et l'on ajoute goutte à goutte 57,1 ml de triéthyl phosphonoacétate d'éthyle à une température inférieure à 35°C. Après 1 heure à température ambiante, on ajoute goutte à goutte 24,3 ml de cyclopentanone. On chauffe à 65°C pendant 15 minutes puis on refroidit à température ambiante et décante la liqueur surnageante. On ajoute 25 ml de benzène, chauffe à 65°C pendant 15 minutes, refroidit, décante puis on récupère la liqueur surnageante. L'opération est répétée 1 fois. Par évaporation des liqueurs, on obtient 42 g de produit attendu qui est distillé.
   Eb = 102°C sous 11 mm de mercure
   m = 22,8 g
B) (amino-1 cyclopentyl) acétamide.
   On ajoute 150 ml d'ammoniac gazeux à 20 g de cyclopentylidène acétate d'éthyle préparé précédemment et on chauffe à 150°C pendant 72 heures. Le produit obtenu après évaporation est purifié par chromatographie sur silice en éluant par un mélange DCM-méthanol-ammoniaque à 20 % (90/10/1, v/v/v). Le produit obtenu est dissous dans DCM, séché sur sulfate de sodium. On filtre et évapore le DCM pour obtenir 7,2 g de produit attendu.
C) n-butyl-2 spirocyclopentane-6 4(1H)-dihydro-5,6 pyrimidinone-4.
   On chauffe à 100°C pendant 18 heures un mélange contenant 4,57 g d'(amino-1 cyclopentyl) acétamide préparé précédemment, 25 ml d'orthovalérate de méthyle et quelques gouttes d'acide acétique. Après évaporation de l'orthovalérate en excès, le résidu est repris par un mélange acétate d'éthyle-bicarbonate de sodium puis lavé par une solution aqueuse de chlorure de sodium, séché sur sulfate de sodium puis purifié par chromatographie sur silice en éluant par un mélange DCM-méthanol (98/2, v/v).
   m = 5 g

   Spectre RMN
   0,75 ppm : t, 3 H : CH₃ (nBu)
   1,2 ppm : sext, 2 H : CH₃-CH₂-
   1,3-1,8 ppm : m, 10 H : CH₃-CH₂-CH₂ et cyclopentane
   2 ppm : t, 2 H : CH₃-CH₂-CH₂-CH₂-
   2,15 ppm : s, 2 H : CH₂-CO
   9,95 ppm : s.e., 1 H : NH

   Ce composé est celui obtenu à l'exemple 10, étape C.
D) n-butyl-2 spirocyclopentane-4 [(triphénylméthyl tétrazolyl-5)-2' biphényl-4-yl méthyl]-1 pyrimidinone-6.
   On mélange pendant 30 minutes sous azote 327 mg d'hydrure de sodium à 80 % dans 30 ml de DMF et 1,5 g de la pyrimidinone préparée précédemment et l'on ajoute 5,27 g de bromométhyl-4 (triphénylméthyltétrazolyl-5)-2' biphényle. Après 4 heures sous agitation à température ambiante, on évapore les solvants, reprend par de l'acétate d'éthyle et de l'eau, sèche sur sulfate de sodium et concentre. Le produit obtenu est purifié par chromatographie sur silice en éluant par un mélange acétate d'éthyle-hexane (3/7, v/v).
   m = 3,2 g
E)
   3 g du composé obtenu à l'étape précédente sont placés dans 15 ml de méthanol et on refroidit par un bain eau-glace, on ajoute 2,2 ml d'HCl 4N et on laisse 5 heures sous agitation à température ambiante. Après évaporation, on reprend par de l'acétate d'éthyle et de l'eau puis ajoute de la soude pour atteindre un pH basique (pH 11). On laisse décanter, lave la phase aqueuse par de l'éther éthylique et du toluène, puis à nouveau de l'éther. On amène cette phase aqueuse à pH 5 par addition d'acide chlorhydrique dilué puis on extrait par de l'acétate d'éthyle, sèche et concentre. Le produit obtenu est purifié sur silice en éluant par un mélange DCM-méthanol (95/5, v/v). On obtient 800 mg du produit attendu.
   Spectre RMN
   0,85 ppm : t, 3 H : CH₃ (nBu)
   1,30 ppm : sext, 2 H : CH₃-CH₂
   1,40-1,95 ppm : m, 10 H : cyclopentane et CH₂-CH₂-CH₂-CH₃
   2,30 ppm : t, 2 H : CH₂-CH₂-CH₂-CH₃
   2,55 ppm : s, 2 H : CH₂-CO
   4,95 ppm : s, 2 H : N-CH₂-C₆H₄-
   7,05 ppm : m, 4 H : CH₂-C₆H₄-
   7,55-7,82 ppm : m, 4 H : CH₂-C₆H₄-C₆H₄-

### EXEMPLE 29

Trifluoroacétate de n-butyl-2 [(carboxy-2' biphényl-4-yl) méthyl]-3 spirocyclopentane-5 5(1H)-dihydro-5,6 pyrimidinone-4.
A) cyano-1 cyclopentane carboxylate d'éthyle.
   Ce composé est préparé selon Helv. Chim. Acta, 1952, 35 (7), 2561.
   On dissout 9,2 g de sodium dans 200 cm³ d'éthanol absolu. La moitié de la solution d'éthylate de sodium formée est versée dans une ampoule. A la moitié restant on ajoute 24,88 g de cyanoacétate d'éthyle et on porte à reflux.
   Dans une autre ampoule, on verse 43,19 g de dibromo-1,4 butane, on ajoute goutte à goutte et simultanément au milieu réactionnel la solution d'éthylate de sodium et le dibromo-1,4 butane. Après la fin de l'addition on maintient le reflux pendant 2 heures. On évapore, reprend par un mélange éther éthylique-eau, lave par une solution saturée de chlorure de sodium puis sèche. Le produit obtenu distille à 115-120°C sous 11 mm de mercure
   m = 24 g
B) aminométhyl-1 cyclopentane carboxylate d'éthyle.
   Ce composé est préparé par hydrogénation catalytique du cyano-1 cyclopentane carboxylate d'éthyle.
   20 g de cyano-1 cyclopentane carboxylate d'éthyle sont placés dans 200 ml d'éthanol à 10 % d'ammoniac et hydrogénés à 60°C sous une pression de 100 bars en présence de Rhodium sur alumine pendant 72 heures. Après filtration sur cellite ^{R} et évaporation, le résidu est chromatographié sur silice en éluant par un mélange DCM-méthanol-ammoniaque à 20 % (98/2/0,5 ; v/v/v)
   m = 12,8 g
C) n-butyl-2 spirocyclopentane-5 4(1H)-dihydro-5,6 pyrimidinone-4.
   On porte à reflux pendant 13 heures un mélange contenant 13,12 g du composé obtenu à l'étape précédente, et 13,5 g de valérimidate d'éthyle dans 100 ml de xylène contenant quelques gouttes d'acide acétique. On évapore le milieu réactionnel, reprend par de l'acétate d'éthyle et une solution de carbonate de sodium à 10 %, puis sèche et concentre.
   m = 14 g
   Fc = 89-91°C

   Spectre RMN
   0,80 ppm : t, 3 H : CH₃ (nBu)
   1,10-1,80 ppm : m, 12 H : CH₃-CH₂-CH₂- et cyclopentane
   2,05 ppm : t, 2 H : CH₃-CH₂-CH₂-CH₂-
   3,20 ppm : s, 2 H : CH₂ (pyrimidinone)
   10 ppm : 1 H, s : NH-CO
D) n-butyl-2 spirocyclopentane-5 [(*tert*-butoxycarbonyl-2' biphényl-4-yl) méthyl]-3 4(1H)-dihydro-5,6 pyrimidinone-4.
   500 mg du produit obtenu à l'étape précédente sont placés dans 40 ml de DMF, en présence de 115 mg d'hydrure de sodium à 80 % dans l'huile, sous argon et agités à température ambiante pendant une demi-heure. On ajoute 1,08 g de bromométhyl-4 *tert*-butoxycarbonyl-2' biphényle et on maintient 2 heures sous agitation. Après évaporation, le résidu est repris par un mélange acétate d'éthyle-eau, lavé par une solution saturée de chlorure de sodium, puis séché, concentré et chromatographié sur silice en éluant par un mélange acétate d'éthyle-hexane (3/7, v/v)
   m = 280 mg
E)
   250 ml de l'ester terbutylique préparé à l'étape précédente sont dissous dans 10 ml de DCM. On refroidit dans un bain d'eau glacée puis ajoute 5 ml d'acide trifluoroacétique froid et laisse une heure sous agitation à froid, puis 1 heure à température ambiante. On évapore sous pression réduite. Le résidu est repris par de l'éther éthylique puis évaporé. L'opération est répétée 3 fois puis on reprend le résidu d'évaporation par de l'hexane, triture puis décante l'hexane. On reprend par de l'éther éthylique et filtre le précipité.
   m = 190 mg
   Fc = 153-155°C
Spectre RMN
   0,85 ppm : t, 3 H : CH₃ (nBu)
   1,35 ppm : sext, 2 H : CH₃-CH₂-
   1,45-2,20 ppm : m, 10 H : CH₃-CH₂-CH₂- et cyclopentane
   2,80 ppm : t, 2 H : CH₃-CH₂-CH₂-CH₂-
   3,80 ppm : s, 2 H : CH₂ (pyrimidinone)
   5,15 ppm : s, 2 H : N-CH₂-
   7,25 ppm : m, 8 H : aromatiques

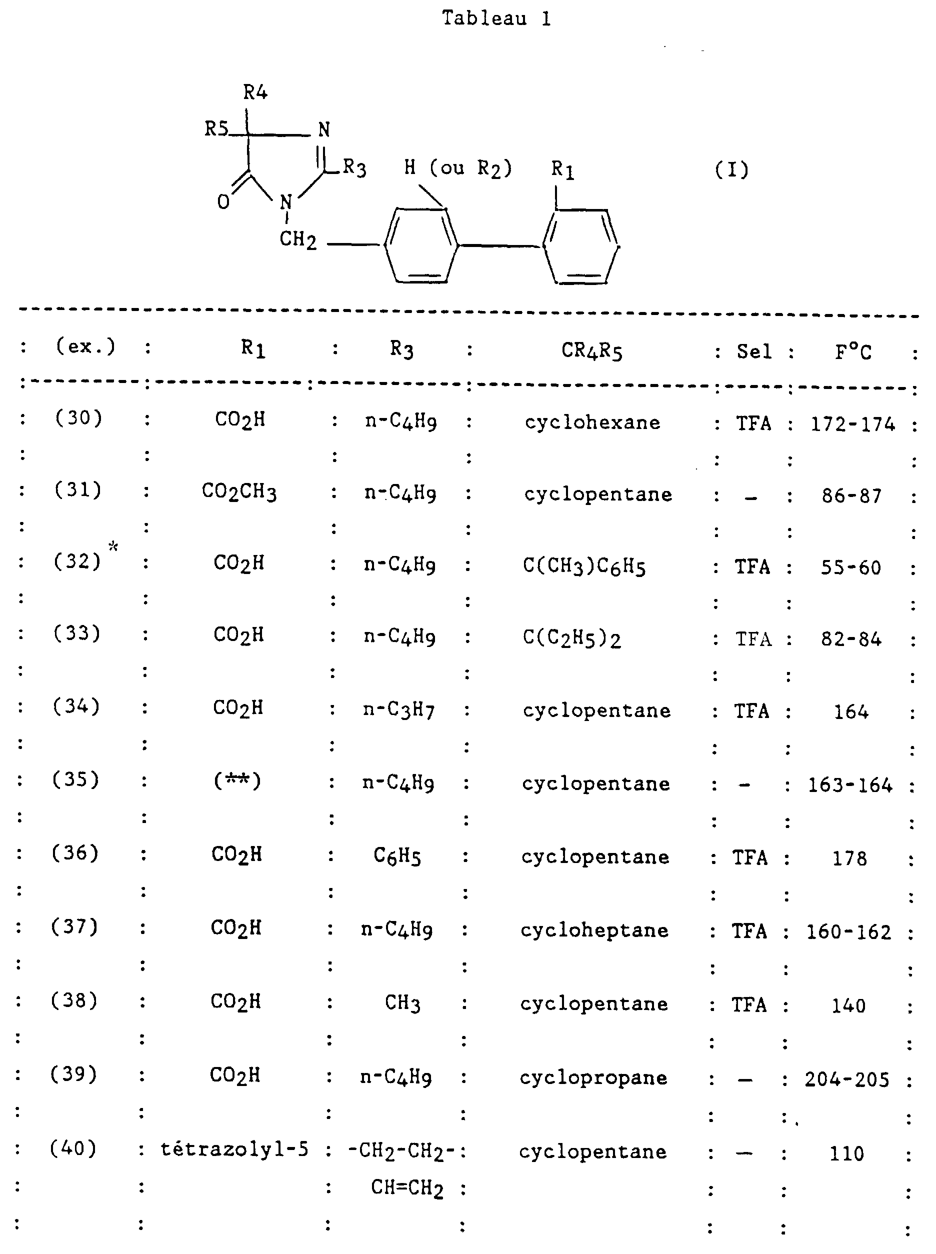

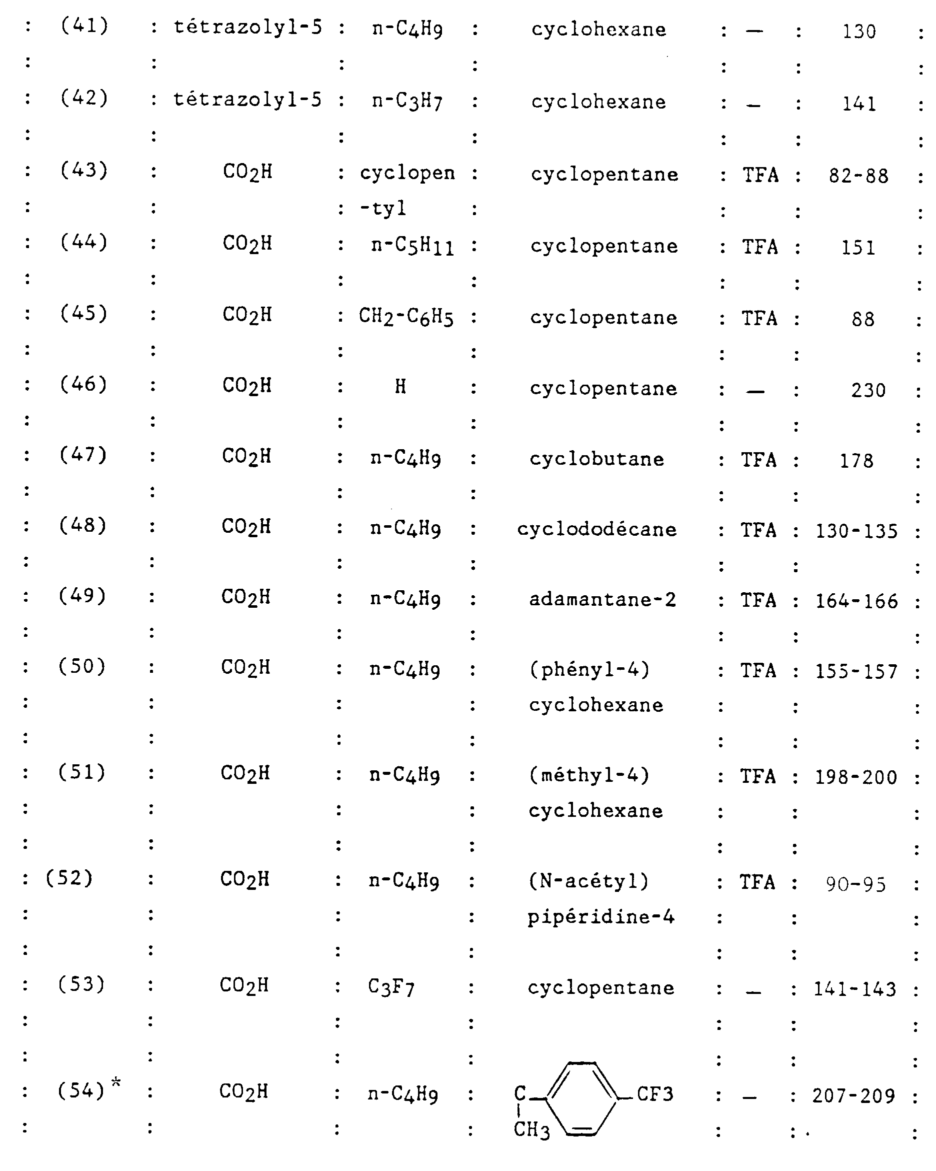

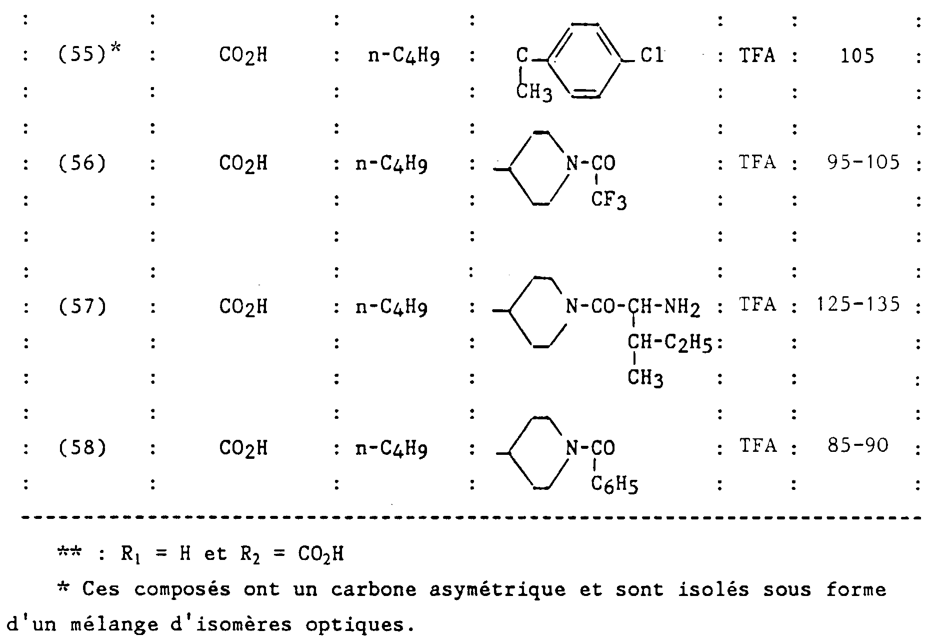

## Revendications (Revendications pour l'(les) Etat(s) contractant(s) suivant(s): AT, BE, CH, DE, DK, FR, GB, IT, LI, LU, NL, SE)

1. Un composé de formule : 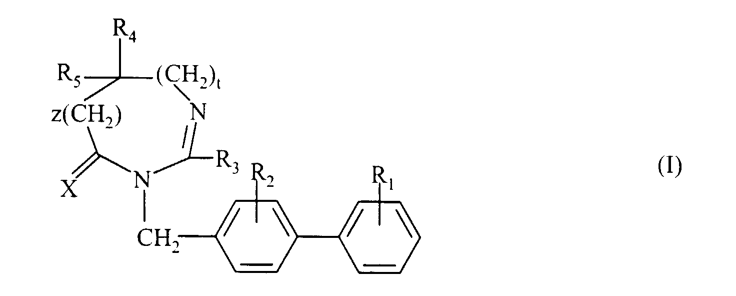 dans laquelle:
- R₁ et R₂ sont semblables ou différents et représentent chacun indépendamment l'hydrogène ou un groupe choisi parmi un alkyle en C₁-C₆, un alcoxy en C₁-C₄, un amino, un aminométhyle, un carboxy, un alcoxycarbonyle dans lequel l'alcoxy est en C₁-C₄, un cyano, un tétrazolyle, un méthyltétrazolyle, un méthylsulfonylamino, un trifluorométhylsulfonylamino, un trifluorométhylsulfonylaminométhyle, un N-cyanocarbamoyle, un N-hydroxycarbamoyle, un N-((carboxy-4) thiazol-1,3-yl-2)carbamoyle, un uréido, un cyano-2 guanidinocarbonyle, un cyano-2 guanidinométhyle, un imizadol-1-yl-carbonyle, un cyano-3 méthyl-2 isothiouréidométhyle, à la condition qu'au moins l'un des substituants R₁ ou R₂ soit différent de l'hydrogène ;
- R₃ représente un hydrogène, un alkyle en C₁-C₆, non substitué ou substitué par un ou plusieurs atomes d'halogène, un alcényle en C₂-C₆, un cycloalkyle en C₃-C₇, un phényle, un phénylalkyle dans lequel l'alkyle est en C₁-C₃, un phénylalcényle dans lequel l'alcényle est en C₂-C₃, lesdits groupes phényles étant non substitués ou substitués une ou plusieurs fois par un atome d'halogène, un alkyle en C₁-C₄, un halogénoalkyle en C₁-C₄, un polyhalogénoalkyle en C₁-C₄, un hydroxyle ou un alcoxy en C₁-C₄;
- R₄ et R₅ représentent chacun indépendamment un alkyle en C₁-C₆, un phényle, un phénylalkyle dans lequel l'alkyle est en C₁-C₃, lesdits groupes alkyle, phényle et phénylalkyle étant non substitués ou substitués par un ou plusieurs atomes d'halogène ou par un groupe choisi parmi un perfluoroalkyle en C₁-C₄, un hydroxyle, un alcoxy en C₁-C₄ ;
- ou R₄ et R₅ ensemble forment un groupe de formule =CR₇R₈, dans laquelle R₇ représente l'hydrogène, un alkyle en C₁-C₄ ou un phényle, et R₈ représente un alkyle en C₁-C₄ ou un phényle ;
- ou encore R₄ et R₅ liés ensemble représentent, soit un groupe de formule (CH₂)ₙ, soit un groupe de formule (CH₂)ₚY(CH₂)_{q}, dans lequel Y est, soit un atome d'oxygène, soit un atome de soufre, soit un atome de carbone substitué par un groupe alkyle en C₁-C₄, un phényle ou un phénylalkyle dans lequel l'alkyle est en C₁-C₃, soit un groupe N-R₆ dans lequel R₆ représente un hydrogène, un alkyle en C₁-C₄, un phénylalkyle dans lequel l'alkyle est en C₁-C₃, un alkylcarbonyle en C₁-C₄, un halogénoalkylcarbonyle en C₁-C₄, un polyhalogénoalkylcarbonyle en C₁-C₄, un benzoyle, un alpha aminoacyle ou un groupe N-protecteur, ou R₄ et R₅ liés ensemble avec l'atome de carbone auquel ils sont liés constituent un indane ou un adamantane ;
- p + q = m ;
- n est un nombre entier compris entre 2 et 11 ;
- m est un nombre entier compris entre 2 et 5 ;
- X représente un atome d'oxygène ou un atome de soufre ;
- z et t son nuls ou l'un est nul et l'autre représente un ;
et ses sels.

2. Composé selon la revendication 1, caractérisé en ce que R₁ est en position ortho et représente un groupe carboxy ou tétrazolyle et R₂ est l'hydrogène.

3. Composé selon l'une quelconque des revendications 1 et 2, caractérisé en ce que R₄ et R₅ constituent avec le carbone auquel ils sont liés un cyclopentane ou un cyclohexane.

4. Composé selon l'une quelconque des revendications 1 à 3, caractérisé en ce que R₃ représente un groupe alkyle droit en C₁-C₆.

5. Composé selon l'une quelconque des revendications 1 à 4, caractérisé en ce que X est l'oxygène.

6. Composé selon l'une quelconque des revendications 1 à 5, caractérisé en ce que z = t = 0.

7. Composé selon la revendication 1, caractérisé en ce qu'il est le n-butyl-2 spirocyclopentane-4 [((tétrazolyl-5)-2' biphényl-4-yl) méthyl]-1 imidazoline-2 one-5 ou l'un de ses sels avec des acides ou des bases.

8. Procédé pour la préparation d'un composé (I) selon l'une quelconque des revendications 1 à 7, caractérisé en ce que :
a1) on fait réagir un dérivé hétérocyclique de formule : 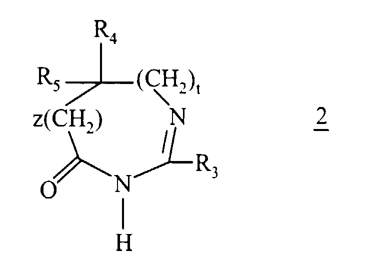 dans laquelle z, t, R₃, R₄ et R₅ ont les significations indiquées pour (I) dans la revendication 1, sur un dérivé du (biphényl-4-yl) méthyl de formule : 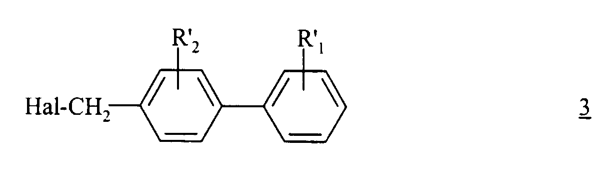 dans laquelle Hal représente un atome d'halogène et R'₁ et R'₂ représentent respectivement soit R₁ et R₂, soit un groupement précurseur de R₁ et R₂ ;
b1) éventuellement, le composé ainsi obtenu de formule : 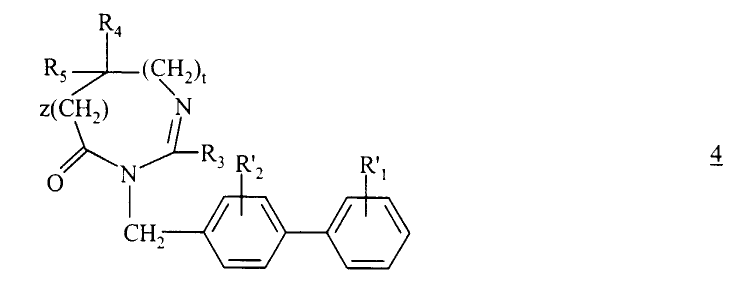 est traité par le réactif de Lawesson [bis(méthoxy-4 phényl)-2,4 dithia-1,3 diphosphétane-2,4 disulfure 2,4];
c1) le composé obtenu en a1) ou en b1), de formule : 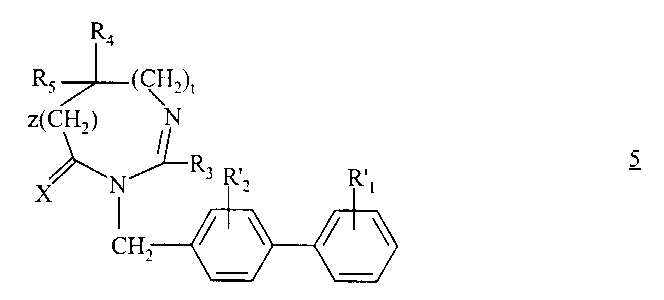 dans laquelle X représente un atome d'oxygène ou un atome de soufre, est traité pour préparer le composé (I) par transformation des groupes R'₁ et/ou R'₂, en respectivement, les groupes R₁ et/ou R₂, qui est ensuite éventuellement transformé en l'un de ses sels.

9. Procédé pour la préparation d'un composé (I), selon l'une quelconque des revendications 1 à 7, caractérisé en ce que :
a2) on fait réagir un aminoacide de formule : 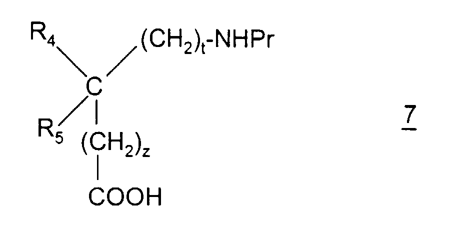 dans laquelle z, t, R₄ et R₅ ont les significations indiquées pour (I) dans la revendication 1 et dont la fonction amine est protégée par le groupe Pr, sur un dérivé (biphényl-4-yl) méthylamine de formule : 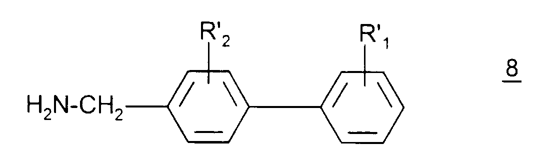 dans laquelle R'₁ et R'₂ représentent respectivement soit R₁ et R₂, soit un groupement précurseur de R₁ et R₂ ;
b2) après déprotection de l'amine, le composé ainsi obtenu de formule: 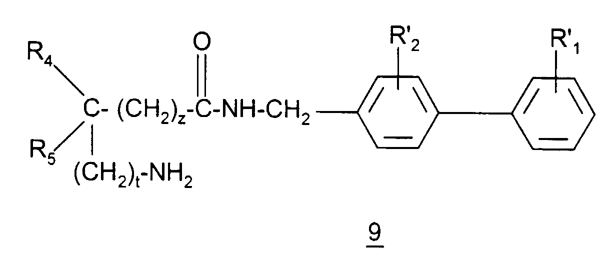 est ensuite traité par un ortho-ester d'alkyle de formule R₃C(OR)₃ (10) dans laquelle R₃ a la signification indiquée pour (I) dans la revendication 1 et R est un alkyle en C₁-C₄ ;
c2) éventuellement, le composé ainsi obtenu de formule : 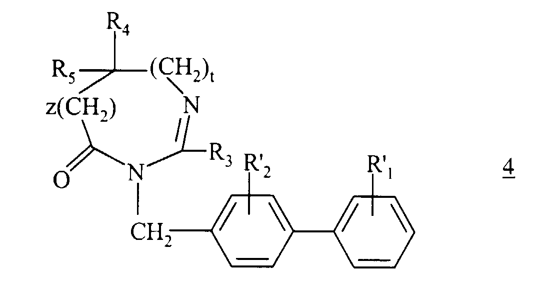 est traité par le réactif de Lawesson [bis(méthoxy-4 phényl)-2,4 dithia-1,3 diphosphétane-2,4 disulfure-2,4] ;
d2) le composé ainsi obtenu en b2 ou en c2 de formule 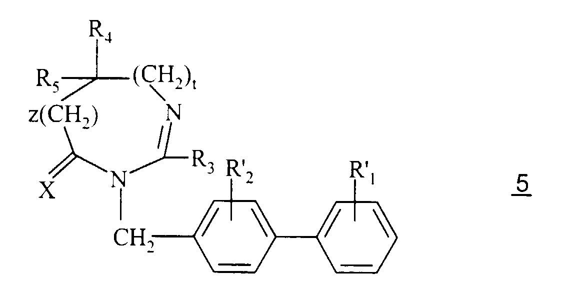 est ensuite traité dans des conditions convenables pour préparer le composé (I) par transformation des groupes R'₂ et/ou R'₁ en, respectivement, les groupes R₂ et/ou R₁, qui est ensuite éventuellement transformé en l'un de ses sels.

10. Procédé pour la préparation d'un composé (I) selon la revendication 6 caractérisé en ce que :
a3) on fait agir sur un dérivé de l'imidazole de formule : 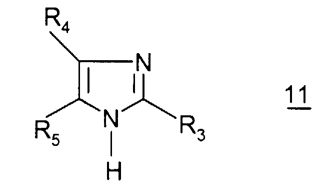 dans laquelle R₃, R₄, R₅ ont les significations indiquées pour (I) dans la revendication 1,
un dérivé du (biphényl-4-yl) méthyl de formule : 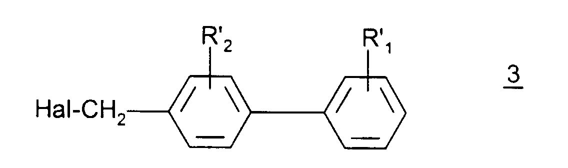 dans laquelle Hal représente un atome d'halogène et R'₁ et R'₂ représentent respectivement soit R₁ et R₂, soit un groupement précurseur de R₁ et R₂, en présence d'oxygène et d'une irradiation UV, en milieu basique ;
b3) éventuellement, le composé ainsi obtenu de formule : 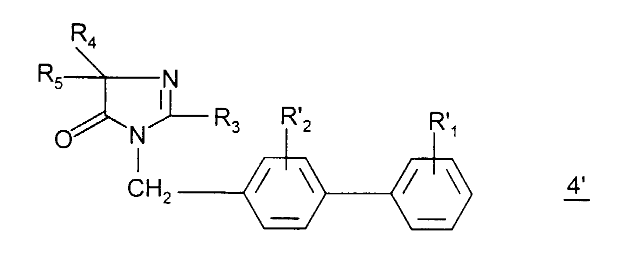 est traité par le réactif de Lawesson [bis(méthoxy-4 phényl)-2,4 dithia-1,3 diphosphétane-2,4 disulfure-2,4];
c3) le composé ainsi obtenu en b3 ou en c3 de formule : 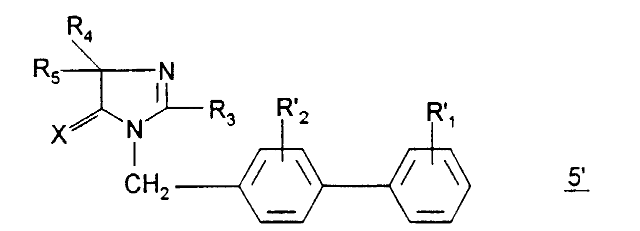 est ensuite traité dans des conditions convenables pour préparer le composé (I) par transformation des groupes R'₁ et/ou R'₂ en respectivement les groupes R₁ et/ou R₂, qui est ensuite éventuellement transformé en l'un de ses sels.

11. Un composé de formule : 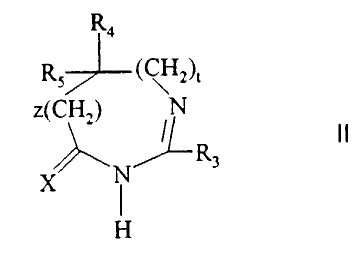 dans laquelle :
- R₃ représente un hydrogène, un alkyle en C₁-C₆, non substitué ou substitué par un ou plusieurs atomes d'halogène, un alcényle en C₂-C₆, un cycloalkyle en C₃-C₇, un phényle, un phénylalkyle dans lequel l'alkyle est en C₁-C₃, un phénylalcényle dans lequel l'alcényle est en C₂-C₃, lesdits groupes phényles étant non substitués ou substitués une ou plusieurs fois par un atome d'halogène, un alkyle en C₁-C₄, un halogénoalkyle en C₁-C₄, un polyhalogénoalkyle en C₁-C₄, un hydroxyle ou un alcoxy en C₁-C₄ ; R₄ et R₅ représentent chacun indépendamment un alkyle en C₁-C₆, un phényle, un phénylalkyle dans lequel l'alkyle est en C₁-C₃, lesdits groupes alkyle, phényle et phénylalkyle étant non substitués ou substitués par un ou plusieurs atomes d'halogène ou par un groupe choisi parmi un perfluoroalkyle en C₁-C₄, un hydroxyle, un alcoxy en C₁-C₄ ;
- ou R₄ et R₅ ensemble forment un groupe de formule =CR₇R₈, dans laquelle R₇ représente l'hydrogène, un alkyle en C₁-C₄ ou un phényle, et R₈ représente un alkyle en C₁-C₄ ou un phényle ;
- ou encore R₄ et R₅ liés ensemble représentent, soit un groupe de formule (CH₂)ₙ, soit un groupe de formule (CH₂)ₚY(CH₂)_{q}, dans lequel Y est soit un atome d'oxygène, soit un atome de soufre, soit un atome de carbone substitué par un groupe alkyle en C₁-C₄, un phényle ou un phénylalkyle dans lequel l'alkyle est en C₁-C₃, soit un groupe N-R₆ dans lequel R₆ représente un hydrogène, un alkyle en C₁-C₄, un phénylalkyle dans lequel l'alkyle est en C₁-C₃, un alkylcarbonyle en C₁-C₄, un halogénoalkylcarbonyle en C₁-C₄, un polyhalogénoalkylcarbonyle en C₁-C₄, un benzoyle, un alphaaminoacyle ou un groupe N-protecteur, ou R₄ et R₅ liés ensemble avec l'atome de carbone auquel ils sont liés constituent un indane ou un adamantane ;
- p + q = m ;
- n est un nombre entier compris entre 2 et 11 ;
- m est un nombre entier compris entre 2 et 5 ;
- X représente un atome d'oxygène ou un atome de soufre ;
- z et t sont nuls ou l'un est nul et l'autre représente un ;
avec la limitation que
A/ lorsque z et t sont nuls et X représente l'oxygène :
1) R₄ et R₅ sont autres qu'un alkyle en C₁-C₆ ; un phényle, un phénylalkyle dans lequel l'alkyle est en C₁-C₃, lesdits groupes alkyle, phényle et phénylalkyle étant non substitués ou substitués par un ou plusieurs atomes d'halogène ou par un groupe choisi parmi un perfluoroalkyle en C₁-C₄, un hydroxyle, un alcoxy en C₁-C₄ ; ou
2) R₄ et R₅ liés ensemble sont autres qu'un groupe de formule (CH₂)ₚY(CH₂)_{q} dans lequel Y est un groupe N-R₆ dans lequel R₆ est un hydrogène, un alkyle en C₁-C₄, un phénylalkyle dans lequel l'alkyle est en C₁-C₃ ; ou
3) R₄ et R₅ ensemble sont différents du groupe = CR₇R₈ lorsque R₃ est un hydrogène, un phényle, un méthylphényle ou un benzyle ;
4) n est différent de 6 ; ou bien
5) R₄ et R₅ liés ensemble sont autres qu'un groupe (CH₂)ₙ dans lequel n est compris entre 3 et 5, lorsque R₃ représente un groupe phényle substitué ;
B/ lorsque z = 1 et R₃ est un phényle, R₄ et R₅ sont chacun différent d'un méthyle ;
C/ lorsque z et t sont nuls et X est le soufre :
1) R₄ et R₅ sont autres qu'un phényle lorsque R₃ est un méthyle, un phényle, un chlorophényle, un méthoxyphényle ou un méthylphényle ; ou
2) R₄ et R₅ sont autres qu'un alkyle en C₁-C₃ lorsque R₃ est un méthyle ou un phényle; ou
3) R₄ et R₅ sont autres qu'un méthoxyphényle ou chlorophényle lorsque R₃ est phényle ; ou
4) R₄ et R₅ sont autres qu'un chlorophényle lorsque R₃ est un méthyle.

12. Un composé selon la revendication 11 de formule : 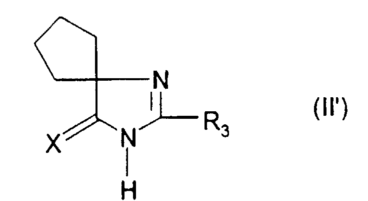 dans laquelle X représente un atome d'oxygène ou un atome de soufre et R₃ représente un hydrogène, un alkyle en C₁-C₆, non substitué ou substitué par un ou plusieurs atomes d'halogène ; un alcényle en C₂-C₆, un cycloalkyle en C₃-C₇, un phényle, un phénylalkyle dans lequel l'alkyle est en C₁-C₃, un phénylalcényle dans lequel l'alcényle est en C₂-C₃, lesdits groupes phényles étant non substitués ou substitués une ou plusieurs fois par un atome d'halogène, un alkyle en C₁-C₄, un halogénoalkyle en C₁-C₄, un polyhalogénoalkyle en C₁-C₄, un hydroxyle ou un alcoxy en C₁-C₄ ; à la condition que R₃ soit autre qu'un groupe phényle substitué lorsque X est l'oxygène.

13. Composé selon la revendication 12, caractérisé en ce que, dans la formule II', X est l'oxygène et R₃ est le n-butyle et en ce qu'il est le n-butyl-2 spirocyclopentane-4 imidazoline-2 one-5.

14. Chlorhydrate de n-butyl-2 spirocyclopentane-4 imidazoline-2 one-5.

15. Un composé selon la revendication 11, de formule : 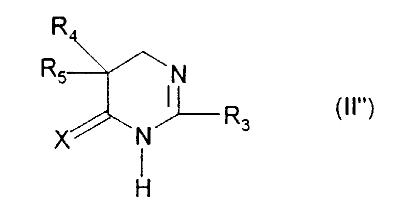 dans laquelle R₃, R_{4,} R₅ et X ont les définitions données ci-dessus pour (II) dans la revendication 11.

16. Un composé selon la revendication 11 de formule : 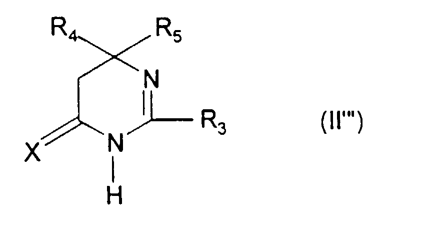 dans laquelle :
- R₃ représente un hydrogène, un alkyle en C₁-C₆, non substitué ou substitué par un ou plusieurs atomes d'halogène, un alcényle en C₂-C₆, un cycloalkyle en C₃-C₇, un phényle, un phénylalkyle dans lequel l'alkyle est en C₁-C₃, un phénylalcényle dans lequel l'alcényle est en C₂-C₃, lesdits groupes phényles étant non substitués ou substitués une ou plusieurs fois par un atome d'halogène, un alkyle en C₁-C₄, un halogénoalkyle en C₁-C₄, un polyhalogénoalkyle en C₁-C₄, un hydroxyle ou un alcoxy en C₁-C₄ ;
- R₄ et R₅ représentent chacun indépendamment un alkyle en C₁-C₆, un phényle, un phénylalkyle dans lequel l'alkyle est C₁-C₃, lesdits groupes alkyle, phényle et phénylalkyle étant non substitués ou substitués par un ou plusieurs atomes d'halogène ou par un groupe choisi parmi un perfluoroalkyle en C₁-C₄, un hydroxyle, un alcoxy en C₁-C₄ ;
- ou R₄ et R₅ ensemble forment un groupe de formule =CR₇R₈, dans laquelle R₇ représente l'hydrogène, un alkyle en C₁-C₄ ou un phényle, et R₈ représente un alkyle en C₁-C₄ ou un phényle ;
- ou R₄ et R₅ liés ensemble représentent, soit un groupe de formule (CH₂)ₙ, soit un groupe de formule (CH₂)ₚY(CH₂)_{q}, dans lequel Y est, soit un atome d'oxygène, soit un atome de soufre, soit un atome de carbone substitué par un groupe alkyle en C₁-C₄, un phényle ou phénylalkyle dans lequel l'alkyle est en C₁-C₃, soit un groupe N-R₆ dans lequel R₆ représente un hydrogène, un alkyle en C₁-C₄, un phénylalkyle dans lequel l'alkyle est en C₁-C₄, un alkylcarbonyle en C₁-C₄, un halogénoalkylcarbonyle en C₁-C₄, un polyhalogénoalkylcarbonyle en C₁-C₄, un benzoyle, un alpha aminoacyle ou un groupe N-protecteur, ou R₄ et R₅ liés ensemble avec l'atome de carbone auquel ils sont liés constituent un indane ou un adamantane ;
- p + q = m ;
- n est un nombre entier compris entre 2 et 11 ;
- m est un nombre entier compris entre 2 et 5 ;
- X représente un atome d'oxygène ou un atome de soufre;
avec la limitation que R₃ est autre qu'un phényle lorsque R₄ et R₅ représentent chacun un méthyle.

17. Procédé pour la préparation d'un composé selon l'une quelconque des revendications 11 à 16, caractérisé en ce que : on fait agir sur un composé de formule : 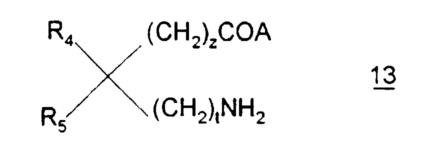 dans laquelle R₄, R₅ ont les définitions indiquées ci-dessus pour (II) dans la revendication 11, A représente un groupe OH, un groupe NH₂ ou un groupe OR', R' étant l'hydrogène ou un alkyle en C₁-C₄, un composé de formule :
R₃ - B 14
dans laquelle R₃ a la définition indiquée dans les revendications 11 à 16 et B représente :
- un groupement C (OR)₃
- un groupement 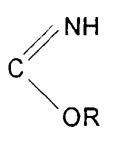 ou
- un groupement COHal,
R étant un alkyle en C₁-C₄ et Hal désignant un atome d'halogène, de préférence le chlore ;
puis éventuellement, le composé ainsi obtenu est traité par le réactif de Lawesson (bis/méthoxy-4 phényl)-2,4 dithia-1,3 diphosphétane-2,4 disulfure.

18. Composition pharmaceutique contenant à titre de principe actif un composé selon l'une quelconque des revendications 1 à 7.

19. Composition pharmaceutique contenant un composé selon l'une quelconque des revendications 1 à 7 en association avec un composé bêtabloquant.

20. Composition pharmaceutique contenant un composé selon l'une quelconque des revendications 1 à 7 en association avec un diurétique.

21. Composition pharmaceutique contenant un composé selon l'une quelconque des revendications 1 à 7 en association avec un anti-inflammatoire non stéroïdien.

22. Composition pharmaceutique contenant un composé selon l'une quelconque des revendications 1 à 7 en association avec un antagoniste calcique.

23. Composition pharmaceutique contenant un composé selon l'une quelconque des revendications 1 à 7 en association avec un tranquillisant.

## Revendications (Revendications pour l'(les) Etat(s) contractant(s) suivant(s): ES)

1. Procédé pour l'obtention d'un composé de formule : 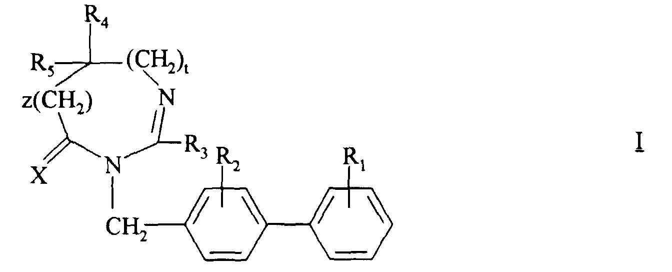 dans laquelle:
- R₁ et R₂ sont semblables ou différents et représentent chacun indépendamment l'hydrogène ou un groupe choisi parmi un alkyle en C₁-C₆, un alcoxy en C₁-C₄, un amino, un aminométhyle, un carboxy, un alcoxycarbonyle dans lequel l'alcoxy est en C₁-C₄, un cyano, un tétrazolyle, un méthyltétrazolyle, un méthylsulfonylamino, un trifluorométhylsulfonylamino, un trifluorométhylsulfonylaminométhyle, un N-cyanocarbamoyle, un N-hydroxycarbamoyle, un N-((carboxy-4) thiazol-1,3-yl-2)carbamoyle, un uréido, un cyano-2 guanidinocarbonyle, un cyano-2 guanidinométhyle, un imizadol-1-yl-carbonyle, un cyano-3 méthyl-2 isothiouréidométhyle, à la condition qu'au moins l'un des substituants R₁ ou R₂ soit différent de l'hydrogène ;
- R₃ représente un hydrogène, un alkyle en C₁-C₆, non substitué ou substitué par un ou plusieurs atomes d'halogène, un alcényle en C₂-C₆, un cycloalkyle en C₃-C₇, un phényle, un phénylalkyle dans lequel l'alkyle est en C₁-C₃, un phénylalcényle dans lequel l'alcényle est en C₂-C₃, lesdits groupes phényles étant non substitués ou substitués une ou plusieurs fois par un atome d'halogène, un alkyle en C₁-C₄, un halogénoalkyle en C₁-C₄, un polyhalogénoalkyle en C₁-C₄, un hydroxyle ou un alcoxy en C₁-C₄;
- R₄ et R₅ représentent chacun indépendamment un alkyle en C₁-C₆, un phényle, un phénylalkyle dans lequel l'alkyle est en C₁-C₃, lesdits groupes alkyle, phényle et phénylalkyle étant non substitués ou substitués par un ou plusieurs atomes d'halogène ou par un groupe choisi parmi un perfluoroalkyle en C₁-C₄, un hydroxyle, un alcoxy en C₁-C₄ ;
- ou R₄ et R₅ forment ensemble un groupe de formule =CR₇R₈, dans laquelle R₇ représente l'hydrogène, un alkyle en C₁-C₄ ou un phényle, et R₈ représente un alkyle en C₁-C₄ ou un phényle ;
- ou encore R₄ et R₅ liés ensemble représentent, soit un groupe de formule (CH₂)ₙ, soit un groupe de formule (CH₂)ₚY(CH₂)_{q}, dans lequel Y est, soit un atome d'oxygène, soit un atome de soufre, soit un atome de carbone substitué par un groupe alkyle en C₁-C₄, un phényle ou un phénylalkyle dans lequel l'alkyle est en C₁-C₃, soit un groupe N-R₆ dans lequel R₆ représente un hydrogène, un alkyle en C₁-C₄, un phénylalkyle dans lequel l'alkyle est en C₁-C₃, un alkylcarbonyle en C₁-C₄, un halogénoalkylcarbonyle en C₁-C₄, un polyhalogénoalkylcarbonyle en C₁-C₄, un benzoyle, un alpha aminoacyle ou un groupe N-protecteur, ou R₄ et R₅ liés ensemble avec l'atome de carbone auquel ils sont liés constituent un indane ou un adamantane ;
- p + q = m ;
- n est un nombre entier compris entre 2 et 11 ;
- m est un nombre entier compris entre 2 et 5 ;
- X représente un atome d'oxygène ou un atome de soufre ;
- z et t son nuls ou l'un est nul et l'autre représente un ;
et ses sels, caractérisé en ce que :
a1) on fait réagir un dérivé hétérocyclique de formule : 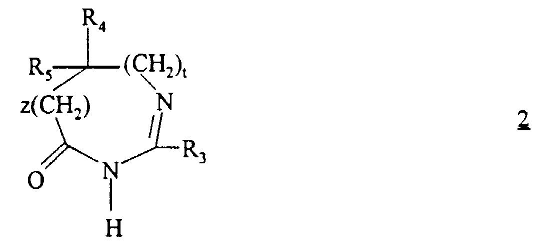 dans laquelle z, t, R₃, R₄ et R₅ ont les significations indiquées pour (I) ci-dessus, sur un dérivé du (biphényl-4-yl)méthyl de formule : 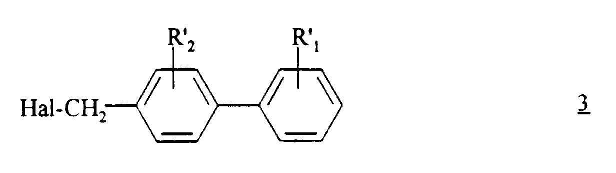 dans laquelle Hal représente un atome d'halogène et R'₁ et R'₂ représentent respectivement soit R₁ et R₂, soit un groupement précurseur de R₁ et R₂ ;
b1)éventuellement, le composé ainsi obtenu de formule : 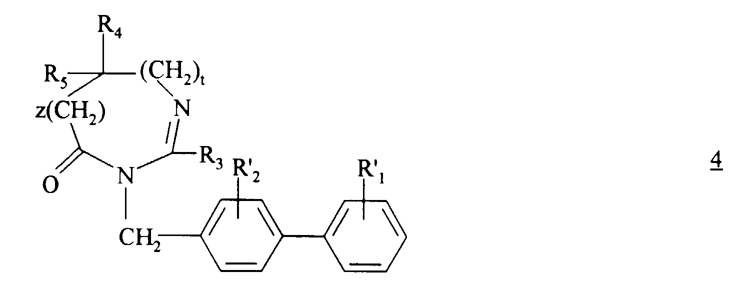 est traité par le réactif de Lawesson [bis(méthoxy-4 phényl)-2,4 dithia-1,3 diphosphétane-2,4 disulfure-2,4] ;
c1) le composé obtenu en a1) ou en b1), de formule : 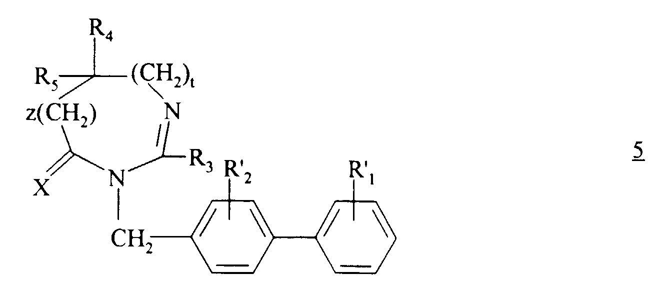 dans laquelle X représente un atome d'oxygène ou un atome de soufre, est traité pour préparer le composé (I) par transformation des groupes R'₁ et/ou R'₂, en respectivement, les groupes R₁ et/ou R₂, qui est ensuite éventuellement transformé en l'un de ses sels.

2. Procédé pour l'obtention de composés de formule (I) : 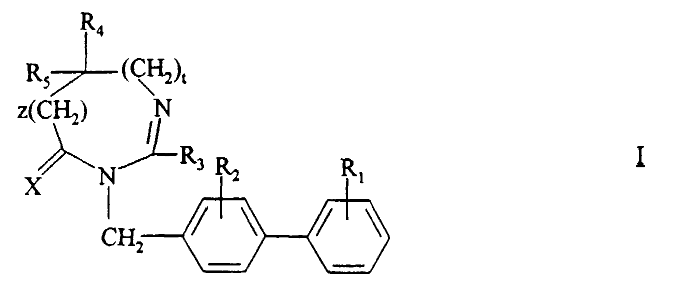 dans laquelle R₁, R₂, R₃, R₄, R₅, X, z et t sont tels que définis dans la revendication 1, caractérisé en ce que :
a2) on fait réagir un aminoacide de formule: 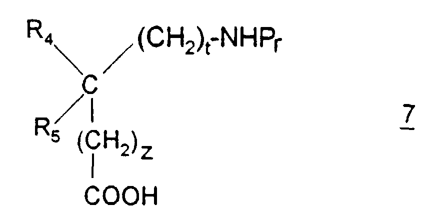 dans laquelle z, t, R₄ et R₅ ont les significations indiquées pour (I) dans la revendication 1 et dont la fonction amine est protégée par le groupe Pr, sur un dérivé (biphényl-4-yl) méthylamine de formule : 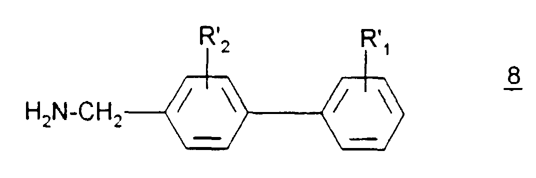 dans laquelle R'₁ et R'₂ représentent respectivement soit R₁ et R₂, soit un groupement précurseur de R₁ et R₂ ;
b2) après déprotection de l'amine, le composé ainsi obtenu de formule : 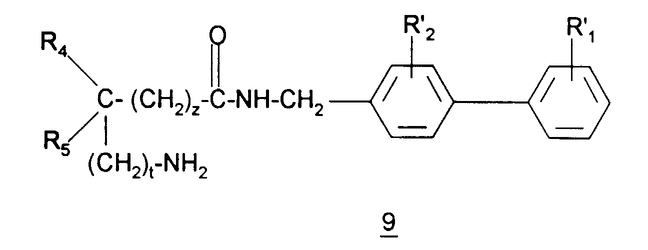 est ensuite traité par un ortho-ester d'alkyle de formule R₃C(OR)₃ (10) dans laquelle R₃ a la signification indiquée pour (I) dans la revendication 1 et R est un alkyle en C₁-C₄ ;
c2) éventuellement, le composé ainsi obtenu de formule : 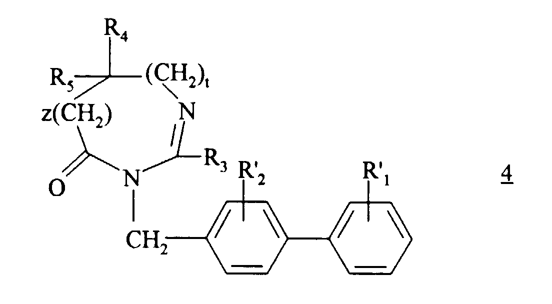 est traité par le réactif de Lawesson [bis(méthoxy-4 phényl)-2,4 dithia-1,3 diphosphétane-2,4 disulfure-2,4] ;
d2) le composé ainsi obtenu en b2 ou en c2 de formule : 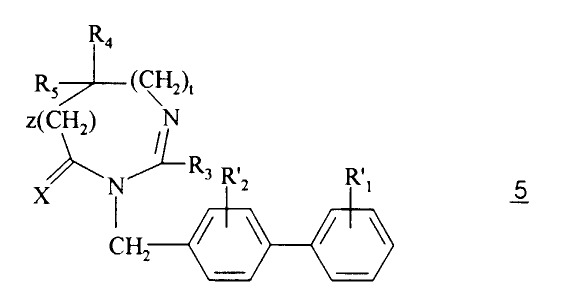 est ensuite traité dans des conditions convenables pour préparer le composé (I) par transformation des groupes R'₂ et/ou R'₁ en respectivement, les groupes R₂ et/ou R₁, qui est ensuite éventuellement transformé en l'un de ses sels.

3. Procédé pour l'obtention de composés de formule (I) : 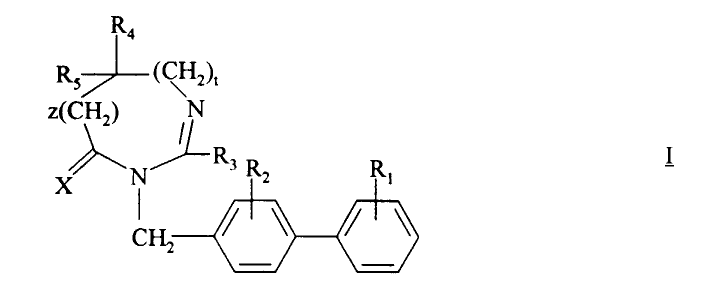 dans laquelle R₁, R₂, R₃, R₄, R₅, X sont tels que définis dans la revendication 1, z et t sont égaux à zéro, caractérisé en ce que :
a3) on fait agir sur un dérivé de l'imidazole de formule : 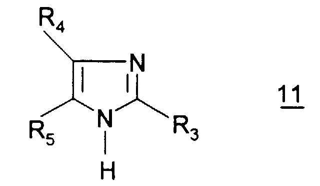 dans laquelle R₃, R₄, R₅ ont les significations indiquées pour (I) dans la revendication 1, un dérivé du (biphényl-4-yl) méthyl de formule : 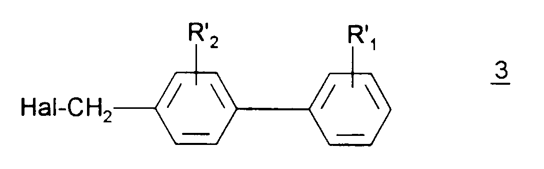 dans laquelle Hal représente un atome d'halogène et R'₁ et R'₂ représentent respectivement soit R₁ et R₂, soit un groupement précurseur de R₁ et R₂, en présence d'oxygène et d'une irradiation UV, en milieu basique ;
b3) éventuellement, le composé ainsi obtenu de formule : 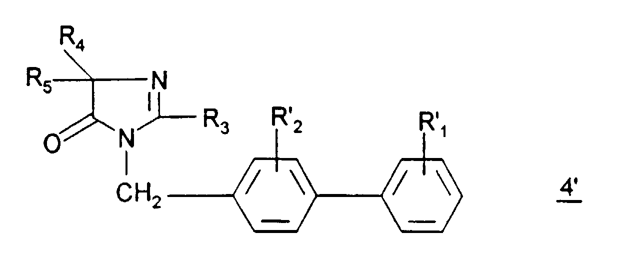 est traité par le réactif de Lawesson [bis(méthoxy-4 phényl)-2,4 dithia-1,3 diphosphétane-2,4 disulfure-2,4] ;
c3) le composé ainsi obtenu en b3 ou en c3 de formule : 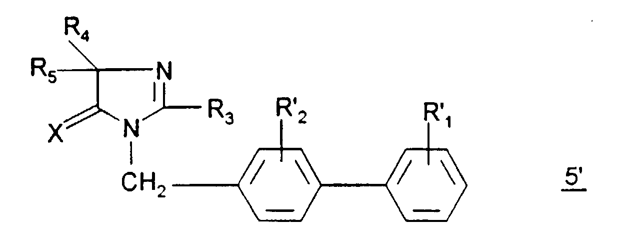 est ensuite traité dans des conditions convenables pour préparer le composé (I) par transformation des groupes R'₁ et/ou R'₂ en respectivement les groupes R₁ et/ou R₂, qui est éventuellement transformé en l'un de ses sels.

4. Procédé selon l'une quelconque des revendications 1 à 3, caractérisé en ce que dans le composé de formule 3 ou 8, R'₂ est l'hydrogène et R'₁ est un groupe carboxy estérifié qui est transformé dans les étapes c1), d2) ou c3) en un groupe carboxy.

5. Procédé selon l'une quelconque des revendications 1 à 3, caractérisé en ce que dans le composé de formule 3 ou 8, R'₂ est l'hydrogène et R'₁ est soit un groupe tétrazolyle protégé, soit un groupe cyano, qui sont transformés dans les étapes c1), d2) ou c3) en un groupe tétrazolyle.

6. Procédé selon l'une quelconque des revendications 1 à 5, caractérisé en ce que dans les composés de formules 2, 7 ou 11, R₄ et R₅ constituent avec l'atome de carbone auquel ils sont liés un cyclopentane ou un cyclohexane.

7. Procédé selon l'une quelconque des revendications 1 à 6, caractérisé en ce que dans les composés de formules 2, 10 et 11, R₃ représente un groupe alkyle droit en C₁-C₆.

8. Procédé selon l'une quelconque des revendications 1 à 7, caractérisé en ce qu'il comporte les étapes a1) et c1) ; a2), b2) et d2) ou a3), b3) respectivement.

9. Procédé selon l'une des revendications 1 ou 2, caractérisé en ce que dans les composés de formules 2 et 7, z et t sont nuls.

10. Procédé selon la revendication 1, caractérisé en ce que l'on utilise :
1) un composé de formule 2 dans laquelle R₃ est le groupe n-butyle ; R₄ et R₅ forment ensemble avec l'atome de carbone auxquels ils sont liés le groupe spirocyclopentane et z et t sont nuls ; 2) un composé de formule 3 dans laquelle R'₂ est l'hydrogène et R'₁ est un groupe tétrazolyle protégé pour obtenir le n-butyl-2 spirocyclopentane-4 [((tétrazolyl-5)-2' biphényl-4-yl) méthyl]-1 imidazoline-2 one-5 ou l'un de ses sels avec des acides ou des bases.

11. Procédé pour l'obtention d'un composé de formule : 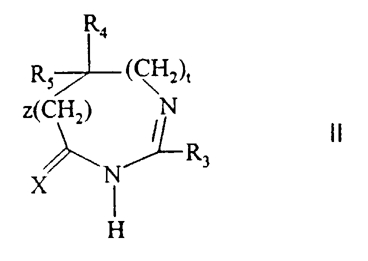 dans laquelle :
- R₃ représente un hydrogène, un alkyle en C₁-C₆, non substitué ou substitué par un ou plusieurs atomes d'halogène, un alcényle en C₂-C₆, un cycloalkyle en C₃-C₇, un phényle, un phénylalkyle dans lequel l'alkyle est en C₁-C3, un phénylalcényle dans lequel l'alcényle est en C₂-C₃, lesdits groupes phényles étant non substitués ou susbstitués une ou plusieurs fois par un atome d'halogène, un alkyle en C₁-C₄, un halogénoalkyle en C₁-C₄, un polyhalogénoalkyle en C₁-C₄, un hydroxyle ou un alcoxy en C₁-C₄ ;
- R₄ et R₅ représentent chacun indépendamment un alkyle en C₁-C₆, un phényle, un phénylalkyle dans lequel l'alkyle est en C₁-C₃, lesdits groupes alkyle, phényle et phénylalkyle étant non substitués ou substitués par un ou plusieurs atomes d'halogène ou par un groupe choisi parmi un perfluoroalkyle en C₁-C₄, un hydroxyle, un alcoxy en C₁-C₄ ;
- ou R₄ et R₅ ensemble forment un groupe de formule =CR₇R₈, dans laquelle R₇ représente l'hydrogène, un alkyle en C₁-C₄ ou un phényle, et R₈ représente un alkyle en C₁-C₄ ou un phényle ;
- ou encore R₄ et R₅ liés ensemble représentent, soit un groupe de formule (CH₂)ₙ, soit un groupe de formule (CH₂)ₚY(CH₂)_{q}, dans lequel Y est soit un atome d'oxygène, soit un atome de soufre, soit un atome de carbone substitué par un groupe alkyle en C₁-C₄, un phényle ou un phénylalkyle dans lequel l'alkyle est en C₁-C₃, soit un groupe N-R₆ dans lequel R₆ représente un hydrogène, un alkyle en C₁-C₄, un phénylalkyle dans lequel l'alkyle est en C₁-C₃, un alkylcarbonyle en C₁-C₄, un halogénoalkylcarbonyle en C₁-C₄, un polyhalogénoalkylcarbonyle en C₁-C₄, un benzoyle, un alphaaminoacyle ou un groupe N-protecteur, ou R₄ et R₅ liés ensemble avec l'atome de carbone auquel ils sont liés constituent un indane ou un adamantane ;
- p + q = m ;
- n est un nombre entier compris entre 2 et 11 ;
- m est un nombre entier compris entre 2 et 5 ;
- X représente un atome d'oxygène ou un atome de soufre ;
- z et t sont nuls ou l'un est nul et l'autre représente un ;
avec la limitation que
A/ lorsque z et t sont nuls et X représente l'oxygène :
1) R₄ et R₅ sont autres qu'un alkyle en C₁-C₆ ; un phényle, un phénylalkyle dans lequel l'alkyle est en C₁-C₃, lesdits groupes alkyle, phényle et phénylalkyle étant non substitués ou substitués par un ou plusieurs atomes d'halogène ou par un groupe choisi parmi un perfluoroalkyle en C₁-C₄, un hydroxyle, un alcoxy en C₁-C₄ ou
2) R₄ et R₅ liés ensemble sont autres qu'un groupe de formule (CH₂)ₚY(CH₂)_{q} dans lequel Y est un groupe N-R₆ dans lequel R₆ est un hydrogène, un alkyle en C₁-C₄, un phénylalkyle dans lequel l'alkyle est en C₁-C₃ ; ou
3) R₄ et R₅ ensemble sont différents du groupe = CR₇R₈ lorsque R₃ est un hydrogène, un phényle, un méthylphényle ou un benzyle ;
4) n est différent de 6 ; ou bien
5) R₄ et R₅ liés ensemble sont autres qu'un groupe (CH₂)ₙ dans lequel n est compris entre 3 et 5, lorsque R₃ représente un groupe phényle substitué ;
B/ lorsque z = 1 et R₃ est un phényle, R₄ et R₅ sont chacun différent d'un méthyle ;
C/ lorsque z et t sont nuls et X est le soufre :
1) R₄ et R₅ sont autres qu'un phényle lorsque R₃ est un méthyle, un phényle, un chlorophényle, un méthoxyphényle ou un méthylphényle ; ou
2) R₄ et R₅ sont autres qu'un alkyle en C₁-C₃ lorsque R₃ est un méthyle ou un phényle; ou
3) R₄ et R₅ sont autres qu'un méthoxyphényle ou chlorophényle lorsque R₃ est phényle ; ou
4) R₄ et R₅ sont autres qu'un chlorophényle lorsque R₃ est un méthyle, caractérisé en ce qu'on fait agir sur un composé de formule : 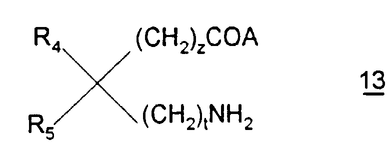
dans laquelle R₄, R₅ ont les définitions indiquées ci-dessus pour (II), A représente un groupe OH, un groupe NH₂ ou un groupe OR', R' étant l'hydrogène ou un alkyle en C₁-C₄, un composé de formule :
R₃ - B 14
dans laquelle R₃ a la définition indiquée ci-dessus pour (II) et B représente :
- un groupement C(OR)₃
- un groupement 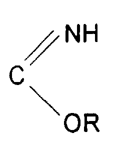 ou
- un groupement COHal,
R étant un alkyle en C₁-C₄ et Hal désignant un atome d'halogène, de préférence le chlore ;
puis éventuellement, le composé ainsi obtenu est traité par le réactif de Lawesson (bis/méthoxy-4 phényl)-2,4 dithia-1,3 diphosphétane-2,4 disulfure.

12. Procédé selon la revendication 11 pour l'obtention d'un composé de formule : 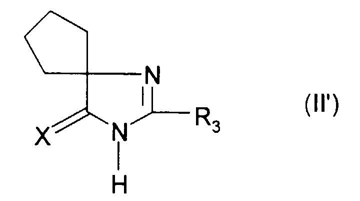 dans laquelle X représente un atome d'oxygène ou un atome de soufre et R₃ représente un hydrogène, un alkyle en C₁-C₆, non substitué ou substitué par un ou plusieurs atomes d'halogène ; un alcényle en C₂-C₆, un cycloalkyle en C₃-C₇, un phényle, un phénylalkyle dans lequel l'alkyle est en C₁-C₃, un phénylalcényle dans lequel l'alcényle est en C₂-C₃, lesdits groupes phényles étant non substitués ou substitués une ou plusieurs fois par un atome d'halogène, un alkyle en C₁-C₄, un halogénoalkyle en C₁-C₄, un polyhalogénoalkyle en C₁-C₄, un hydroxyle ou un alcoxy en C₁-C₄ ; à la condition que R₃ soit autre qu'un groupe phényle substitué lorsque X est l'oxygène, caractérisé en ce que l'on utilise un composé de formule 13 dans laquelle z et t sont nuls, R₄ et R₅ forment ensemble avec l'atome de carbone auquel ils sont liés un groupe spirocyclopentane et un composé de formule 14 dans laquelle R₃ est tel que défini ci-dessus.

13. Procédé selon la revendication 12, caractérisé en ce que l'on fait réagir un composé de formule 13 dans laquelle z et t sont nuls, R₄ et R₅ forment ensemble avec l'atome de carbone auquel ils sont liés un groupe spirocyclopentane avec un composé de formule 14 dans laquelle R₃ est le groupe n-butyle pour former le n-butyl-2 spirocyclopentane-4 imidazoline-2 one-5.

14. Procédé pour l'obtention du chlorydrate de n-butyl-2-spirocyclopcntane-4-imidazoline-2 one-5, caractérisé en ce qu'il consiste à ajouter de l'acide chlorhydrique concentré au composé obtenu selon le procédé de la revendication 13.

15. Procédé selon la revendication 11 pour l'obtention d'un composé de formule : 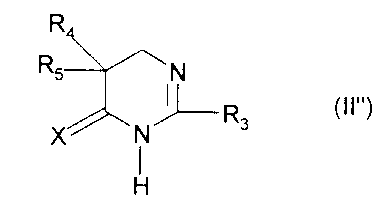 dans laquelle R₃, R₄, R₅ et X ont les définitions données ci-dessus pour (II) dans la revendication 11, caractérisé en ce que l'on utilise un composé de formule 13 dans laquelle t est 1 et z est nul, R₄ et R₅ sont tels que définis dans la revendication 11 et un composé de formule 14 dans laquelle R₃ est tel que défini dans la revendication 11.

16. Procédé selon la revendication 11 pour l'obtention d'un composé de formule : 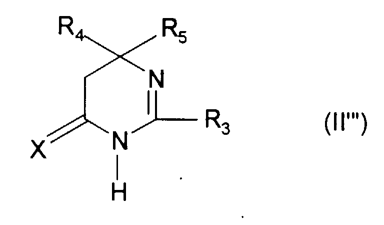 dans laquelle :
- R₃ représente un hydrogène, un alkyle en C₁-C₆, non substitué ou substitué par un ou plusieurs atomes d'halogène, un alcényle en C₂-C₆, un cycloalkyle en C₃-C₇, un phényle, un phénylalkyle dans lequel l'alkyle est en C₁-C₃, un phénylacényle dans lequel l'alcényle est en C₂-C₃, lesdits groupes phényles étant non substitués ou substitués une ou plusieurs fois par un atome d'halogène, un alkyle en C₁-C₄, un halogénoalkyle en C₁-C₄, un polyhalogénoalkyle en C₁-C₄, un hydroxyle ou un alcoxy en C₁-C₄ ;
- R₄ et R₅ représentent chacun indépendamment un alkyle en C₁-C₆, un phényle, un phénylalkyle dans lequel l'alkyle est C₁-C₃, lesdits groupes alkyle, phényle et phénylalkyle étant non substitués ou substitués par un ou plusieurs atomes d'halogène ou par un groupe choisi parmi un perfluoroalkyle en C₁-C₄, un hydroxyle, un alcoxy en C₁-C₄ ;
- ou R₄ et R₅ ensemble forment un groupe de formule =CR₇R₈, dans laquelle R₇ représente l'hydrogène, un alkyle en C₁-C₄ ou un phényle, et R₈ représente un alkyle en C₁-C₄ ou un phényle ;
- ou R₄ et R₅ liés ensemble représentent, soit un groupe de formule (CH₂)ₙ, soit un groupe de formule (CH₂)ₚY(CH₂)_{q}, dans lequel Y est, soit un atome d'oxygène, soit un atome de soufre, soit un atome de carbone substitué par un groupe alkyle en C₁-C₄, un phényle ou phénylalkyle dans lequel l'alkyle est en C₁-C₃, soit un groupe N-R₆ dans lequel R₆ représente un hydrogène, un alkyle en C₁-C₄, un phénylalkyle dans lequel l'alkyle est en C₁-C₄, un alkylcarbonyle en C₁-C₄, un halogénoalkylcarbonyle en C₁-C₄, un polyhalogénoalkylcarbonyle en C₁-C₄, un benzoyle, un alpha aminoacyle ou un groupe N-protecteur, ou R₄ et R₅ liés ensemble avec l'atome de carbone auquel ils sont liés constituent un indane ou un adamantane ;
- p + q = m ;
- n est un nombre entier compris entre 2 et 11 ;
- m est un nombre entier compris entre 2 et 5 ;
- X représente un atome d'oxygène ou un atome de soufre;
avec la limitation que R₃ est autre qu'un phényle lorsque R₄ et R₅ représentent chacun un méthyle, caractérisé en ce que l'on utilise un composé de formule 13 dans laquelle z = 1 et t est nul, R₄ et R₅ sont tels que définis ci-dessus et un composé de formule 13 dans laquelle R₃ est tel que défini ci-dessus.

17. Procédé pour la préparation d'une composition pharmaceutique, caractérisé en ce qu'il consiste à mélanger un composé obtenu par le procédé selon l'une quelconque des revendications 1 à 10 avec un véhicule pharmaceutiquement acceptable.

18. Procédé pour la préparation d'une composition pharmaceutique, caractérisé en ce qu'il consiste à associer un composé obtenu par le procédé selon l'une quelconque des revendications 1 à 10 avec un composé bêtabloquant.

19. Procédé pour la préparation d'une composition pharmaceutique, caractérisé en qu'il consiste à associer un composé obtenu par le procédé selon l'une quelconque des revendications 1 à 10 avec un diurétique.

20. Procédé pour la préparation d'une composition pharmaceutique, caractérisé en ce qu'il consiste à associer un composé obtenu par le procédé selon l'une quelconque des revendications 1 à 10 avec un anti-inflammatoire non stéroïdien.

21. Procédé pour la préparation d'une composition pharmaceutique, caractérisé en ce qu'il consiste à associer un composé obtenu par le procédé selon l'une quelconque des revendications 1 à 10 avec un antagoniste calcique.

22. Procédé pour la préparation d'une composition pharmaceutique, caractérisé en ce qu'il consiste à associer un composé obtenu par le procédé selon l'une quelconque des revendications 1 à 10 avec un tranquillisant.

## Revendications (Revendications pour l'(les) Etat(s) contractant(s) suivant(s): GR)

1. Un composé de formule : 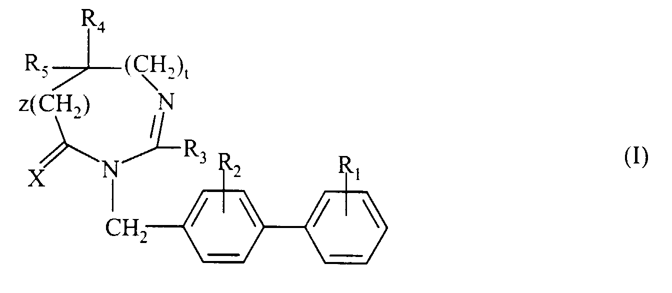 dans laquelle:
- R₁ et R₂ sont semblables ou différents et représentent chacun indépendamment l'hydrogène ou un groupe choisi parmi un alkyle en C₁-C₆, un alcoxy en C₁-C₄, un amino, un aminométhyle, un carboxy, un alcoxycarbonyle dans lequel l'alcoxy est en C₁-C₄, un cyano, un tétrazolyle, un méthyltétrazolyle, un méthylsulfonylamino, un trifluorométhylsulfonylamino, un trifluorométhylsulfonylaminométhyle, un N-cyanocarbamoyle, un N-hydroxycarbamoyle, un N-((carboxy-4) thiazol-1,3-yl-2)carbamoyle, un uréido, un cyano-2 guanidinocarbonyle, un cyano-2 guanidinométhyle, un imizadol-1-yl-carbonyle, un cyano-3 méthyl-2 isothiouréidométhyle, à la condition qu'au moins l'un des substituants R₁ ou R₂ soit différent de l'hydrogène ;
- R₃ représente un hydrogène, un alkyle en C₁-C₆, non substitué ou substitué par un ou plusieurs atomes d'halogène, un alcényle en C₂-C₆, un cycloalkyle en C₃-C₇, un phényle, un phénylalkyle dans lequel l'alkyle est en C₁-C₃, un phénylalcényle dans lequel l'alcényle est en C₂-C₃, lesdits groupes phényles étant non substitués ou substitués une ou plusieurs fois par un atome d'halogène, un alkyle en C₁-C₄, un halogénoalkyle en C₁-C₄, un polyhalogénoalkyle en C₁-C₄, un hydroxyle ou un alcoxy en C₁-C₄;
- R₄ et R₅ représentent chacun indépendamment un alkyle en C₁-C₆, un phényle, un phénylalkyle dans lequel l'alkyle est en C₁-C₃, lesdits groupes alkyle, phényle et phénylalkyle étant non substitués ou substitués par un ou plusieurs atomes d'halogène ou par un groupe choisi parmi un perfluoroalkyle en C₁-C₄, un hydroxyle, un alcoxy en C₁-C₄ ;
- ou R₄ et R₅ ensemble forment un groupe de formule =CR₇R₈, dans laquelle R₇ représente l'hydrogène, un alkyle en C₁-C₄ ou un phényle, et R₈ représente un alkyle en C₁-C₄ ou un phényle ;
- ou encore R₄ et R₅ liés ensemble représentent, soit un groupe de formule (CH₂)ₙ, soit un groupe de formule (CH₂)ₚY(CH₂)_{q}, dans lequel Y est, soit un atome d'oxygène, soit un atome de soufre, soit un atome de carbone substitué par un groupe alkyle en C₁-C₄, un phényle ou un phénylalkyle dans lequel l'alkyle est en C₁-C₃, soit un groupe N-R₆ dans lequel R₆ représente un hydrogène, un alkyle en C₁-C₄, un phénylalkyle dans lequel l'alkyle est en C₁-C₃, un alkylcarbonyle en C₁-C₄, un halogénoalkylcarbonyle en C₁-C₄, un polyhalogénoalkylcarbonyle en C₁-C₄, un benzoyle, un alpha aminoacyle ou un groupe N-protecteur, ou R₄ et R₅ liés ensemble avec l'atome de carbone auquel ils sont liés constituent un indane ou un adamantane ;
- p + q = m ;
- n est un nombre entier compris entre 2 et 11 ;
- m est un nombre entier compris entre 2 et 5 ;
- X représente un atome d'oxygène ou un atome de soufre ;
- z et t son nuls ou l'un est nul et l'autre représente un ;
et ses sels.

2. Composé selon la revendication 1, caractérisé en ce que R₁ est en position ortho et représente un groupe carboxy ou tétrazolyle et R₂ est l'hydrogène.

3. Composé selon l'une quelconque des revendications 1 et 2, caractérisé en ce que R₄ et R₅ constituent avec le carbone auquel ils sont liés un cyclopentane ou un cyclohexane.

4. Composé selon l'une quelconque des revendications 1 à 3, caractérisé en ce que R₃ représente un groupe alkyle droit en C₁-C₆.

5. Composé selon l'une quelconque des revendications 1 à 4, caractérisé en ce que X est l'oxygène.

6. Composé selon l'une quelconque des revendications 1 à 5, caractérisé en ce que z = t = 0.

7. Composé selon la revendication 1, caractérisé en ce qu'il est le n-butyl-2 spirocyclopentane-4 [((tétrazolyl-5)-2' biphényl-4-yl) méthyl]-1 imidazoline-2 one-5 ou l'un de ses sels avec des acides ou des bases.

8. Procédé pour la préparation d'un composé (I) selon l'une quelconque des revendications 1 à 7, caractérisé en ce que :
a1) on fait réagir un dérivé hétérocyclique de formule : 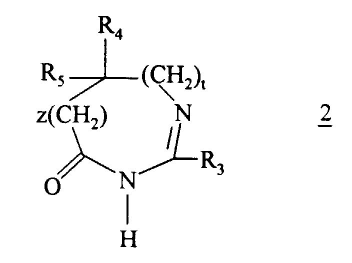 dans laquelle z, t, R₃, R₄ et R₅ ont les significations indiquées pour (I) dans la revendication 1, sur un dérivé du (biphényl-4-yl) méthyl de formule : 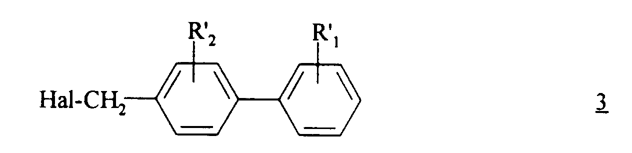 dans laquelle Hal représente un atome d'halogène et R'₁ et R'₂ représentent respectivement soit R₁ et R₂, soit un groupement précurseur de R₁ et R₂ ;
b1) éventuellement, le composé ainsi obtenu de formule : 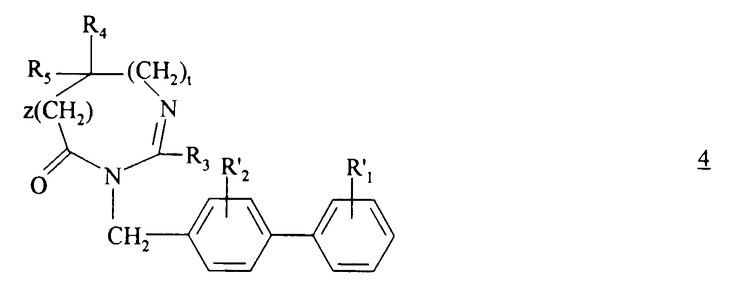 est traité par le réactif de Lawesson [bis(méthoxy-4 phényl)-2,4 dithia-1,3 diphosphétane-2,4 disulfure 2,4];
c1) le composé obtenu en a1) ou en b1), de formule : 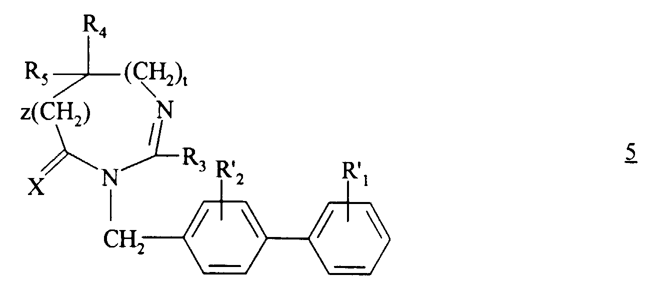 dans laquelle X représente un atome d'oxygène ou un atome de soufre, est traité pour préparer le composé (I) par transformation des groupes R'₁ et/ou R'_{2,} en respectivement, les groupes R₁ et/ou R₂, qui est ensuite éventuellement transformé en l'un de ses sels.

9. Procédé pour la préparation d'un composé (I), selon l'une quelconque des revendications 1 à 7, caractérisé en ce que :
a2) on fait réagir un aminoacide de formule : 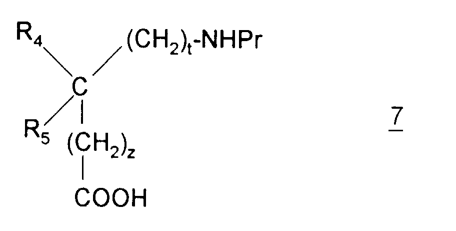 dans laquelle z, t, R₄ et R₅ ont les significations indiquées pour (I) dans la revendication 1 et dont la fonction amine est protégée par le groupe Pr, sur un dérivé (biphényl-4-yl) méthylamine de formule : 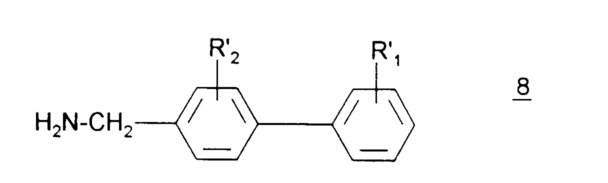 dans laquelle R'₁ et R'₂ représentent respectivement soit R₁ et R₂, soit un groupement précurseur de R₁ et R₂ ;
b2) après déprotection de l'amine, le composé ainsi obtenu de formule : 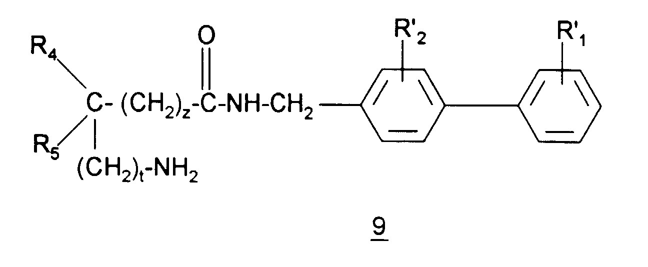 est ensuite traité par un ortho-ester d'alkyle de formule R₃C(OR)₃ (10) dans laquelle R₃ a la signification indiquée pour (I) dans la revendication 1 et R est un alkyle en C₁-C₄ ;
c2) éventuellement, le composé ainsi obtenu de formule : 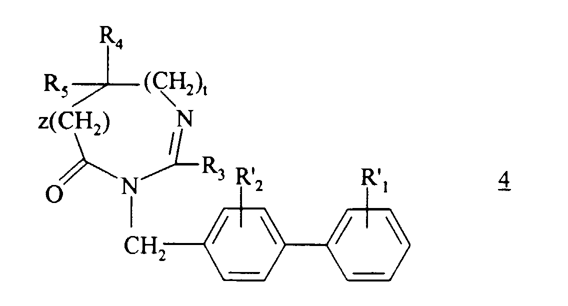 est traité par le réactif de Lawesson [bis(méthoxy-4 phényl)-2,4 dithia-1,3 diphosphétane-2,4 disulfure-2,4] ;
d2) le composé ainsi obtenu en b2 ou en c2 de formule 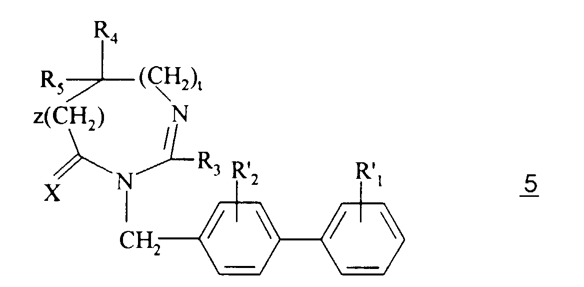 est ensuite traité dans des conditions convenables pour préparer le composé (I) par transformation des groupes R'₂ et/ou R'₁ en, respectivement, les groupes R₂ et/ou R₁, qui est ensuite éventuellement transformé en l'un de ses sels.

10. Procédé pour la préparation d'un composé (I) selon la revendication 6 caractérisé en ce que :
a3) on fait agir sur un dérivé de l'imidazole de formule : 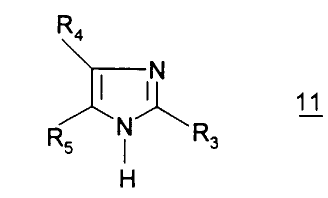 dans laquelle R₃, R₄, R₅ ont les significations indiquées pour (I) dans la revendication 1,
un dérivé du (biphényl-4-yl) méthyl de formule : 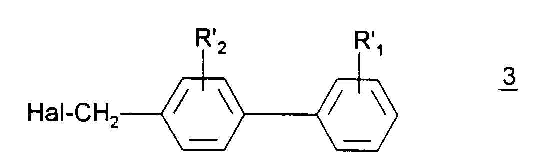 dans laquelle Hal représente un atome d'halogène et R'₁ et R'₂ représentent respectivement soit R₁ et R₂, soit un groupement précurseur de R₁ et R₂, en présence d'oxygène et d'une irradiation UV, en milieu basique ;
b3) éventuellement, le composé ainsi obtenu de formule : 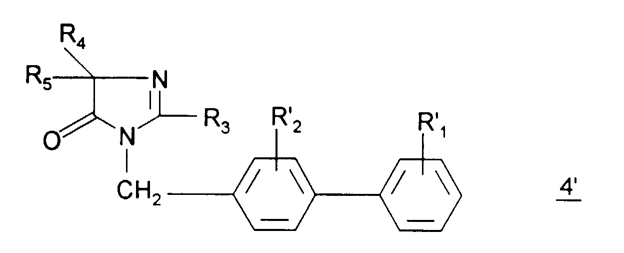 est traité par le réactif de Lawesson [bis(méthoxy-4 phényl)-2,4 dithia-1,3 diphosphétane-2,4 disulfure-2,4] ;
c3) le composé ainsi obtenu en b3 ou en c3 de formule : 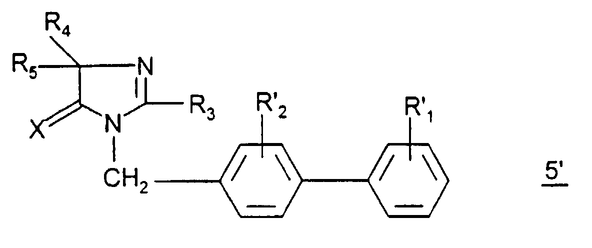 est ensuite traité dans des conditions convenables pour préparer le composé (I) par transformation des groupes R'₁ et/ou R'₂ en respectivement les groupes R₁ et/ou R₂, qui est ensuite éventuellement transformé en l'un de ses sels.

11. Un composé de formule : 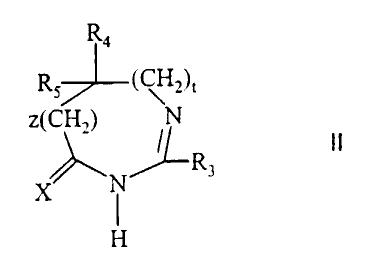 dans laquelle :
- R₃ représente un hydrogène, un alkyle en C₁-C₆, non substitué ou substitué par un ou plusieurs atomes d'halogène, un alcényle en C₂-C₆, un cycloalkyle en C₃-C₇, un phényle, un phénylalkyle dans lequel l'alkyle est en C₁-C₃, un phénylalcényle dans lequel l'alcényle est en C₂-C₃, lesdits groupes phényles étant non substitués ou substitués une ou plusieurs fois par un atome d'halogène, un alkyle en C₁-C₄, un halogénoalkyle en C₁-C₄, un polyhalogénoalkyle en C₁-C₄, un hydroxyle ou un alcoxy en C₁-C₄ ; R₄ et R₅ représentent chacun indépendamment un alkyle en C₁-C₆, un phényle, un phénylalkyle dans lequel l'alkyle est en C₁-C₃, lesdits groupes alkyle, phényle et phénylalkyle étant non substitués ou substitués par un ou plusieurs atomes d'halogène ou par un groupe choisi parmi un perfluoroalkyle en C₁-C₄, un hydroxyle, un alcoxy en C₁-C₄ ;
- ou R₄ et R₅ ensemble forment un groupe de formule =CR₇R₈, dans laquelle R₇ représente l'hydrogène, un alkyle en C₁-C₄ ou un phényle, et R₈ représente un alkyle en C₁-C₄ ou un phényle ;
- ou encore R₄ et R₅ liés ensemble représentent, soit un groupe de formule (CH₂)ₙ, soit un groupe de formule (CH₂)ₚY(CH₂)_{q}, dans lequel Y est soit un atome d'oxygène, soit un atome de soufre, soit un atome de carbone substitué par un groupe alkyle en C₁-C₄, un phényle ou un phénylalkyle dans lequel l'alkyle est en C₁-C₃, soit un groupe N-R₆ dans lequel R₆ représente un hydrogène, un alkyle en C₁-C₄, un phénylalkyle dans lequel l'alkyle est en C₁-C₃, un alkylcarbonyle en C₁-C₄, un halogénoalkylcarbonyle en C₁-C₄, un polyhalogénoalkylcarbonyle en C₁-C₄, un benzoyle, un alphaaminoacyle ou un groupe N-protecteur, ou R₄ et R₅ liés ensemble avec l'atome de carbone auquel ils sont liés constituent un indane ou un adamantane ;
- p + q = m ;
- n est un nombre entier compris entre 2 et 11 ;
- m est un nombre entier compris entre 2 et 5 ;
- X représente un atome d'oxygène ou un atome de soufre ;
- z et t sont nuls ou l'un est nul et l'autre représente un ;
avec la limitation que
A/ lorsque z et t sont nuls et X représente l'oxygène :
1) R₄ et R₅ sont autres qu'un alkyle en C₁-C₆ ; un phényle, un phénylalkyle dans lequel l'alkyle est en C₁-C₃, lesdits groupes alkyle, phényle et phénylalkyle étant non substitués ou substitués par un ou plusieurs atomes d'halogène ou par un groupe choisi parmi un perfluoroalkyle en C₁-C₄, un hydroxyle, un alcoxy en C₁-C₄ ; ou
2) R₄ et R₅ liés ensemble sont autres qu'un groupe de formule (CH₂)ₚY(CH₂)_{q} dans lequel Y est un groupe N-R₆ dans lequel R₆ est un hydrogène, un alkyle en C₁-C₄, un phénylalkyle dans lequel l'alkyle est en C₁-C₃; ou
3) R₄ et R₅ ensemble sont différents du groupe = CR₇R₈ lorsque R₃ est un hydrogène, un phényle, un méthylphényle ou un benzyle ;
4) n est différent de 6 ; ou bien
5) R₄ et R₅ liés ensemble sont autres qu'un groupe (CH₂)ₙ dans lequel n est compris entre 3 et 5, lorsque R₃ représente un groupe phényle substitué ;
B/ lorsque z = 1 et R₃ est un phényle, R₄ et R₅ sont chacun différent d'un méthyle ;
C/ lorsque z et t sont nuls et X est le soufre :
1) R₄ et R₅ sont autres qu'un phényle lorsque R₃ est un méthyle, un phényle, un chlorophényle, un méthoxyphényle ou un méthylphényle ; ou
2) R₄ et R₅ sont autres qu'un alkyle en C₁-C₃ lorsque R₃ est un méthyle ou un phényle; ou
3) R₄ et R₅ sont autres qu'un méthoxyphényle ou chlorophényle lorsque R₃ est phényle ; ou
4) R₄ et R₅ sont autres qu'un chlorophényle lorsque R₃ est un méthyle.

12. Un composé selon la revendication 11 de formule : 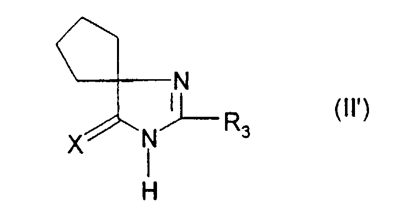 dans laquelle X représente un atome d'oxygène ou un atome de soufre et R₃ représente un hydrogène, un alkyle en C₁-C₆, non substitué ou substitué par un ou plusieurs atomes d'halogène ; un alcényle en C₂-C₆, un cycloalkyle en C₃-C₇, un phényle, un phénylalkyle dans lequel l'alkyle est en C₁-C₃, un phénylalcényle dans lequel l'alcényle est en C₂-C₃, lesdits groupes phényles étant non substitués ou substitués une ou plusieurs fois par un atome d'halogène, un alkyle en C₁-C₄, un halogénoalkyle en C₁-C₄, un polyhalogénoalkyle en C₁-C₄, un hydroxyle ou un alcoxy en C₁-C₄ ; à la condition que R₃ soit autre qu'un groupe phényle substitué lorsque X est l'oxygène.

13. Composé selon la revendication 12, caractérisé en ce que, dans la formule II', X est l'oxygène et R₃ est le n-butyle et en ce qu'il est le n-butyl-2 spirocyclopentane-4 imidazoline-2 one-5.

14. Chlorhydrate de n-butyl-2 spirocyclopentane-4 imidazoline-2 one-5.

15. Un composé selon la revendication 11, de formule : 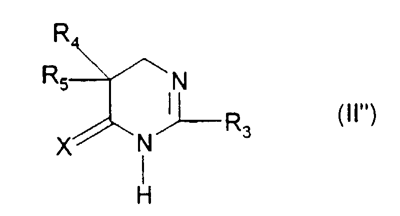 dans laquelle R₃, R_{4,} R₅ et X ont les définitions données ci-dessus pour (II) dans la revendication 11.

16. Un composé selon la revendication 11 de formule : 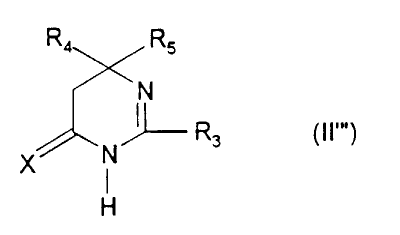 dans laquelle :
- R₃ représente un hydrogène, un alkyle en C₁-C₆, non substitué ou substitué par un ou plusieurs atomes d'halogène, un alcényle en C₂-C₆, un cycloalkyle en C₃-C₇, un phényle, un phénylalkyle dans lequel l'alkyle est en C₁-C₃, un phénylacényle dans lequel l'alcényle est en C₂-C₃, lesdits groupes phényles étant non substitués ou substitués une ou plusieurs fois par un atome d'halogène, un alkyle en C₁-C₄, un halogénoalkyle en C₁-C₄, un polyhalogénoalkyle en C₁-C₄, un hydroxyle ou un alcoxy en C₁-C₄ ;
- R₄ et R₅ représentent chacun indépendamment un alkyle en C₁-C₆, un phényle, un phénylalkyle dans lequel l'alkyle est C₁-C₃, lesdits groupes alkyle, phényle et phénylalkyle étant non substitués ou substitués par un ou plusieurs atomes d'halogène ou par un groupe choisi parmi un perfluoroalkyle en C₁-C₄, un hydroxyle, un alcoxy en C₁-C₄ ;
- ou R₄ et R₅ ensemble forment un groupe de formule =CR₇R₈, dans laquelle R₇ représente l'hydrogène, un alkyle en C₁-C₄ ou un phényle, et R₈ représente un alkyle en C₁-C₄ ou un phényle ;
- ou R₄ et R₅ liés ensemble représentent, soit un groupe de formule (CH₂)ₙ, soit un groupe de formule (CH₂)ₚY(CH₂)_{q}, dans lequel Y est, soit un atome d'oxygène, soit un atome de soufre, soit un atome de carbone substitué par un groupe alkyle en C₁-C₄, un phényle ou phénylalkyle dans lequel l'alkyle est en C₁-C₃, soit un groupe N-R₆ dans lequel R₆ représente un hydrogène, un alkyle en C₁-C₄, un phénylalkyle dans lequel l'alkyle est en C₁-C₄, un alkylcarbonyle en C₁-C₄, un halogénoalkylcarbonyle en C₁-C₄, un polyhalogénoalkylcarbonyle en C₁-C₄, un benzoyle, un alpha aminoacyle ou un groupe N-protecteur, ou R₄ et R₅ liés ensemble avec l'atome de carbone auquel ils sont liés constituent un indane ou un adamantane ;
- p + q = m ;
- n est un nombre entier compris entre 2 et 11 ;
- m est un nombre entier compris entre 2 et 5 ;
- X représente un atome d'oxygène ou un atome de soufre;
avec la limitation que R₃ est autre qu'un phényle lorsque R₄ et R₅ représentent chacun un méthyle.

17. Procédé pour la préparation d'un composé selon l'une quelconque des revendications 11 à 16, caractérisé en ce que : on fait agir sur un composé de formule : 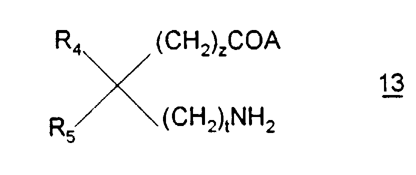 dans laquelle R₄, R₅ ont les définitions indiquées ci-dessus pour (II) dans la revendication 11, A représente un groupe OH, un groupe NH₂ ou un groupe OR', R' étant l'hydrogène ou un alkyle en C₁-C₄, un composé de formule :
R₃ - B 14
dans laquelle R₃ a la définition indiquée dans les revendications 11 à 16 et B représente :
- un groupement C (OR)₃
- un groupement 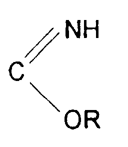 ou
- un groupement COHal,
R étant un alkyle en C₁-C₄ et Hal désignant un atome d'halogène, de préférence le chlore ;
puis éventuellement, le composé ainsi obtenu est traité par le réactif de Lawesson (bis/méthoxy-4 phényl)-2,4 dithia-1,3 diphosphétane-2,4 disulfure.

18. Procédé pour la préparation d'une composition pharmaceutique, caractérisé en ce qu'il consiste à mélanger un composé selon l'une quelconque des revendications 1 à 7 avec un véhicule pharmaceutiquement acceptable.

19. Procédé pour la préparation d'une composition pharmaceutique, caractérisé en qu'il consiste à associer un composé selon l'une quelconque des revendications 1 à 7 avec un composé bêtabloquant.

20. Procédé pour la préparation d'une composition pharmaceutique, caractérisé en ce qu'il consiste à associer un composé selon l'une quelconque des revendications 1 à 7 avec un diurétique.

21. Procédé pour la préparation d'une composition pharmaceutique, caractérisé en ce qu'il consiste à associer un composé selon l'une quelconque des revendications 1 à 7 avec un anti-inflammatoire non stéroïdien.

22. Procédé pour la préparation d'une composition pharmaceutique, caractérisé en ce qu'il consiste à associer un composé selon l'une quelconque des revendications 1 à 7 avec un antagoniste calcique.

23. Procédé pour la préparation d'une composition pharmaceutique, caractérisé en ce qu'il consiste à associer un composé selon l'une quelconque des revendications 1 à 7 avec un tranquillisant.

## Claims (Claims for the following Contracting State(s): AT, BE, CH, DE, DK, FR, GB, IT, LI, LU, NL, SE)

1. A compound of the formula 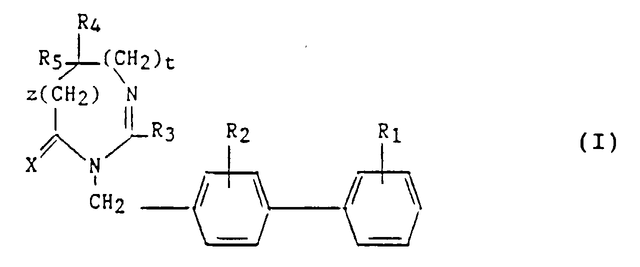 in which:
- R₁ and R₂ are similar or different and are each independently hydrogen or a group selected from a C₁-C₆ alkyl, a C₁-C₄ alkoxy, an amino, an aminomethyl, a carboxyl, an alkoxycarbonyl in which the alkoxy is C₁-C₄, a cyano, a tetrazolyl, a methyltetrazolyl, a methylsulfonylamino, a trifluoromethylsulfonylanino, a trifluoromethylsulfonylaminomethyl, an N-cyanocarbamoyl, an N-hydroxycarbamoyl, an N-(4-carboxy-1,3-thiazol-2-yl)carbamoyl, a ureido, a 2-cyanoguanidinocarbonyl, a 2-cyanoguanidinomethyl, an imidazol-1-ylcarbonyl, a 3-cyano-2-methylisothioureidomethyl, with the proviso that at least one of the substituents R₁ or R₂ is other than hydrogen;
- R₃ is a hydrogen, a C₁-C₆ alkyl which is unsubstituted or substituted by one or more halogen atoms, a C₂-C₆ alkenyl, a C₃-C₇ cycloalkyl, a phenyl, a phenylalkyl in which the alkyl is C₁-C₃, a phenylalkenyl in which the alkenyl is C₂-C₃, said phenyl groups being unsubstituted or monosubstituted or polysubstituted by a halogen atom, a C₁-C₄ alkyl, a C₁-C₄ halogenoalkyl, a C₁-C₄ polyhalogenoalkyl, a hydroxyl or a C₁-C₄ alkoxy;
- R₄ and R₅ are each independently a C₁-C₆ alkyl, a phenyl, a phenylalkyl in which the alkyl is C₁-C₃, said alkyl, phenyl and phenylalkyl groups being unsubstituted or substituted by one or more halogen atoms or by a group selected from a C₁-C₄ perfluoroalkyl, a hydroxyl, a C₁-C₄ alkoxy;
- or R₄ and R₅ together form a group of the formula =CR₇R₈, in which R₇ is hydrogen, a C₁-C₄ alkyl or a phenyl and R₈ is a C₁-C₄ alkyl or a phenyl;
- or else R₄ and R₅ bonded together are either a group of the formula (CH₂)ₙ or a group of the formula (CH₂)ₚY-(CH₂)_{q}, in which Y is either an oxygen atom, or a sulfur atom, or a carbon atom substituted by a C₁-C₄ alkyl group, a phenyl or a phenylalkyl in which the alkyl is C₁-C₃, or a group N-R₆, in which R₆ is a hydrogen, a C₁-C₄ alkyl, a phenylalkyl in which the alkyl is C₁-C₃, a C₁-C₄ alkylcarbonyl, a C₁-C₄ halogenoalkylcarbonyl, a C₁-C₄ polyhalogenoalkylcarbonyl, a benzoyl, an alpha-aminoacyl or an N-protecting group, or R₄ and R₅, bonded together with the carbon atom to which they are bonded, form an indane or an adamantane;
- p + q = m;
- n is an integer between 2 and 11;
- m is an integer between 2 and 5;
- X is an oxygen atom or sulfur atom;
- z and t are zero or one is zero and the other is one; and its salts.

2. A compound according to claim 1, characterized in that R₁ is in the ortho position and is a carboxyl or tetrazolyl group and R₂ is hydrogen.

3. A compound according to any one of claims 1 and 2,characterized in that R₄ and R₅ form a cyclopentane or cyclohexane with the carbon to which they are bonded.

4. A compound according to any one of claims 1 to 3, characterized in that R₃ is a linear C₁-C₆ alkyl group.

5. A compound according to any one of claims 1 to 4, characterized in that X is oxygen.

6. A compound according to any one of claims 1 to 5, characterized in that z = t = 0.

7. A compound according to claim 1, characterized in that it is the 2-n-butyl-4-spirocyclopentane-1-( (2'-(tetrazol-5-yl)biphenyl-4-yl)methyl)-2-imidazolin-5-one or one of its salts with acids or bases.

8. A method of preparing a compound (I) according to any one of claims 1 to 7, characterized in that:
a1) a heterocyclic derivative of the formula 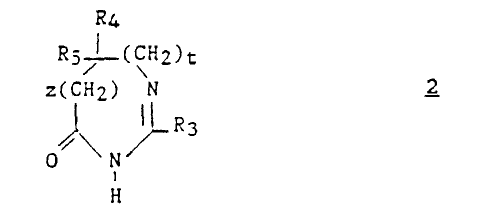 in which z, t, R₃, R₄ and R₅ have the meanings indicated for (I) in claim 1, is reacted with a (biphenyl-4-yl)methyl derivative of the formula 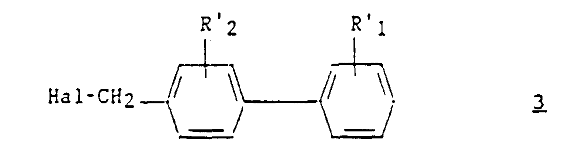 in which Hal is a halogen atom and R'₁ and R'₂ are respectively either R₁ and R₂ or a precursor group of R₁ and R₂;
b1) if appropriate, the thus obtained compound of the formula 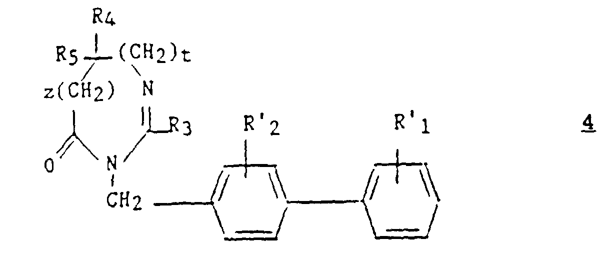 is treated with Lawesson's reagent [2,4-bis(4-methoxyphenyl)-1,3-dithia-2,4-diphosphetane 2,4-disulfide];
c1) the compound obtained in a1) or b1), of the formula 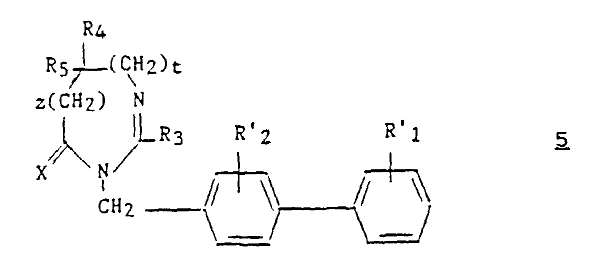 in which X is an oxygen atom or a sulfur atom, is treated to prepare the compound (I) by conversion of the groups R'₁ and/or R'₂ to the groups R₁ and/or R₂ respectively, which is then possibly converted into one of its salts.

9. A method of preparing a compound (I) according to any one of claims 1 to 7, characterized in that
a2) an amino acid of the formula 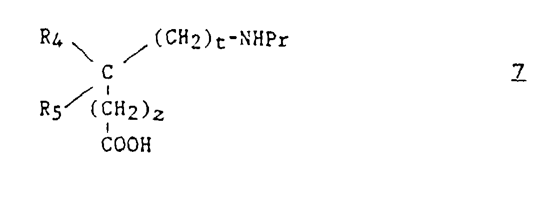 in which z, t, R₄ and R₅ have the meanings indicated for (I) in claim 1, and of which the amine group is protected by the Pr group, is reacted with a (biphenyl-4-yl)methyl-amine derivative of the formula 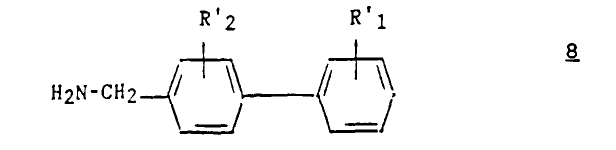 in which R'₁ and R'₂ are respectively either R₁ and R₂ or a precursor group of R₁ and R₂;
b2) after deprotection of the amine, the thus obtained compound of the formula 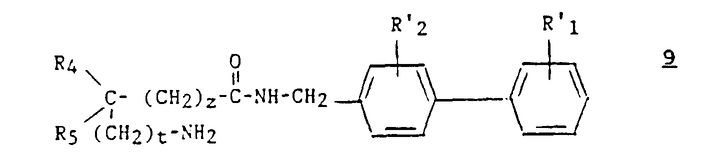 is then treated with an alkyl ortho-ester of the formula R₃C(OR)₃ (10), in which R₃ has the meaning indicated for (I) in claim 1 and R is a C₁-C₄ alkyl;
c2) if appropriate, the thus obtained compound of the formula 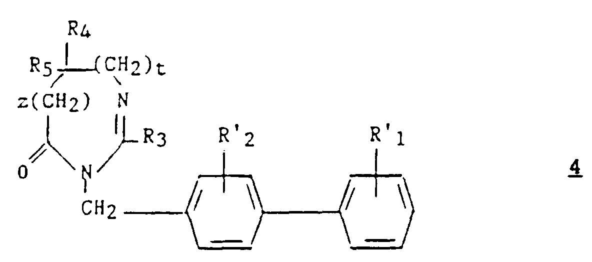 is treated with Lawesson's reagent [2,4-bis(4-methoxyphenyl)-1,3-dithia-2,4-diphosphetane 2,4-disulfide]; and
d2) the compound thus obtained in b2 or c2, of the formula 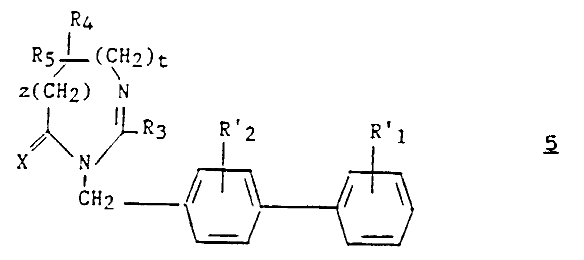 is then treated under suitable conditions for preparing the compound (I) by conversion of the groups R'₂ and/or R'₁ to the groups R₂ and/or R₁ respectively which is then possibly converted into one of its salts.

10. A method of preparing a compound (I) according to claim 6, characterized in that
a3) a (biphenyl-4-yl)methyl derivative of the formula 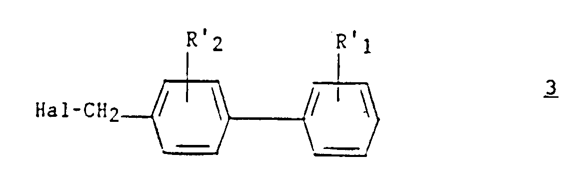 in which Hal is a halogen atom and R'₁ and R'₂ are respectively either R₁ and R₂ or a precursor group of R₁ and R₂, is reacted with an imidazole derivative of the formula 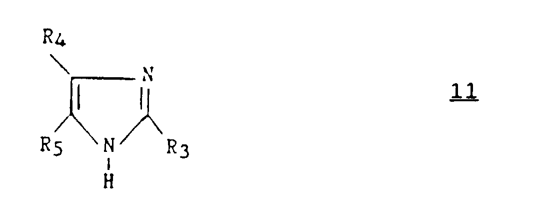 in which R₃, R₄ and R₅ are as defined for (I) in claim 1, in the presence of oxygen and UV irradiation and in a basic medium;
b3) if appropriate, the thus obtained compound of the formula 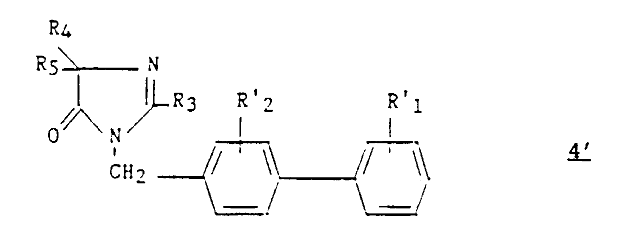 is treated with Lawesson's reagent [2,4-bis(4-methoxyphenyl)-1,3-dithia-2,4-diphosphetane 2,4-disulfide]; and
c3) the compound thus obtained in b3 or c3, of the formula 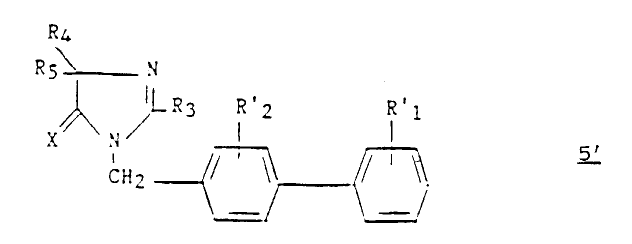 is then treated under suitable conditions for preparing the compound (I) by conversion of the groups R'₁ and/or R'₂ to the groups R₁ and/or R₂ respectively, which is then possibly converted into one of its salts.

11. A compound of the formula 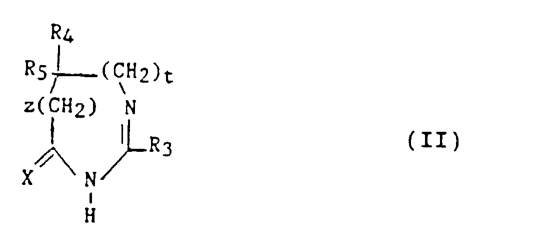 in which:
- R₃ is a hydrogen, a C₁-C₆ alkyl which is unsubstituted or substituted by one or more halogen atoms, a C₂-C₆ alkenyl, a C₃-C₇ cycloalkyl, a phenyl, a phenylalkyl in which the alkyl is C₁-C₃, a phenylalkenyl in which the alkenyl is C₂-C₃, said phenyl groups being unsubstituted or monosubstituted or polysubstituted by a halogen atom, a C₁-C₄ alkyl, a C₁-C₄ halogenoalkyl, a C₁-C₄ polyhalogenoalkyl, a hydroxyl or a C₁-C₄ alkoxy; R₄ and R₅ are each independently a C₁-C₆ alkyl, a phenyl, a phenylalkyl in which the alkyl is C₁-C₃, said alkyl, phenyl and phenylalkyl groups being unsubstituted or substituted by one or more halogen atoms or by a group selected from a C₁-C₄ perfluoroalkyl, a hydroxyl, a C₁-C₄ alkoxy;
- or R₄ and R₅ together form a group of the formula =CR₇R₈, in which R₇ is hydrogen, a C₁-C₄ alkyl or a phenyl and R₈ is a C₁-C₄ alkyl or a phenyl;
- or else R₄ and R₅ bonded together are either a group of the formula (CH₂)ₙ or a group of the formula (CH₂)ₚY-(CH₂)_{q}, in which Y is either an oxygen atom, or a sulfur atom, or a carbon atom substituted by a C₁-C₄ alkyl group, a phenyl or a phenylalkyl in which the alkyl is C₁-C₃, or a group N-R₆, in which R₆ is a hydrogen, a C₁-C₄ alkyl, a phenylalkyl in which the alkyl is C₁-C₃, a C₁-C₄ alkylcarbonyl, a C₁-C₄ halogenoalkylcarbonyl, a C₁-C₄ polyhalogenoalkylcarbonyl, a benzoyl, an alpha-aminoacyl or an N-protecting group, or R₄ and R₅, bonded together with the carbon atom to which they are bonded, form an indane or an adamantane;
- p + q = m;
- n is an integer between 2 and 11;
- m is an integer between 2 and 5;
- X is an oxygen atom or sulfur atom;
- z and t are zero or one is zero and the other is one; with the limitation that
A/ if z and t are zero and X is oxygen:
1) R₄ and R₅ are other than a C₁-C₆ alkyl, a phenyl, a phenylalkyl in which the alkyl is C₁-C₃, said alkyl, phenyl and phenylalkyl groups being unsubstituted or substituted by one or more halogen atoms or by a group selected from a C₁-C₄ perfluoroalkyl, a hydroxyl, a C₁-C₄ alkoxy; or
2) R₄ and R₅ bonded together are other than a group of formula (CH₂)ₚY(CH₂)_{q} in which Y is a group N-R₆ in which R₆ is a hydrogen, a C₁-C₄ alkyl or a phenylalkyl in which the alkyl is C₁-C₃ ; or
3) R₄ and R₅ together are different from the group =CR₇R₈ when R₃ is a hydrogen, a phenyl, a methylphenyl or a benzyl;
4) n is different from 6; or else
5) R₄ and R₅ bonded together are other than a group (CH₂)ₙ in which n is comprised between 3 and 5, when R₃ is a substituted phenyl group;
B/ when z = 1 and R₃ is a phenyl, R₄ and R₅ are each other than a methyl;
C/ when z and t are zero and X is sulphur:
1) R₄ and R₅ are other than a phenyl when R₃ is a methyl, a phenyl, a chlorophenyl, a methoxyphenyl or a methylphenyl; or
2) R₄ and R₅ are other than a C₁-C₃ alkyl when R₃ is a methyl or a phenyl; or
3) R₄ and R₅ are other than a methoxyphenyl or chlorophenyl when R₃ is phenyl; or
4) R₄ and R₅ are other than a chlorophenyl when R₃ is a methyl.

12. A compound according to claim 11 of the formula 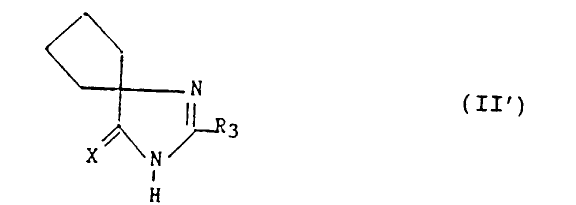 in which X is an oxygen atom or a sulfur atom and R₃ is a hydrogen, a C₁-C₆ alkyl which is unsubstituted or substituted by one or more halogen atoms; a C₂-C₆ alkenyl, a C₃-C₇ cycloalkyl, a phenyl, a phenylalkyl in which the alkyl is C₁-C₃, a phenylalkenyl in which the alkenyl is C₂-C₃, said phenyl groups being unsubstituted or monosubstituted or polysubstituted by a halogen atom, a C₁-C₄ alkyl, a C₁-C₄ halogenoalkyl, a C₁-C₄ polyhalogenoalkyl, a hydroxyl or a C₁-C₄ alkoxy. with the proviso that R₃ is other than a phenyl group that is substituted when X is oxygen.

13. A compound according to claim 12, characterized in that, in formula II', X is oxygen and R₃ is n-butyl and in that it is the 2-n-butyl-4-spirocyclopentane-2-imidazolin-5-one.

14. 2-n-Butyl-4-spirocyclopentane-2-imidazolin-5-one hydrochloride.

15. A compound according to claim 11 of the formula 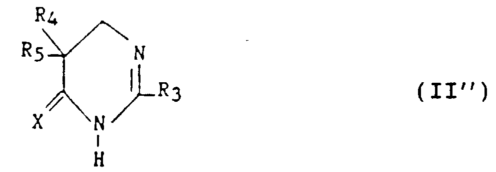 in which R₃, R₄, R₅ and X have the meanings given above for (II) in claim 11.

16. A compound according to claim 11 of the formula 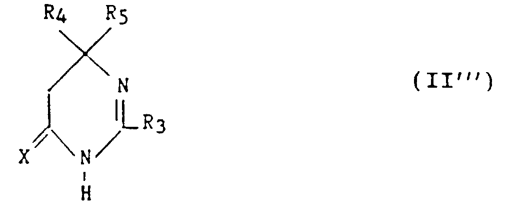 in which:
- R₃ is a hydrogen, a C₁-C₆ alkyl which is unsubstituted or substituted by one or more halogen atoms, a C₂-C₆ alkenyl, a C₃-C₇ cycloalkyl, a phenyl, a phenylalkyl in which the alkyl is C₁-C₃, a phenylalkenyl in which the alkenyl is C₂-C₃, said phenyl groups being unsubstituted or monosubstituted or polysubstituted by a halogen atom, a C₁-C₄ alkyl, a C₁-C₄ halogenoalkyl, a C₁-C₄ polyhalogenoalkyl, a hydroxyl or a C₁-C₄ alkoxy;
- R₄ and R₅ are each independently a C₁-C₆ alkyl, a phenyl or a phenylalkyl in which the alkyl is C₁-C₃, said alkyl, phenyl and phenylalkyl groups being unsubstituted or substituted by one or more halogen atoms or by a group selected from a C₁-C₄ perfluoroalkyl, a hydroxyl, a C₁-C₄ alkoxy;
- or R₄ and R₅ together form a group of the formula =CR₇R₈, in which R₇ is hydrogen, a C₁-C₄ alkyl or a phenyl and R₈ is a C₁-C₄ alkyl or a phenyl;
- or R₄ and R₅ together are either a group of the formula (CH₂)ₙ or a group of the formula (CH₂)ₚY-(CH₂)_{q}, in which Y is either an oxygen atom, or a sulfur atom, or a carbon atom substituted by a C₁-C₄ alkyl group, a phenyl or a phenylalkyl in which the alkyl is C₁-C₃, or a group N-R₆, in which R₆ is a hydrogen, a C₁-C₄ alkyl, a phenylalkyl in which the alkyl is C₁-C₄, a C₁-C₄ alkylcarbonyl, a C₁-C₄ halogenoalkylcarbonyl, a C₁-C₄ polyhalogenoalkylcarbonyl, a benzoyl, an alpha-aminoacyl or an N-protecting group, or R₄ and R₅, bonded together with the carbon atom to which they are bonded, form an indane or an adamantane;
- p + q = m;
- n is an integer between 2 and 11;
- m is an integer between 2 and 5; and
- X is an oxygen atom or a sulfur atom;
with the limitation that R₃ is other than a phenyl if R₄ and R₅ are each a methyl.

17. A method of preparing a compound according to any one of claims 11 to 16, characterized in that a compound of the formula
R₃-B 14
in which R₃ has the definition indicated in claims 11 to 16 and B is
- a group C(OR)₃
- a group 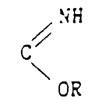 or
- a group COHal
R being a C₁-C₄ alkyl and Hal denoting a halogen atom, preferably chlorine, is reacted with a compound of the formula 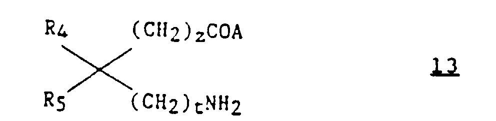 in which R₄ and R₅ are as defined above for (II) in claim 11, A is an OH group, an NH₂ group or a group OR', R' being hydrogen or a C₁-C₄ alkyl,
and then if appropriate, the thus obtained compound is treated with Lawesson's reagent (2,4-bis(4-methoxyphenyl)-1,3-dithia-2,4-diphosphetane disulfide).

18. A pharmaceutical composition containing a compound according to any one of claims 1 to 7 as the active principle.

19. A pharmaceutical composition containing a compound according to any one of claims 1 to 7 in association with a beta-blocking compound.

20. A pharmaceutical composition containing a compound according to any one of claims 1 to 7 in association with a diuretic.

21. A pharmaceutical composition containing a compound according to any one of claims 1 to 7 in association with a non-steroidal anti-inflammatory.

22. A pharmaceutical composition containing a compound according to any one of claims 1 to 7 in association with a calcium antagonist.

23. A pharmaceutical composition containing a compound according to any one of claims 1 to 7 in association with a tranquilizer.

## Claims (Claims for the following Contracting State(s): ES)

1. A process for obtaining a compound of the formula 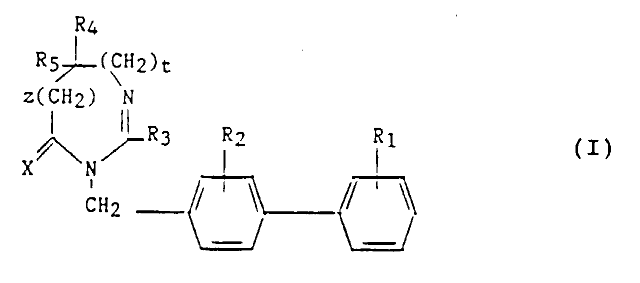 in which:
- R₁ and R₂ are similar or different and are each independently hydrogen or a group selected from a C₁-C₆ alkyl, a C₁-C₄ alkoxy, an amino, an aminomethyl, a carboxyl, an alkoxycarbonyl in which the alkoxy is C₁-C₄, a cyano, a tetrazolyl, a methyltetrazolyl, a methylsulfonylamino, a trifluoromethylsulfonylanino, a trifluoromethylsulfonylaminomethyl, an N-cyanocarbamoyl, an N-hydroxycarbamoyl, an N-(4-carboxy-1,3-thiazol-2-yl)carbamoyl, a ureido, a 2-cyanoguanidinocarbonyl, a 2-cyanoguanidinomethyl, an imidazol-1-ylcarbonyl and a 3-cyano-2-methylisothioureidomethyl, with the proviso that at least one of the substituents R₁ or R₂ is other than hydrogen;
- R₃ is a hydrogen, a C₁-C₆ alkyl which is unsubstituted or substituted by one or more halogen atoms, a C₂-C₆ alkenyl, a C₃-C₇ cycloalkyl, a phenyl, a phenylalkyl in which the alkyl is C₁-C₃, or a phenylalkenyl in which the alkenyl is C₂-C₃, said phenyl groups being unsubstituted or monosubstituted or polysubstituted by a halogen atom, a C₁-C₄ alkyl, a C₁-C₄ halogenoalkyl, a C₁-C₄ polyhalogenoalkyl, a hydroxyl or a C₁-C₄ alkoxy;
- R₄ and R₅ are each independently a C₁-C₆ alkyl, a phenyl or a phenylalkyl in which the alkyl is C₁-C₃, said alkyl, phenyl and phenylalkyl groups being unsubstituted or substituted by one or more halogen atoms or by a group selected from a C₁-C₄ perfluoroalkyl, a hydroxyl and a C₁-C₄ alkoxy;
- or R₄ and R₅ together form a group of the formula =CR₇R₈, in which R₇ is hydrogen, a C₁-C₄ alkyl or a phenyl and R₈ is a C₁-C₄ alkyl or a phenyl;
- or else R₄ and R₅ bonded together are either a group of the formula (CH₂)ₙ or a group of the formula (CH₂)ₚY-(CH₂)_{q}, in which Y is either an oxygen atom, or a sulfur atom, or a carbon atom substituted by a C₁-C₄ alkyl group, a phenyl or a phenylalkyl in which the alkyl is C₁-C₃, or a group N-R₆, in which R₆ is a hydrogen, a C₁-C₄ alkyl, a phenylalkyl in which the alkyl is C₁-C₃, a C₁-C₄ alkylcarbonyl, a C₁-C₄ halogenoalkylcarbonyl, a C₁-C₄ polyhalogenoalkylcarbonyl, a benzoyl, an alpha-aminoacyl or an N-protecting group, or R₄ and R₅, bonded together with the carbon atom to which they are bonded, form an indane or an adamantane;
- p + q = m;
- n is an integer between 2 and 11;
- m is an integer between 2 and 5;
- X is an oxygen atom or sulfur atom;
- z and t are zero or one is zero and the other is one; and its salts, characterized in that:
a1) a heterocyclic derivative of the formula 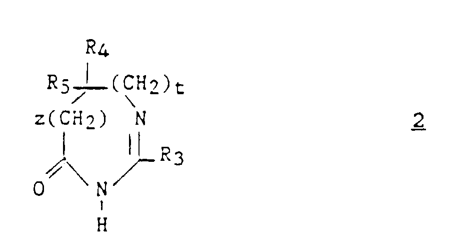 in which z, t, R₃, R₄ and R₅ have the meanings indicated above for (I), is reacted with a (biphenyl-4-yl)methyl derivative of the formula 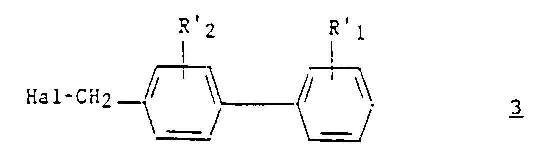 in which Hal is a halogen atom and R'₁ and R'₂ are respectively either R₁ and R₂ or a precursor group of R₁ and R₂;
b1) if appropriate, the thus obtained compound of the formula 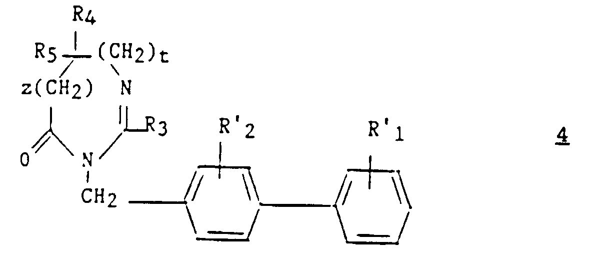 is treated with Lawesson's reagent [2,4-bis(4-methoxyphenyl)-1,3-dithia-2,4-diphosphetane 2,4-disulfide]; and
c1) the compound obtained in a1) or b1), of the formula 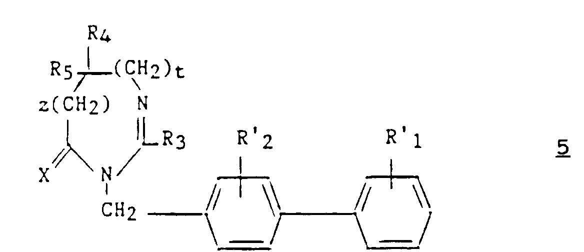 in which X is an oxygen atom or a sulfur atom, is treated to prepare the compound (I) by conversion of the groups R'₁ and/or R'₂ to the groups R₁ and/or R₂ respectively, which is then possibly converted into one of its salts.

2. A process for obtaining compounds of formula (I) : 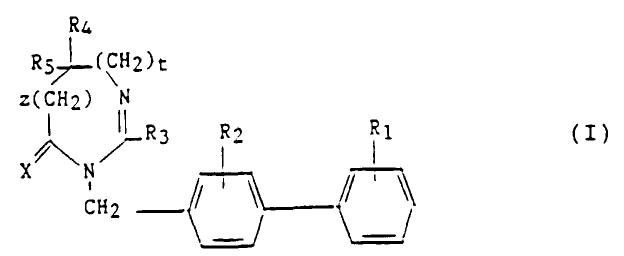 in which R₁, R₂, R₃, R₄, R₅, X, z and t are as defined in claim 1, characterized in that:
a2) an amino acid of the formula 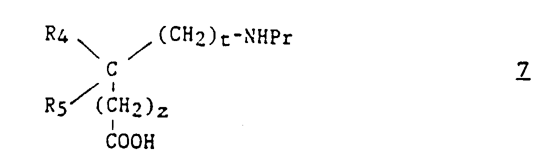 in which z, t, R₄ and R₅ have the meanings indicated for (I) in claim 1, and of which the amine group is protected by the Pr group, is reacted with a (biphenyl-4-yl)methyl-amine derivative of the formula 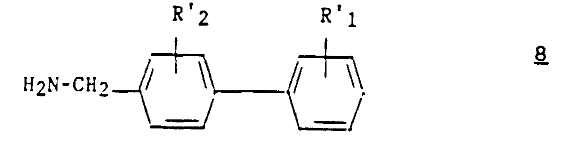 in which R'₁ and R'₂ are respectively either R₁ and R₂ or a precursor group of R₁ and R₂;
b2) after deprotection of the amine, the thus obtained compound of the formula 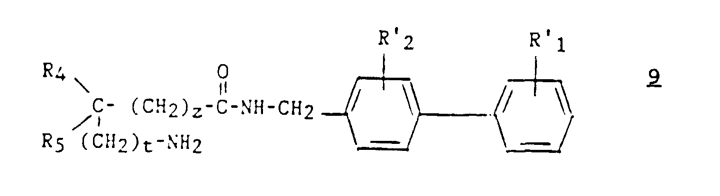 is then treated with an alkyl ortho-ester of the formula R₃C(OR)₃ (10), in which R₃ has the meaning indicated for (I) in claim 1 and R is a C₁-C₄ alkyl;
c2) if appropriate, the thus obtained compound of the formula 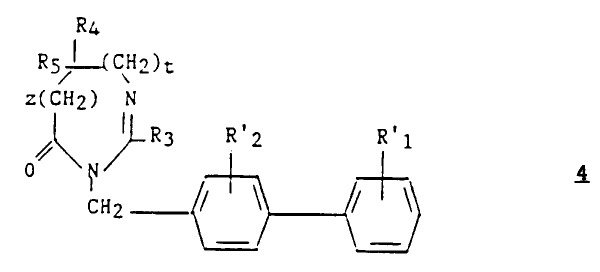 is treated with Lawesson's reagent [2,4-bis(4-methoxyphenyl)-1,3-dithia-2,4-diphosphetane 2,4-disulfide]; and
d2) the compound thus obtained in b2 or c2, of the formula 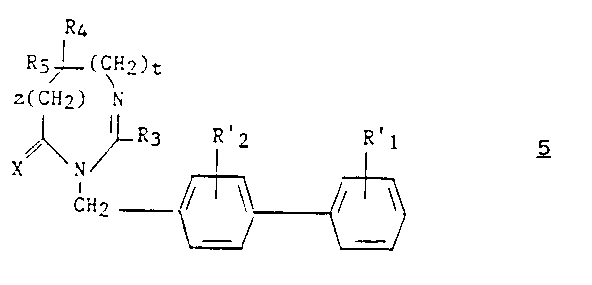 is then treated under suitable conditions for preparing the compound (I) by conversion of the groups R'₂ and/or R'₁ to the groups R₂ and/or R₁ respectively which is then possibly converted into one of its salts.

3. A process for obtaining compounds of formula (I): 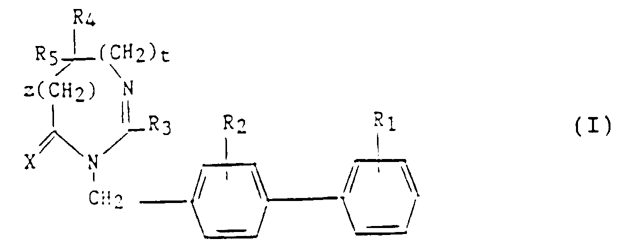 in which R₁,R₂, R₃, R₄, R₅ and X are as defined in claim 1, z and t are equal to zero, charactezized in that :
a3) a (biphenyl-4-yl)methyl derivative of the formula 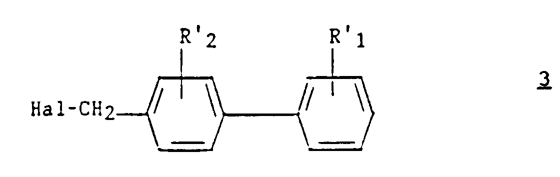 in which Hal is a halogen atom and R'₁ and R'₂ are respectively either R₁ and R₂ or a precursor group of R₁ and R₂, is reacted with an imidazole derivative of the formula 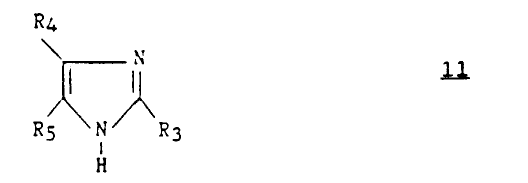 in which R₃, R₄ and R₅ are as defined for (I) in claim 1, in the presence of oxygen and UV irradiation and in a basic medium;
b3) if appropriate, the thus obtained compound of the formula 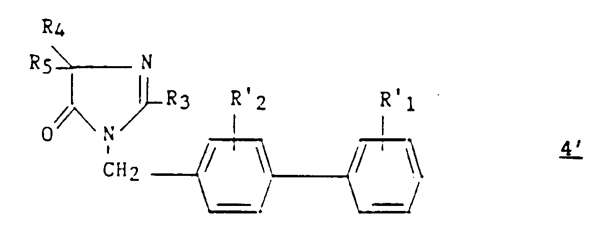 is treated with Lawesson's reagent [2,4-bis(4-methoxyphenyl)-1,3-dithia-2,4-diphosphetane 2,4-disulfide]; and
c3) the compound thus obtained in b3 or c3, of the formula 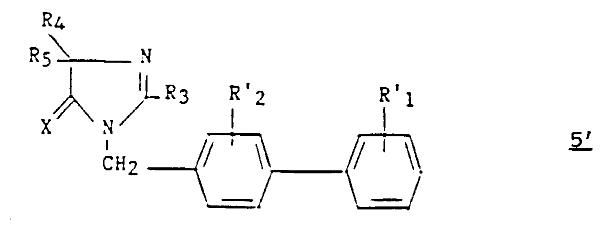 is then treated under suitable conditions for preparing the compound (I) by conversion of the groups R'₁ and/or R'₂ to the groups R₁ and/or R₂ respectively which is then possibly converted into one of its salts.

4. A process according to any one of claims 1 to 3, characterized in that in the compound of formula 3 or 8, R'₂ is hydrogen and R'₁ is an esterified carboxy group which is converted in steps c1) d2) or c3) into a carboxy group.

5. A process according to any one of claims 1 to 3, characterized in that in the compound of formula 3 or 8, R'₂ is hydrogen and R'₁ is either a protected tetrazolyl group or a cyano group, which are converted in steps c1),d2) or c3) into a tetrazolyl group.

6. A process according to any one of claims 1 to 5, characterized in that in the compounds of formulae 2, 7 or 11, R₄ and R₅ form, with the carbon atom to which they are bonded, a cyclopentane or a cyclohexane.

7. A process according to any one of claims 1 to 6, characterized in that in the compounds of formulae 2, 10 and 11, R₃ is a linear C₁-C₆ alkyl group.

8. A process according to any one of claims 1 to 7, characterized in that it comprises steps a1) and c1); a2), b2) and d2) or a3), b3) respectively.

9. A process acccrding to one of claims 1 or 2, characterized in that in the compounds of formulae 2 and 7, z and t are zero.

10. Process according to claim 1, characterized in that
1) a compound of formula 2 in which R₃ is the n-butyl group; R₄ and R₅ together form with the carbon atom to which they are bonded the spirocyclopentane group and z and t are zero;
2) a compound of formula 3 in which R'₂ is hydrogen and R'₁ is a protected tetrazolyl group are used to obtain the 2-n-butyl-4-spirocyclopentane-1-[(2'-(tetrazol-5-yl)biphenyl-4-yl)methyl]-2-imidazolin-5-one or one of its salts with acids or bases.

11. A process for obtaining a compound of the formula 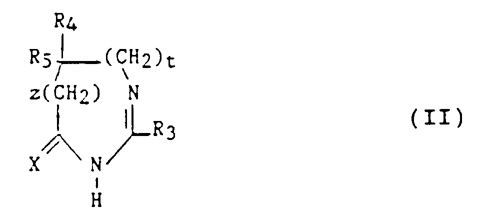 in which:
- R₃ is a hydrogen, a C₁-C₆ alkyl which is unsubstituted or substituted by one or more halogen atoms, a C₂-C₆ alkenyl, a C₃-C₇ cycloalkyl, a phenyl, a phenylalkyl in which the alkyl is C₁-C₃, or a phenylalkenyl in which the alkenyl is C₂-C₃, said phenyl groups being unsubstituted or monosubstituted or polysubstituted by a halogen atom, a C₁-C₄ alkyl, a C₁-C₄ halogenoalkyl, a C₁-C₄ polyhalogenoalkyl, a hydroxyl or a C₁-C₄ alkoxy;
- R₄ et R₅ are each independently a C₁-C₆ alkyl, a phenyl, a phenylalkyl in which the alkyl is C₁-C₃, said alkyl, phenyl and phenylalkyl groups being unsubstituted or substituted by one or more halogen atoms or by a group selected from a C₁-C₄ perfluoroalkyl, a hydroxyl and a C₁-C₄ alkoxy;
- or R₄ and R₅ together form a group of the formula =CR₇R₈, in which R₇ is hydrogen, a C₁-C₄ alkyl or a phenyl and R₈ is a C₁-C₄ alkyl or a phenyl;
- or else R₄ and R₅ bonded together are either a group of the formula (CH₂)ₙ or a group of the formula (CH₂)ₚY-(CH₂)_{q}, in which Y is either an oxygen atom, or a sulfur atom, or a carbon atom substituted by a C₁-C₄ alkyl group, a phenyl or a phenylalkyl in which the alkyl is C₁-C₃, or a group N-R₆, in which R₆ is a hydrogen, a C₁-C₄ alkyl, a phenylalkyl in which the alkyl is C₁-C₃, a C₁-C₄ alkylcarbonyl, a C₁-C₄ halogenoalkylcarbonyl, a C₁-C₄ polyhalogenoalkylcarbonyl, a benzoyl, an alpha-aminoacyl or an N-protecting group, or R₄ and R₅, bonded together with the carbon atom to which they are bonded, form an indane or an adamantane;
- p + q = m;
- n is an integer between 2 and 11;
- m is an integer between 2 and 5;
- X is an oxygen atom or sulfur atom;
- z and t are zero or one is zero and the other is one; with the limitation that
A/ if z and t are zero and X is oxygen:
1) R₄ and R₅ are other than a C₁-C₆ alkyl, a phenyl, a phenylalkyl in which the alkyl is C₁-C₃, said alkyl, phenyl and phenylalkyl groups being unsubstituted or substituted by one or more halogen atoms or by a group selected from a C₁-C₄ perfluoroalkyl, a hydroxyl, a C₁-C₄ alkoxy; or
2) R₄ and R₅ bonded together are other than a group of formula (CH₂)ₚY(CH₂)_{q} in which Y is a group N-R₆ in which R₆ is a hydrogen, a C₁-C₄ alkyl or a phenylalkyl in which the alkyl is C₁-C₃ ; or
3) R₄ and R₅ together are different from the group =CR₇R₈ when R₃ is a hydrogen, a phenyl, a methylphenyl or a benzyl;
4) n is different from 6; or else
5) R₄ and R₅ bonded together are other than a group (CH₂)ₙ in which n is comprised between 3 and 5, when R₃ is a substituted phenyl group;
B/ when z = 1 and R₃ is a phenyl, R₄ and R₅ are each other than a methyl;
C/ when z and t are zero and X is sulphur:
1) R₄ and R₅ are other than a phenyl when R₃ is a methyl, a phenyl, a chlorophenyl, a methoxyphenyl or a methylphenyl; or
2) R₄ and R₅ are other than a C₁-C₃ alkyl when R₃ is a methyl or a phenyl; or
3) R₄ and R₅ are other than a methoxyphenyl or chlorophenyl when R₃ is phenyl; or
4) R₄ and R₅ are other than a chlorophenyl when R₃ is a methyl,
characterized in that a compound of the formula
R₃-B 14
in which R₃ is a defined above for (II) and B is
- a group C(OR)₃
- a group 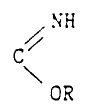 or
- a group COHal
R being a C₁-C₄ alkyl and Hal denoting a halogen atom, preferably chlorine, is reacted with a compound of the formula 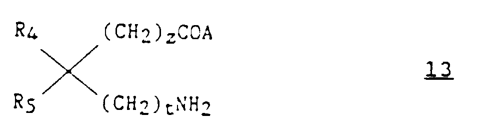 in which R₄ and R₅ are as defined above for (II), A is and OH group, an NH₂ group or a group OR', R' being hydrogen or a C₁-C₄ alkyl, and then, if appropriate, the thus obtained compound is treated with Lawesson's reagent (2,4-bis (4-methoxyphenyl)-1,3-dithia-2,4-diphosphetane disulfide).

12. A process according to claim 11 for obtaining a compound of formula 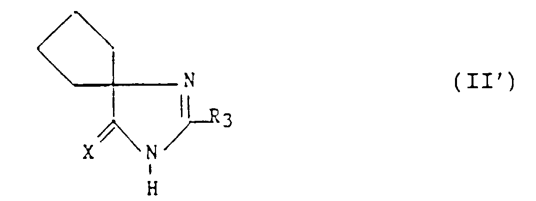 in which X is an oxygen atom or a sulfur atom and R₃ is a hydrogen, a C₁-C₆ alkyl which is unsubstituted or substituted by one or more halogen atoms, a C₂-C₆ alkenyl, a C₃-C₇ cycloalkyl, a phenyl, a phenylalkyl in which the alkyl is C₁-C₃, or a phenylalkenyl in which the alkenyl is C₂-C₃, said phenyl groups being unsubstituted or monosubstituted or polysubstituted by a halogen atom, a C₁-C₄ alkyl, a C₁-C₄ halogenoalkyl, a C₁-C₄ polyhalogenoalkyl, a hydroxyl or a C₁-C₄ alkoxy; with the proviso that R₃ is other than a phenyl group that is substituted when X is oxygen,
characterized in that a compound of formula 13 in which z and t are zero, R₄ and R₅ together, with the carbon atom to which they are bonded, form a spirocyclopentane group and a compound of formula 14 in which R₃ is as defined above, are used.

13. A process according to claim 12, characterized in that a compound of formula 13 in which z and t are zero, R₄ and R₅ together, with the carbon atom to which they are bonded, form a spirocyclopentane group is reacted with a compound of formula 14 in which R₃ is the n-butyl group so as to form the 2-n-butyl-4-spirocyclopentane-2-imidazolin-5-one.

14. A process for obtaining the 2-n-Butyl-4-spirocyclopentane-2-imidazolin-5-one hydrochloride, characterized in that it consists in adding concentrated hydrochloric acid to the compound obtained according to the process of claim 13.

15. A process according to claim 11 for obtaining a compound of formula 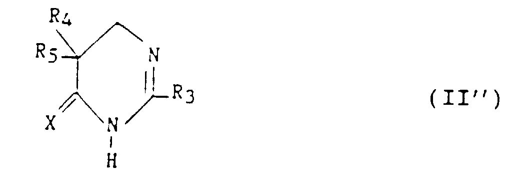 in which R₃, R₄, R₅ and X are as defined above for (II) in claim 11,
characterized in that a compound of formula 13 in which t is 1 and z is zero, R₄ and R₅ are as defined in claim 11 and a compound of formula 14 in which R₃ is as defined in claim 11, are used.

16. A process according to claim 11 for obtaining a compound of formula 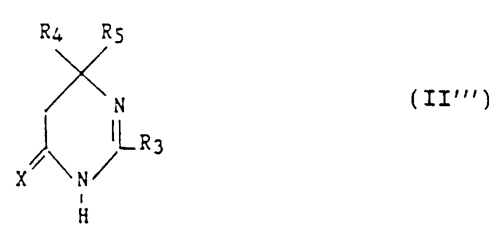 in which:
- R₃ is a hydrogen, a C₁-C₆ alkyl which is unsubstituted or substituted by one or more halogen atoms, a C₂-C₆ alkenyl, a C₃-C₇ cycloalkyl, a phenyl, a phenylalkyl in which the alkyl is C₁-C₃, or a phenylalkenyl in which the alkenyl is C₂-C₃, said phenyl groups being unsubstituted or monosubstituted or polysubstituted by a halogen atom, a C₁-C₄ alkyl, a C₁-C₄ halogenoalkyl, a C₁-C₄ polyhalogenoalkyl, a hydroxyl or a C₁-C₄ alkoxy;
- R₄ and R₅ are each independently a C₁-C₆ alkyl, a phenyl or a phenylalkyl in which the alkyl is C₁-C₃, said alkyl, phenyl and phenylalkyl groups being unsubstituted or substituted by one or more halogen atoms or by a group selected from a C₁-C₄ perfluoroalkyl, a hydroxyl, a C₁-C₄ alkoxy;
- or R₄ and R₅ together form a group of the =CR₇R₈, in which R₇ is hydrogen, a C₁-C₄ alkyl or a phenyl and R₈ is a C₁-C₄ alkyl or a phenyl;
- or R₄ and R₅ bonded together are either a group of the formula (CH₂)ₙ or a group of the formula (CH₂)ₚY-(CH₂)_{q}, in which Y is either an oxygen atom, or a sulfur atom, or a carbon atom substituted by a C₁-C₄ alkyl group, a phenyl or a phenylalkyl in which the alkyl is C₁-C₃, or a group N-R₆, in which R₆ is a hydrogen, a C₁-C₄ alkyl, a phenylalkyl in which the alkyl is C₁-C₄, a C₁-C₄ alkylcarbonyl, a C₁-C₄ halogenoalkylcarbonyl, a C₁-C₄ polyhalogenoalkylcarbonyl, a benzoyl, an alpha-aminoacyl or an N-protecting group, or R₄ and R₅, bonded together with the carbon atom to which they are bonded, form an indane or an adamantane;
- p + q = m;
- n is an integer between 2 and 11;
- m is an integer between 2 and 5; and
- X is an oxygen atom or a sulfur atom;
with the limitation that R₃ is other than a phenyl if R₄ and R₅ are each a methyl,
characterized in that a compound of formula 13 in which z = 1 and t is zero, R₄ and R₅ are as defined above and a compound of formula 13 in which R₃ is as defined above are used.

17. A process for preparing a pharmaceutical composition, characterized in that it consists in mixing a compound obtained by the process according to any one of claims 1 to 10 with a pharmaceutically acceptable vehicle.

18. A process for preparing a pharmaceutical composition, characterized in that it consists in associating a compound obtained by the process according to any one of claims 1 to 10 with a beta-blocking compound.

19. A process for preparing a pharmaceutical composition, characterized in that it consists in associating a compound obtained by the process according to any one of claims 1 to 10 with a diuretic.

20. A process for preparing a pharmaceutical composition, characterized in that it consists in associating a compound obtained by the process according to any one of claims 1 to 10 with a non-steroidal antiinflammatory.

21. A process for preparing a pharmaceutical composition, characterized in that it consists in associating a compound obtained by the process according to any one of claims 1 to 10 with a calcium antagonist.

22. A process for preparing a pharmaceutical composition, characterized in that it consists in associating a compound obtained by the process according to any one of claims 1 to 10 with a tranquilizer.

## Claims (Claims for the following Contracting State(s): GR)

1. A compound of the formula 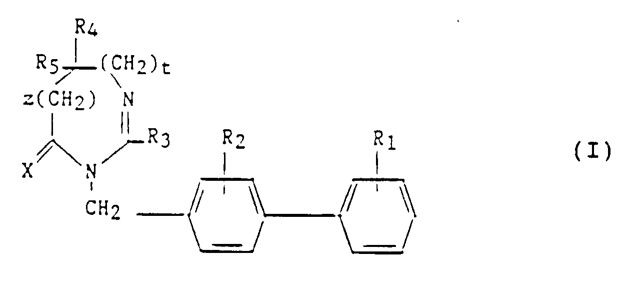 in which:
- R₁ and R₂ are similar or different and are each independently hydrogen or a group selected from a C₁-C₆ alkyl, a C₁-C₄ alkoxy, an amino, an aminomethyl, a carboxyl, an alkoxycarbonyl in which the alkoxy is C₁-C₄, a cyano, a tetrazolyl, a methyltetrazolyl, a methylsulfonylamino, a trifluoromethylsulfonylanino, a trifluoromethylsulfonylaminomethyl, an N-cyanocarbamoyl, an N-hydroxycarbamoyl, an N-(4-carboxy-1,3-thiazol-2-yl)carbamoyl, a ureido, a 2-cyanoguanidinocarbonyl, a 2-cyanoguanidinomethyl, an imidazol-1-ylcarbonyl, a 3-cyano-2-methylisothioureidomethyl, with the proviso that at least one of the substituents R₁ or R₂ is different from hydrogen;
- R₃ is a hydrogen, a C₁-C₆ alkyl which is unsubstituted or substituted by one or more halogen atoms, a C₂-C₆ alkenyl, a C₃-C₇ cycloalkyl, a phenyl, a phenylalkyl in which the alkyl is C₁-C₃, a phenylalkenyl in which the alkenyl is C₂-C₃, said phenyl groups being unsubstituted or monosubstituted or polysubstituted by a halogen atom, a C₁-C₄ alkyl, a C₁-C₄ halogenoalkyl, a C₁-C₄ polyhalogenoalkyl, a hydroxyl or a C₁-C₄ alkoxy;
- R₄ and R₅ are each independently a C₁-C₆ alkyl, a phenyl, a phenylalkyl in which the alkyl is C₁-C₃, said alkyl, phenyl and phenylalkyl groups being unsubstituted or substituted by one or more halogen atoms or by a group selected from a C₁-C₄ perfluoroalkyl, a hydroxyl, a C₁-C₄ alkoxy;
- or R₄ and R₅ together form a group of the formula =CR₇R₈, in which R₇ is hydrogen, a C₁-C₄ alkyl or a phenyl and R₈ is a C₁-C₄ alkyl or a phenyl;
- or else R₄ and R₅ bonded together are either a group of the formula (CH₂)ₙ or a group of the formula (CH₂)ₚY-(CH₂)_{q}, in which Y is either an oxygen atom, or a sulfur atom, or a carbon atom substituted by a C₁-C₄ alkyl group, a phenyl or a phenylalkyl in which the alkyl is C₁-C₃, or a group N-R₆, in which R₆ is a hydrogen, a C₁-C₄ alkyl, a phenylalkyl in which the alkyl is C₁-C₃, a C₁-C₄ alkylcarbonyl, a C₁-C₄ halogenoalkylcarbonyl, a C₁-C₄ polyhalogenoalkylcarbonyl, a benzoyl, an alpha-aminoacyl or an N-protecting group, or R₄ and R₅, bonded together with the carbon atom to which they are bonded, form an indane or an adamantane;
- p + q = m;
- n is an integer between 2 and 11;
- m is an integer between 2 and 5;
- X is an oxygen atom or sulfur atom;
- z and t are zero or one is zero and the other is one; and its salts.

2. A compound according to claim 1, characterized in that R₁ is in the ortho position and is a carboxyl or tetrazolyl group and R₂ is hydrogen.

3. A compound according to any one of claims 1 and 2,characterized in that R₄ and R₅ form a cyclopentane or cyclohexane with the carbon to which they are bonded.

4. A compound according to any one of claims 1 to 3, characterized in that R₃ is a linear C₁-C₆ alkyl group.

5. A compound according to any one of claims 1 to 4, characterized in that X is oxygen.

6. A compound according to any one of claims 1 to 5, characterized in that z = t = 0.

7. A compound according to claim 1, characterized in that it is the 2-n-butyl-4-spirocyclopentane-1-( ( (2'-(tetrazol-5-yl)biphenyl-4-yl)methyl)-2-imidazolin-5-one or one of its salts with acids or bases.

8. A method of preparing a compound (I) according to any one of claims 1 to 7, characterized in that:
a1) a heterocyclic derivative of the formula 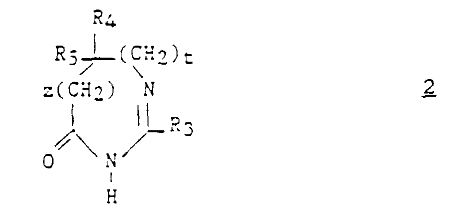 in which z, t, R₃, R₄ and R₅ have the meanings indicated for (I) in claim 1, is reacted with a (biphenyl-4-yl)methyl derivative of the formula 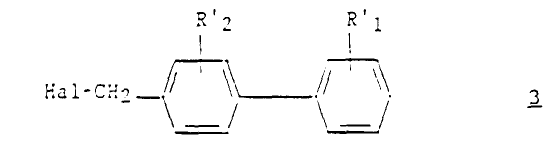 in which Hal is a halogen atom and R'₁ and R'₂ are respectively either R₁ and R₂ or a precursor group of R₁ and R₂;
b1) if appropriate, the thus obtained compound of the formula 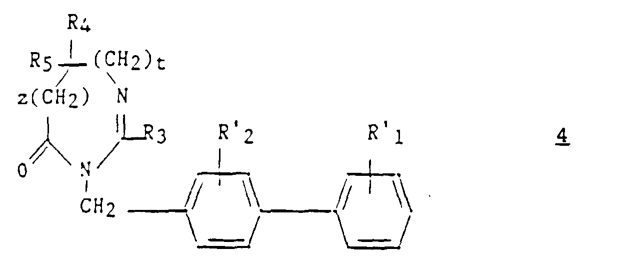 is treated with Lawesson's reagent [2,4-bis(4-methoxyphenyl)-1,3-dithia-2,4-diphosphetane 2,4-disulfide];
c1) the compound obtained in a1) or b1), of the formula 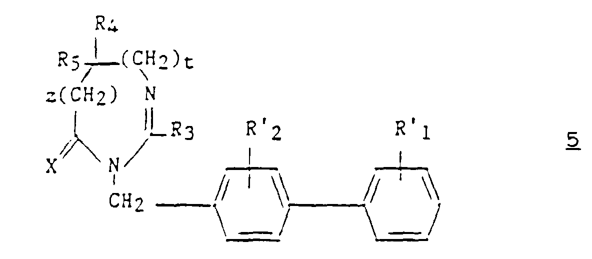 in which X is an oxygen atom or a sulfur atom, is treated to prepare the compound (I) by conversion of the groups R'₁ and/or R'₂ to the groups R₁ and/or R₂ respectively, which is then possibly converted into one of its salts.

9. A method of preparing a compound (I) according to any one of claims 1 to 7, characterized in that
a2) an amino acid of the formula 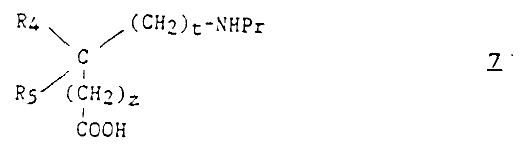 in which z, t, R₄ and R₅ have the meanings indicated for (I) in claim 1, and of which the amine group is protected by the Pr group, is reacted with a (biphenyl-4-yl)methyl-amine derivative of the formula 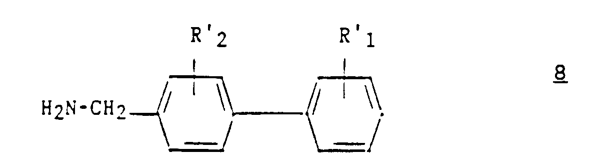 in which R'₁ and R'₂ are respectively either R₁ and R₂ or a precursor group of R₁ and R₂;
b2) after deprotection of the amine, the thus obtained compound of the formula 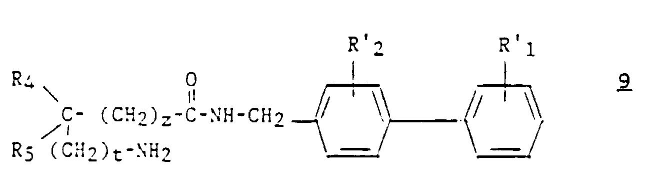 is then treated with an alkyl ortho-ester of the formula R₃C(OR)₃ (10), in which R₃ has the meaning indicated for (I) in claim 1 and R is a C₁-C₄ alkyl;
c2) if appropriate, the thus obtained compound of the formula 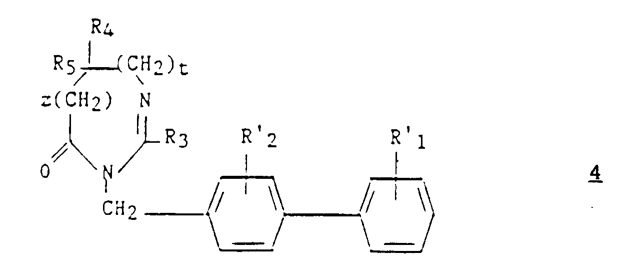 is treated with Lawesson's reagent [2,4-bis(4-methoxyphenyl)-1,3-dithia-2,4-diphosphetane 2,4-disulfide]; and
d2) the compound thus obtained in b2 or c2, of the formula 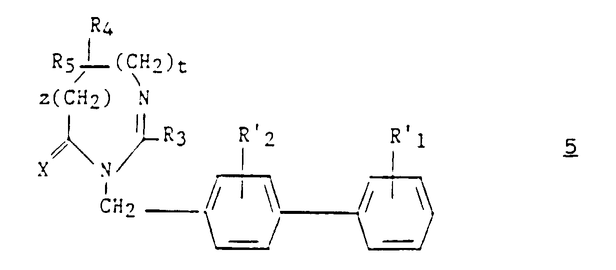 is then treated under suitable conditions for preparing the compound (I) by conversion of the groups R'₂ and/or R'₁ to the groups R₂ and/or R₁ respectively which is then possibly converted into one of its salts.

10. A method of preparing a compound (I) according to claim 6, characterized in that
a3) a (biphenyl-4-yl)methyl derivative of the formula 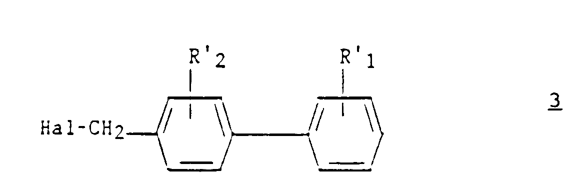 in which Hal is a halogen atom and R'₁ and R'₂ are respectively either R₁ and R₂ or a precursor group of R₁ and R₂, is reacted with an imidazole derivative of the formula 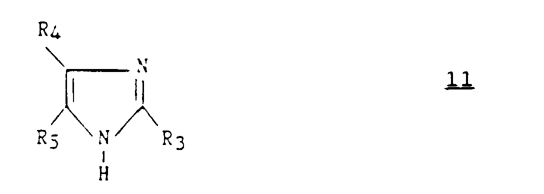 in which R₃, R₄ and R₅ are as defined for (I) in claim 1, in the presence of oxygen and UV irradiation and in a basic medium;
b3) if appropriate, the thus obtained compound of the formula 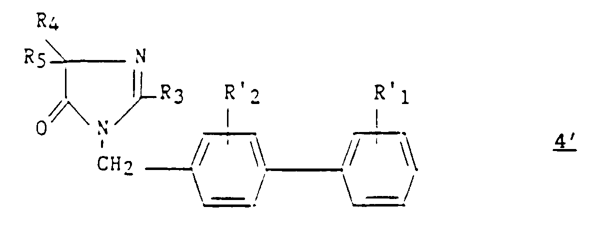 is treated with Lawesson's reagent [2,4-bis(4-methoxyphenyl)-1,3-dithia-2,4-diphosphetane 2,4-disulfide]; and
c3) the compound thus obtained in b3 or c3, of the formula 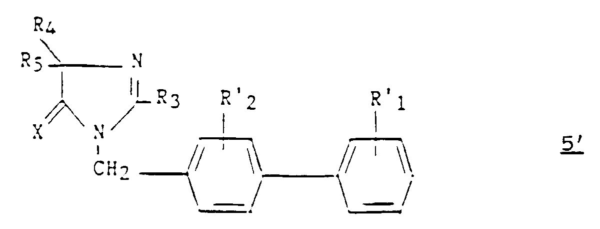 is then treated under suitable conditions for preparing the compound (I) by conversion of the groups R'₁ and/or R'₂ to the groups R₁ and/or R₂ respectively.

11. A compound of the formula 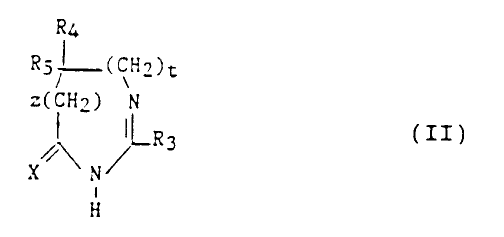 in which:
- R₃ is a hydrogen, a C₁-C₆ alkyl which is unsubstituted or substituted by one or more halogen atoms, a C₂-C₆ alkenyl, a C₃-C₇ cycloalkyl, a phenyl, a phenylalkyl in which the alkyl is C₁-C₃, a phenylalkenyl in which the alkenyl is C₂-C₃, said phenyl groups being unsubstituted or monosubstituted or polysubstituted by a halogen atom, a C₁-C₄ alkyl, a C₁-C₄ halogenoalkyl, a C₁-C₄ polyhalogenoalkyl, a hydroxyl or a C₁-C₄ alkoxy; R₄ and R₅ are each independently a C₁-C₆ alkyl, a phenyl, a phenylalkyl in which the alkyl is C₁-C₃, said alkyl, phenyl and phenylalkyl groups being unsubstituted or substituted by one or more halogen atoms or by a group selected from a C₁-C₄ perfluoroalkyl, a hydroxyl, a C₁-C₄ alkoxy-;
- or R₄ and R₅ together form a group of the formula =CR₇R₈, in which R₇ is hydrogen, a C₁-C₄ alkyl or a phenyl and R₈ is a C₁-C₄ alkyl or a phenyl;
- or else R₄ and R₅ bonded together are either a group of the formula (CH₂)ₙ or a group of the formula (CH₂)ₚY-(CH₂)_{q}, in which Y is either an oxygen atom, or a sulfur atom, or a carbon atom substituted by a C₁-C₄ alkyl group, a phenyl or a phenylalkyl in which the alkyl is C₁-C₃, or a group N-R₆, in which R₆ is a hydrogen, a C₁-C₄ alkyl, a phenylalkyl in which the alkyl is C₁-C₃, a C₁-C₄ alkylcarbonyl, a C₁-C₄ halogenoalkylcarbonyl, a C₁-C₄ polyhalogenoalkylcarbonyl, a benzoyl, an alpha-aminoacyl or an N-protecting group, or R₄ and R₅, bonded together with the carbon atom to which they are bonded, form an indane or an adamantane;
- p + q = m;
- n is an integer between 2 and 11;
- m is an integer between 2 and 5;
- X is an oxygen atom or sulfur atom; and
- z and t are zero or one is zero and the other is one; with the limitation that
A/ if z and t are zero and X is oxygen:
1) R₄ and R₅ are other than a C₁-C₆ alkyl, a phenyl, a phenylalkyl in which the alkyl is C₁-C₃, said alkyl, phenyl and phenylalkyl groups being unsubstituted or substituted by one or more halogen atoms or by a group selected from a C₁-C₄ perfluoroalkyl, a hydroxyl, a C₁-C₄ alkoxy; or
2) R₄ and R₅ bonded together are other than a group of formula (CH₂)ₚY(CH₂)_{q} in which Y is a group N-R₆ in which R₆ is a hydrogen, a C₁-C₄ alkyl or a phenylalkyl in which the alkyl is C₁-C₃ ; or
3) R₄ and R₅ together are different from the group =CR₇R₈ when R₃ is a hydrogen, a phenyl, a methylphenyl or a benzyl;
4) n is different from 6; or else
5) R₄ and R₅ bonded together are other than a group (CH₂)ₙ in which n is comprised between 3 and 5, when R₃ is a substituted phenyl group;
B/ when z = 1 and R₃ is a phenyl, R₄ and R₅ are each other than a methyl;
C/ when z and t are zero and X is sulphur:
1) R₄ and R₅ are other than a phenyl when R₃ is a methyl, a phenyl, a chlorophenyl, a methoxyphenyl or a methylphenyl; or
2) R₄ and R₅ are other than a C₁-C₃ alkyl when R₃ is a methyl or a phenyl; or
3) R₄ and R₅ are other than a methoxyphenyl or chlorophenyl when R₃ is phenyl; or
4) R₄ and R₅ are other than a chlorophenyl when R₃ is a methyl.

12. A compound according to claim 11 of the formula 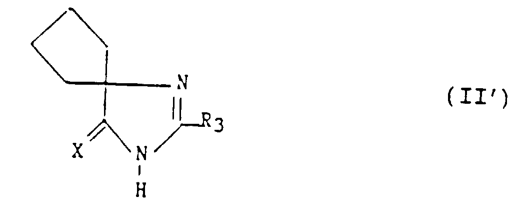 in which X is an oxygen atom or a sulfur atom and R₃ is a hydrogen, a C₁-C₆ alkyl which is unsubstituted or substituted by one or more halogen atoms; a C₂-C₆ alkenyl, a C₃-C₇ cycloalkyl, a phenyl, a phenylalkyl in which the alkyl is C₁-C₃, a phenylalkenyl in which the alkenyl is C₂-C₃, said phenyl groups being unsubstituted or monosubstituted or polysubstituted by a halogen atom, a C₁-C₄ alkyl, a C₁-C₄ halogenoalkyl, a C₁-C₄ polyhalogenoalkyl, a hydroxyl or a C₁-C₄ alkoxy. with the proviso that R₃ is other than a phenyl group that is substituted when X is oxygen.

13. A compound according to claim 12, characterized in that, in formula II', X is oxygen and R₃ is n-butyl and in that it is the 2-n-butyl-4-spirocyclopentane-2-imidazolin-5-one.

14. 2-n-Butyl-4-spirocyclopentane-2-imidazolin-5-one hydrochloride.

15. A compound according to claim 11 of the formula 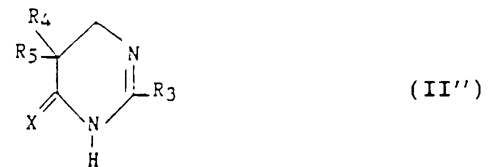 in which R₃, R₄, R₅ and X have the meanings given above for (II) in claim 11.

16. A compound according to claim 11 of the formula 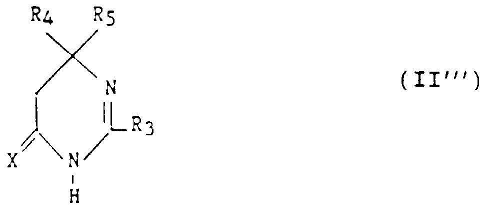 in which:
- R₃ is a hydrogen, a C₁-C₆ alkyl which is unsubstituted or substituted by one or more halogen atoms, a C₂-C₆ alkenyl, a C₃-C₇ cycloalkyl, a phenyl, a phenylalkyl in which the alkyl is C₁-C₃, or a phenylalkenyl in which the alkenyl is C₂-C₃, said phenyl groups being unsubstituted or monosubstituted or polysubstituted by a halogen atom, a C₁-C₄ alkyl, a C₁-C₄ halogenoalkyl, a C₁-C₄ polyhalogenoalkyl, a hydroxyl or a C₁-C₄ alkoxy;
- R₄ and R₅ are each independently a C₁-C₆ alkyl, a phenyl or a phenylalkyl in which the alkyl is C₁-C₃, said alkyl, phenyl and phenylalkyl groups being unsubstituted or substituted by one or more halogen atoms or by a group selected from a C₁-C₄ perfluoroalkyl, a hydroxyl and a C₁-C₄ alkoxy;
- or R₄ and R₅ together form a group of the formula =CR₇R₈, in which R₇ is hydrogen, a C₁-C₄ alkyl or a phenyl and R₈ is a C₁-C₄ alkyl or a phenyl;
- or R₄ and R₅ bonded together are either a group of the formula (CH₂)ₙ or a group of the formula (CH₂)ₚY-(CH₂)_{q}, in which Y is either an oxygen atom, or a sulfur atom, or a carbon atom substituted by a C₁-C₄ alkyl group, a phenyl or a phenylalkyl in which the alkyl is C₁-C₃, or a group N-R₆, in which R₆ is a hydrogen, a C₁-C₄ alkyl, a phenylalkyl in which the alkyl is C₁-C₄, a C₁-C₄ alkylcarbonyl, a C₁-C₄ halogenoalkylcarbonyl, a C₁-C₄ polyhalogenoalkylcarbonyl, a benzoyl, an alpha-aminoacyl or an N-protecting group, or R₄ and R₅, bonded together with the carbon atom to which they are bonded, form an indane or an adamantane;
- p + q = m;
- n is an integer between 2 and 11;
- m is an integer between 2 and 5;
- X is an oxygen atom or a sulfur atom;
with the limitation that R₃ is other than a phenyl if R₄ and R₅ are each a methyl.

17. A method of preparing a compound according to any one of claims 11 to 16, characterized in that a compound of the formula
R₃-B 14
in which R₃ has the definition indicated in claims 11 to 16 and B is
- a group C(OR)₃
- a group 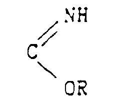 or
- a group COHal
R being a C₁-C₄ alkyl and Hal denoting a halogen atom, preferably chlorine,
is reacted with a compound of formula 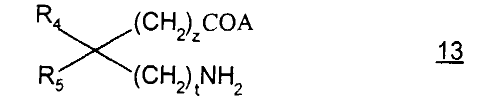 in which R₄, R₅ are as defined above for II in claim 11, A is an OH group, an NH₂ group or a group OR', R' being hydrogen or a C₁-C₄ alkyl,
and then, if appropriate, the thus obtained compound is treated with Lawesson's reagent (2,4-bis-(4-methoxyphenyl)-1,3-dithia-2,4-diphosphenate disulfide).

18. A process for preparing a pharmaceutical composition, characterized in that it consists in mixing a compound according to any one of claims 1 to 7 with a pharmaceutically acceptable vehicle.

19. A process for preparing a pharmaceutical composition, characterized in that it consists in associating a compound according to any one of claims 1 to 7 with a beta-blocking compound.

20. A process for preparing a pharmaceutical composition, characterized in that it consists in associating a compound according to any one of claims 1 to 7 with a diuretic.

21. A process for preparing a pharmaceutical composition, characterized in that it consists in associating a compound according to any one of claims 1 to 7 with a non-steroidal antiinflammatory.

22. A process for preparing a pharmaceutical composition, characterized in that it consists in associating a compound according to any one of claims 1 to 7 with a calcium antagonist.

23. A process for preparing a pharmaceutical composition, characterized in that it consists in associating a compound according to any one of claims 1 to 7 with a tranquilizer.

## Patentansprüche (Patentansprüche für folgende(n) Vertragsstaat(en): AT, BE, CH, DE, DK, FR, GB, IT, LI, LU, NL, SE)

1. Verbindung der Formel 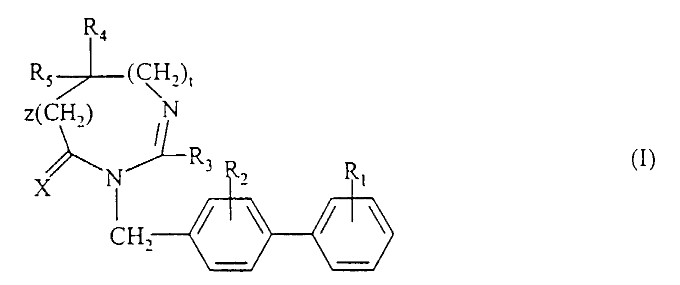 in der:
- R₁ und R₂ gleichartig oder verschieden sind und jeweils unabhängig voneinander Wasserstoff oder eine unter C₁-C₆-Alkyl, C₁-C₄-Alkoxy, Amino, Aminomethyl, Carboxy, Alkoxycarbonyl mit C₁-C₄-Alkoxy, Cyano, Tetrazolyl, Methyltetrazolyl, Methylsulfonylamino, Trifluormethylsulfonylamino, Trifluormethylsulfonylaminomethyl, N-Cyanocarbamoyl, N-Hydroxycarbamoyl, N-((4-Carboxy)-1,3-thiazol-2-yl)-carbamoyl, Ureido, 2-Cyanoguanidinocarbonyl, 2-Cyanoguanidinomethyl, Imidazol-1-ylcarbonyl und 3-Cyano-2-methylisothioureidomethyl ausgewählte Gruppe bedeutet, mit der Bedingung, daß mindestens einer der Substituenten R₁ oder R₂ eine andere Bedeutung als Wasserstoff hat;
- R₃ Wasserstoff, unsubstituiertes oder durch ein oder mehr Halogenatome substituiertes C₁-C₆-Alkyl, C₂-C₆-Alkenyl, C₃-C₇-Cycloalkyl, Phenyl, Phenylalkyl mit C₁-C₃-Alkyl oder Phenylalkenyl mit C₂-C₃-Alkenyl bedeutet, wobei die Phenylgruppen unsubstituiert oder ein- oder mehrfach durch ein Halogenatom, C₁-C₄-Alkyl, C₁-C₄-Halogenalkyl, C₁-C₄-Polyhalogenalkyl, Hydroxyl oder C₁-C₄-Alkoxy substituiert sind;
- R₄ und R₅ jeweils unabhängig voneinander C₁-C₆-Alkyl, Phenyl oder Phenylalkyl mit C₁-C₃-Alkyl bedeuten, wobei die Alkyl-, Phenyl- und Phenylalkylgruppen unsubstituiert oder durch ein oder mehr Halogenatome oder durch eine unter C₁-C₄-Perfluoralkyl, Hydroxyl und C₁-C₄-Alkoxy ausgewählte Gruppe substituiert sind;
- oder R₄ und R₅ zusammen eine Gruppe der Formel =CR₇R₈ bilden, in der R₇ Wasserstoff, C₁-C₄-Alkyl oder Phenyl bedeutet und R₈ C₁-C₄-Alkyl oder Phenyl bedeutet;
- oder R₄ und R₅ miteinander zu einer Gruppe der Formel (CH₂)ₙ oder einer Gruppe der Formel (CH₂)ₚY(CH₂)_{q} gebunden sind, in der Y ein Sauerstoffatom, ein Schwefelatom, ein Kohlenstoffatom, das durch eine C₁-C₄-Alkylgruppe, Phenyl oder Phenylalkyl mit C₁-C₃-Alkyl substituiert ist, oder eine N-R₆-Gruppe bedeutet, in der R₆ Wasserstoff, C₁-C₄-Alkyl, Phenylalkyl mit C₁-C₃-Alkyl, C₁-C₄-Alkylcarbonyl, C₁-C₄-Halogenalkylcarbonyl, C₁-C₄-Polyhalogenalkylcarbonyl, Benzoyl, α-Aminoacyl oder eine N-Schutzgruppe bedeutet, oder R₄ und R₅ zusammen mit dem Kohlenstoffatom, an das sie gebunden sind, einen Indan- oder Adamantanrest bilden;
- p + q = m;
- n eine ganze Zahl von 2 bis 11 bedeutet;
- m eine ganze Zahl von 2 bis 5 bedeutet;
- X ein Sauerstoff- oder Schwefelatom bedeutet; und
- z und t 0 bedeuten oder eines dieser Symbole 0 und das andere 1 bedeutet;
und Salze davon.

2. Verbindung nach Anspruch 1, dadurch gekennzeichnet, daß R₁ in ortho-Stellung vorliegt und eine Carboxy- oder Tetrazolylgruppe bedeutet und R₂ Wasserstoff bedeutet.

3. Verbindung nach einem der Ansprüche 1 und 2, dadurch gekennzeichnet, daß R₄ und R₅ zusammen mit dem Kohlenstoffatom, an das sie gebunden sind, einen Cyclopentan- oder Cyclohexanrest bilden.

4. Verbindung nach einem der Ansprüche 1 bis 3, dadurch gekennzeichnet, daß R₃ eine geradkettige C₁-C₆-Alkylgruppe bedeutet.

5. Verbindung nach einem der Ansprüche 1 bis 4, dadurch gekennzeichnet, daß X Sauerstoff bedeutet.

6. Verbindung nach einem der Ansprüche 1 bis 5, dadurch gekennzeichnet, daß z = t = 0.

7. Verbindung nach Anspruch 1, dadurch gekennzeichnet, daß es sich um 2-n-Butyl-4-spirocyclopentan-1-[(2'-(5-tetrazolyl)-biphenyl-4-yl)-methyl]-2-imidazolin-5-on oder ein Salz davon mit Säuren oder Basen handelt.

8. Verfahren zur Herstellung einer Verbindung (I) nach einem der Ansprüche 1 bis 7, dadurch gekennzeichnet, daß man
a1) ein heterocyclisches Derivat der folgenden Formel 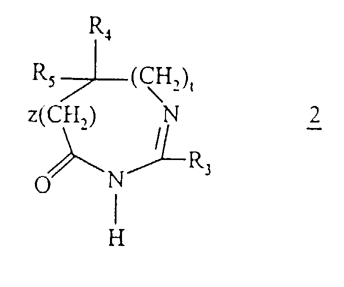 in der z, t, R₃, R₄ und R₅ die im Anspruch 1 für (I) angegebenen Bedeutungen haben, mit einem (Biphenyl-4-yl)-methylderivat der Formel 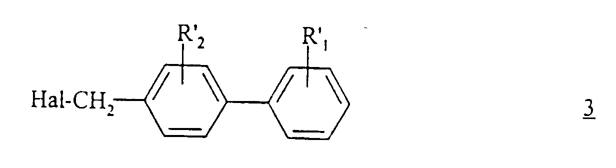 umsetzt, in der Hal ein Halogenatom bedeutet und R'₁ und R'₂ entweder die Bedeutung von R₁ bzw. R₂ haben oder eine Vorstufe für R₁ bzw. R₂ bedeuten;
b1) gegebenenfalls die erhaltene Verbindung der Formel 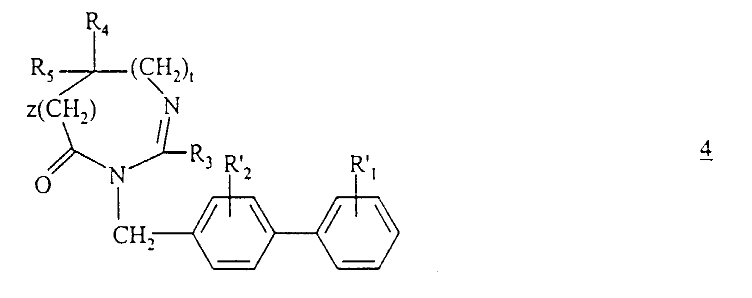 mit dem Lawesson-Reagenz [2,4-Bis-(4-methoxyphenyl)-1,3-dithia-2,4-diphosphetan-2,4-disulfid] behandelt;
c1) die in a1) oder b1) erhaltene Verbindung der Formel 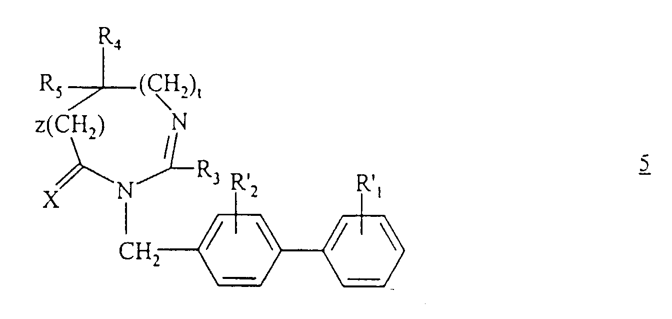 in der X ein Sauerstoff- oder Schwefelatom bedeutet, zur Herstellung der Verbindung (I) durch Umwandlung der Gruppen R'₁ und/oder R'₂ in die entsprechenden Gruppen R₁ und/oder R₂ behandelt, wobei die Verbindung gegebenenfalls anschließend in eines ihrer Salze übergeführt wird.

9. Verfahren zur Herstellung einer Verbindung (I) nach einem der Ansprüche 1 bis 7, dadurch gekennzeichnet, daß man
a2) eine Aminosäure der Formel 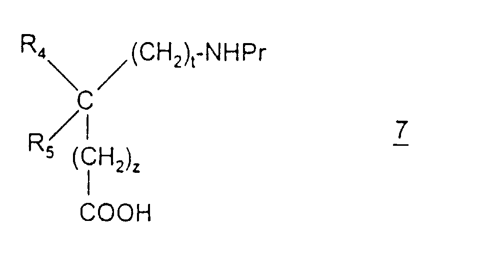 in der z, t, R₄ und R₅ die im Anspruch 1 für (I) angegebenen Bedeutungen haben und deren Aminfunktion durch die Gruppe Pr geschützt ist, mit einem (Biphenyl-4-yl)-methylaminderivat der Formel 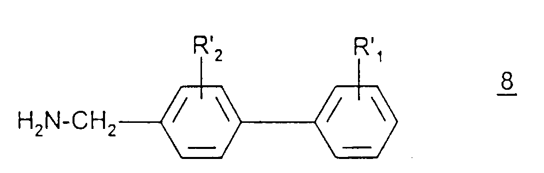 in der R'₁ und R'₂ entweder die Bedeutungen R₁ bzw. R₂ haben oder eine Vorstufe für R₁ bzw. R₂ bedeuten, umsetzt,
b2) nach Schutzgruppenentfernung am Amin die erhaltene Verbindung der Formel 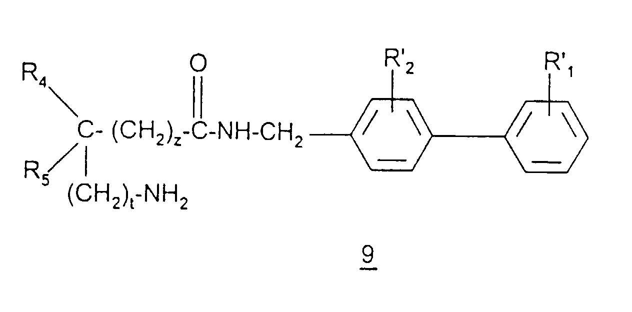 anschließend mit einem Alkylorthoester der Formel R₃C(OR)₃ (10), in der R₃ die im Anspruch 1 für (I) angegebene Bedeutung hat und R einen C₁-C₄-Alkylrest bedeutet, behandelt;
c2) gegebenenfalls die erhaltene Verbindung der Formel 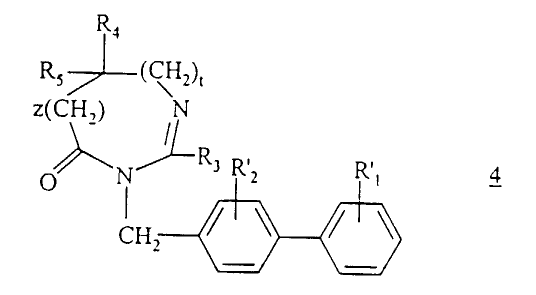 mit dem Lawesson-Reagenz [2,4-Bis-(4-methoxyphenyl)-1,3-dithia-2,4-diphosphetan-2,4-disulfid] behandelt;
d2) die in b2) oder c2) erhaltene Verbindung der Formel 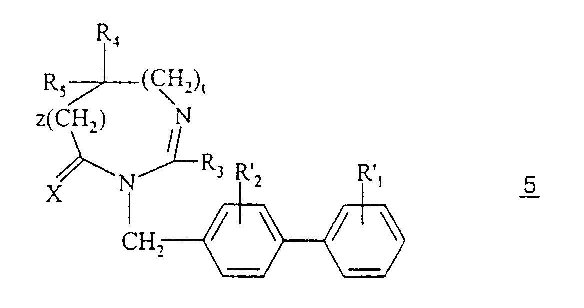 anschließend unter für die Herstellung der Verbindung (I) zweckmäßigen Bedingungen durch Umwandlung der Gruppen R'₂ und/oder R'₁ in die entsprechenden Gruppen R₂ und/oder R₁ behandelt, wobei die Verbindung anschließend gegebenenfalls in eines ihrer Salze übergeführt wird.

10. Verfahren zur Herstellung einer Verbindung (I) nach Anspruch 6, dadurch gekennzeichnet, daß man
a3) ein Imidazolderivat der Formel 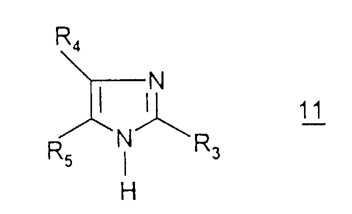 in der R₃, R₄ und R₅ die in Anspruch 1 für (I) angegebenen Bedeutungen haben, mit einem (Biphenyl-4-yl)-methylderivat der Formel 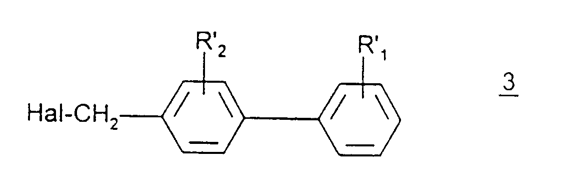 in der Hal ein Halogenatom bedeutet und R'₁ und R'₂ entweder die Bedeutung von R₁ bzw. R₂ haben oder eine Vorstufe für R₁ bzw. R₂ bedeuten, in Gegenwart von Sauerstoff und UV-Strahlung in basischem Milieu umsetzt;
b3) gegebenenfalls die erhaltene Verbindung der Formel 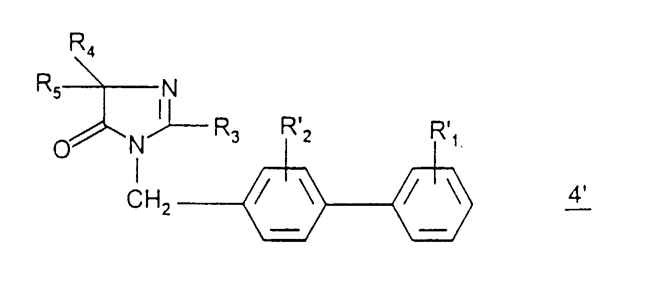 mit dem Lawesson-Reagenz [2,4-Bis- (4-methoxyphenyl)-1,3-dithia-2,4-diphosphetan-2,4-disulfid] behandelt;
c3) die in b3) oder c3) erhaltene Verbindung der Formel 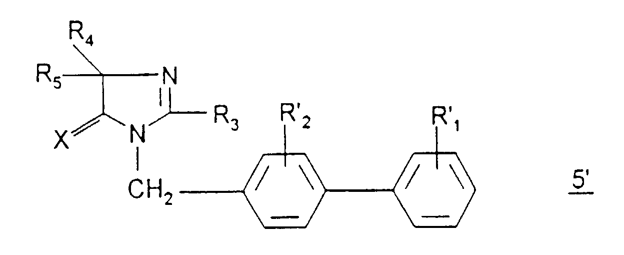 anschließend unter für die Herstellung der Verbindung (I) zweckmäßigen Bedingungen durch Umwandlung der Gruppen R'₁ und/oder R'₂ in die entsprechenden Gruppen R₁ und/oder R₂ behandelt, wobei die Verbindung anschließend gegebenenfalls in eines ihrer Salze übergeführt wird.

11. Verbindung der Formel 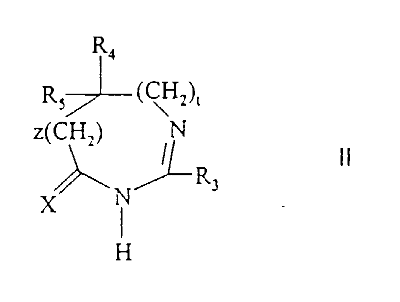 in der:
- R₃ Wasserstoff, unsubstituiertes oder durch ein oder mehr Halogenatome substituiertes C₁-C₆-Alkyl, C₂-C₆-Alkenyl, C₃-C₇-Cycloalkyl, Phenyl, Phenylalkyl mit C₁-C₃-Alkyl oder Phenylalkenyl mit C₂-C₃-Alkenyl bedeutet, wobei die Phenylgruppen unsubstituiert oder ein- oder mehrfach durch ein Halogenatom, C₁-C₄-Alkyl, C₁-C₄-Halogenalkyl, C₁-C₄-Polyhalogenalkyl, Hydroxyl oder C₁-C₄-Alkoxy substituiert sind;
- R₄ und R₅ jeweils unabhängig voneinander C₁-C₆-Alkyl, Phenyl oder Phenylalkyl mit C₁-C₃-Alkyl bedeuten, wobei die Alkyl-, Phenyl- und Phenylalkylgruppen unsubstituiert oder durch ein oder mehr Halogenatome oder durch eine unter C₁-C₄-Perfluoralkyl, Hydroxyl und C₁-C₄-Alkoxy ausgewählte Gruppe substituiert sind;
- oder R₄ und R₅ zusammen eine Gruppe der Formel =CR₇R₈ bilden, in der R₇ Wasserstoff, C₁-C₄-Alkyl oder Phenyl bedeutet und R₈ C₁-C₄-Alkyl oder Phenyl bedeutet;
- oder R₄ und R₅ miteinander zu einer Gruppe der Formel (CH₂)ₙ oder einer Gruppe der Formel (CH₂)ₚY(CH₂)_{q} gebunden sind, in der Y ein Sauerstoffatom, ein Schwefelatom, ein Kohlenstoffatom, das durch eine C₁-C₄-Alkylgruppe, Phenyl oder Phenylalkyl mit C₁-C₃-Alkyl substituiert ist, oder eine N-R₆-Gruppe bedeutet, in der R₆ Wasserstoff, C₁-C₄-Alkyl, Phenylalkyl mit C₁-C₃-Alkyl, C₁-C₄-Alkylcarbonyl, C₁-C₄-Halogenalkylcarbonyl, C₁-C₄-Polyhalogenalkylcarbonyl, Benzoyl, α-Aminoacyl oder eine N-Schutzgruppe bedeutet, oder R₄ und R₅ zusammen mit dem Kohlenstoffatom, an das sie gebunden sind, einen Indan- oder Adamantanrest bilden;
- p + q = m;
- n eine ganze Zahl von 2 bis 11 bedeutet;
- m eine ganze Zahl von 2 bis 5 bedeutet;
- X ein Sauerstoff- oder Schwefelatom bedeutet; und
- z und t 0 bedeuten oder eines dieser Symbole 0 und das andere 1 bedeutet;
mit der Einschränkung, daß
A/ wenn z und t 0 sind und X Sauerstoff bedeutet:
1) R₄ und R₅ eine andere Bedeutung haben als C₁-C₆-Alkyl, Phenyl oder Phenylalkyl mit C₁-C₃-Alkyl, wobei die Alkyl-, Phenyl- und Phenylalkylgruppen unsubstituiert oder durch ein oder mehr Halogenatome oder durch eine unter C₁-C₄-Perfluoralkyl, Hydroxyl und C₁-C₄-Alkoxy ausgewählte Gruppe substituiert sind; oder
2) R₄ und R₅ zusammen eine andere Bedeutung haben als die Gruppe der Formel (CH₂)ₚY(CH₂)_{q}, in der Y eine N-R₆-Gruppe bedeutet, in der R₆ Wasserstoff, C₁-C₄-Alkyl oder Phenylalkyl mit C₁-C₃-Alkyl bedeutet; oder
3) R₄ und R₅ zusammen eine andere Bedeutung haben als die Gruppe =CR₇R₈, wenn R₃ Wassserstoff, Phenyl, Methylphenyl oder Benzyl bedeutet;
4) n einen anderen Wert als 6 hat; oder
5) R₄ und R₅ zusammen eine andere Gruppe als (CH₂)ₙ bedeuten, in der n einen Wert von 3 bis 5 hat, wenn R₃ eine substituierte Phenylgruppe bedeutet;
B/ wenn z = 1 und R₃ Phenyl bedeutet, weisen R₄ und R₅ eine andere Bedeutung als Methyl auf;
C/ wenn z und t 0 sind und X Schwefel bedeutet:
1) R₄ und R₅ haben eine andere Bedeutung als Phenyl, wenn R₃ Methyl, Phenyl, Chlorphenyl, Methoxyphenyl oder Methylphenyl bedeutet; oder
2) R₄ und R₅ haben eine andere Bedeutung als C₁-C₃-Alkyl, wenn R₃ Methyl oder Phenyl bedeutet; oder
3) R₄ und R₅ haben eine andere Bedeutung als Methoxyphenyl oder Chlorphenyl, wenn R₃ Phenyl bedeutet; oder
4) R₄ und R₅ haben eine andere Bedeutung als Chlorphenyl, wenn R₃ Methyl bedeutet.

12. Verbindung nach Anspruch 11 der Formel 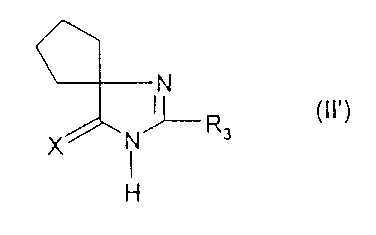 in der X ein Sauerstoff- oder Schwefelatom bedeutet und R₃ Wasserstoff, unsubstituiertes oder durch ein oder mehr Halogenatome substituiertes C₁-C₆-Alkyl, C₂-C₆-Alkenyl, C₃-C₇-Cycloalkyl, Phenyl, Phenylalkyl mit C₁-C₃-Alkyl oder Phenylalkenyl mit C₂-C₃-Alkenyl bedeutet, wobei die Phenylgruppen unsubstituiert oder ein- oder mehrfach durch ein Halogenatom, C₁-C₄-Alkyl, C₁-C₄-Halogenalkyl, C₁-C₄-Polyhalogenalkyl, Hydroxyl oder C₁-C₄-Alkoxy substituiert sind; mit der Bedingung, daß R₃ eine andere Bedeutung als substituiertes Phenyl hat, wenn X Sauerstoff bedeutet.

13. Verbindung nach Anspruch 12, dadurch gekennzeichnet, daß in der Formel II' X Sauerstoff bedeutet und R₃ n-Butyl bedeutet und daß es sich um 2-n-Butyl-4-spirocyclopentan-2-imidazolin-5-on handelt.

14. Hydrochlorid von 2-n-Butyl-4-spirocyclopentan-2-imidazolin-5-on.

15. Verbindung nach Anspruch 11 der Formel 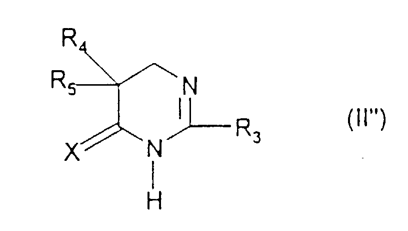 in der R₃, R₄, R₅ und X die vorstehend im Anspruch 11 für (II) angegebenen Bedeutungen aufweisen.

16. Verbindung nach Anspruch 11 der Formel 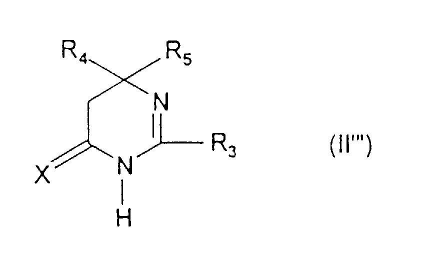 in der
- R₃ Wasserstoff, unsubstituiertes oder durch ein oder mehr Halogenatome substituiertes C₁-C₆-Alkyl, C₂-C₆-Alkenyl, C₃-C₇-Cycloalkyl, Phenyl, Phenylalkyl mit C₁-C₃-Alkyl oder Phenylalkenyl mit C₂-C₃-Alkenyl bedeutet, wobei die Phenylgruppen unsubstituiert oder ein- oder mehrfach durch ein Halogenatom, C₁-C₄-Alkyl, C₁-C₄-Halogenalkyl, C₁-C₄-Polyhalogenalkyl, Hydroxyl oder C₁-C₄-Alkoxy substituiert sind;
- R₄ und R₅ jeweils unabhängig voneinander C₁-C₆-Alkyl, Phenyl oder Phenylalkyl mit C₁-C₃-Alkyl bedeuten, wobei die Alkyl-, Phenyl- und Phenylalkylgruppen unsubstituiert oder durch ein oder mehr Halogenatome oder eine unter C₁-C₄-Perfluoralkyl, Hydroxyl und C₁-C₄-Alkoxy ausgewählte Gruppe substituiert sind;
- oder R₄ und R₅ zusammen eine Gruppe der Formel =CR₇R₈ bilden, in der R₇ Wasserstoff, C₁-C₄-Alkyl oder Phenyl bedeutet und R₈ C₁-C₄-Alkyl oder Phenyl bedeutet;
- oder R₄ und R₅ miteinander zu einer Gruppe der Formel (CH₂)ₙ oder einer Gruppe der Formel (CH₂)ₚY(CH₂)_{q} gebunden sind, in der Y ein Sauerstoffatom, ein Schwefelatom, ein Kohlenstoffatom, das durch eine C₁-C₄-Alkylgruppe, Phenyl oder Phenylalkyl mit C₁-C₃-Alkyl substituiert ist, oder eine N-R₆-Gruppe bedeutet, in der R₆ Wasserstoff, C₁-C₄-Alkyl, Phenylalkyl mit C₁-C₄-Alkyl, C₁-C₄-Alkylcarbonyl, C₁-C₄-Halogenalkylcarbonyl, C₁-C₄-Polyhalogenalkylcarbonyl, Benzoyl, α-Aminoacyl oder eine N-Schutzgruppe bedeutet, oder R₄ und R₅ zusammen mit dem Kohlenstoffatom, an das sie gebunden sind, einen Indan- oder Adamantanrest bilden;
- p + q = m;
- n eine ganze Zahl von 2 bis 11 bedeutet;
- m eine ganze Zahl von 2 bis 5 bedeutet;
- X ein Sauerstoff- oder Schwefelatom bedeutet;
mit der Einschränkung, daß R₃ eine andere Bedeutung als Phenyl hat, wenn R₄ und R₅ jeweils Methyl bedeuten.

17. Verfahren zur Herstellung einer Verbindung nach einem der Ansprüche 11 bis 16, dadurch gekennzeichnet, daß man eine Verbindung der Formel 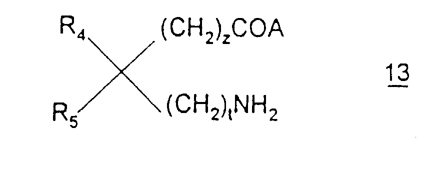 in der R₄ und R₅ die vorstehend im Anspruch 11 für (II) angegebenen Bedeutungen haben, A eine OH-Gruppe, eine NH₂-Gruppe oder eine OR'-Gruppe bedeutet, worin R' Wasserstoff oder einen C₁-C₄-Alkylrest bedeutet, mit einer Verbindung der Formel umsetzt
R₃ - B 14
in der R₃ die in den Ansprüchen 11 bis 16 angegebenen Bedeutungen hat und B
- eine Gruppe der Formel C(OR)₃
- eine Gruppe der Formel 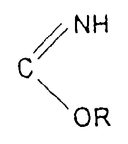 oder
- eine Gruppe der Formel COHal bedeutet,
wobei R C₁-C₄-Alkyl bedeutet und Hal ein Halogenatom und vorzugsweise Chlor bedeutet;
wobei anschließend die auf diese Weise erhaltene Verbindung gegebenenfalls mit dem Lawesson-Reagenz [2,4-Bis-(4-methoxyphenyl)-1,3-dithia-2,4-diphosphetan-2,4-disulfid] umgesetzt wird.

18. Pharmazeutische Zusammensetzung, enthaltend als Wirkstoff eine Verbindung nach einem der Ansprüche 1 bis 7.

19. Pharmazeutische Zusammensetzung, enthaltend eine Verbindung nach einem der Ansprüche 1 bis 7 zusammen mit einer β-Blocker-Verbindung.

20. Pharmazeutische Zusammensetzung, enthaltend eine Verbindung nach einem der Ansprüche 1 bis 7 zusammen mit einem Diuretikum.

21. Pharmazeutische Zusammensetzung, enthaltend eine Verbindung nach einem der Ansprüche 1 bis 7 zusammen mit einem Nichtsteroid-Antiphlogistikum.

22. Pharmazeutische Zusammensetzung, enthaltend eine Verbindung nach einem der Ansprüche 1 bis 7 zusammen mit einem Calciumantagonisten.

23. Pharmazeutische Zusammensetzung, enthaltend eine Verbindung nach einem der Ansprüche 1 bis 7 zusammen mit einem Tranquilizer.

## Patentansprüche (Patentansprüche für folgende(n) Vertragsstaat(en): ES)

1. Verfahren zur Herstellung einer Verbindung der Formel 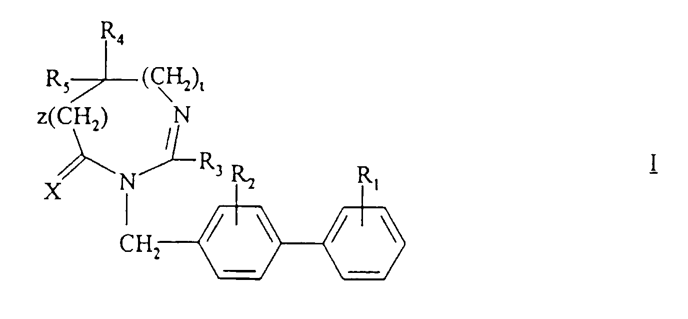 in der:
- R₁ und R₂ gleichartig oder verschieden sind und jeweils unabhängig voneinander Wasserstoff oder eine unter C₁-C₆-Alkyl, C₁-C₄-Alkoxy, Amino, Aminomethyl, Carboxy, Alkoxycarbonyl mit C₁-C₄-Alkoxy, Cyano, Tetrazolyl, Methyltetrazolyl, Methylsulfonylamino, Trifluormethylsulfonylamino, Trifluormethylsulfonylaminomethyl, N-Cyanocarbamoyl, N-Hydroxycarbamoyl, N-((4-Carboxy)-1,3-thiazol-2-yl)-carbamoyl, Ureido, 2-Cyanoguanidinocarbonyl, 2-Cyanoguanidinomethyl, Imidazol-1-ylcarbonyl und 3-Cyano-2-methylisothioureidomethyl ausgewählte Gruppe bedeutet, mit der Bedingung, daß mindestens einer der Substituenten R₁ oder R₂ eine andere Bedeutung als Wasserstoff hat;
- R₃ Wasserstoff, unsubstituiertes oder durch ein oder mehr Halogenatome substituiertes C₁-C₆-Alkyl, C₂-C₆-Alkenyl, C₃-C₇-Cycloalkyl, Phenyl, Phenylalkyl mit C₁-C₃-Alkyl oder Phenylalkenyl mit C₂-C₃-Alkenyl bedeutet, wobei die Phenylgruppen unsubstituiert oder ein- oder mehrfach durch ein Halogenatom, C₁-C₄-Alkyl, C₁-C₄-Halogenalkyl, C₁-C₄-Polyhalogenalkyl, Hydroxyl oder C₁-C₄-Alkoxy substituiert sind;
- R₄ und R₅ jeweils unabhängig voneinander C₁-C₆-Alkyl, Phenyl oder Phenylalkyl mit C₁-C₃-Alkyl bedeuten, wobei die Alkyl-, Phenyl- und Phenylalkylgruppen unsubstituiert oder durch ein oder mehr Halogenatome oder durch eine unter C₁-C₄-Perfluoralkyl, Hydroxyl und C₁-C₄-Alkoxy ausgewählte Gruppe substituiert sind;
- oder R₄ und R₅ zusammen eine Gruppe der Formel =CR₇R₈ bilden, in der R₇ Wasserstoff, C₁-C₄-Alkyl oder Phenyl bedeutet und R₈ C₁-C₄-Alkyl oder Phenyl bedeutet;
- oder R₄ und R₅ miteinander zu einer Gruppe der Formel (CH₂)ₙ oder einer Gruppe der Formel (CH₂)ₚY(CH₂)_{q} gebunden sind, in der Y ein Sauerstoffatom, ein Schwefelatom, ein Kohlenstoffatom, das durch eine C₁-C₄-Alkylgruppe, Phenyl oder Phenylalkyl mit C₁-C₃-Alkyl substituiert ist, oder eine N-R₆-Gruppe bedeutet, in der R₆ Wasserstoff, C₁-C₄-Alkyl, Phenylalkyl mit C₁-C₃-Alkyl, C₁-C₄-Alkylcarbonyl, C₁-C₄-Halogenalkylcarbonyl, C₁-C₄-Polyhalogenalkylcarbonyl, Benzoyl, α-Aminoacyl oder eine N-Schutzgruppe bedeutet, oder R₄ und R₅ zusammen mit dem Kohlenstoffatom, an das sie gebunden sind, einen Indan- oder Adamantanrest bilden;
- p + q = m;
- n eine ganze Zahl von 2 bis 11 bedeutet;
- m eine ganze Zahl von 2 bis 5 bedeutet;
- X ein Sauerstoff- oder Schwefelatom bedeutet; und
- z und t 0 bedeuten oder eines dieser Symbole 0 und das andere 1 bedeutet;
und Salze davon,
dadurch gekennzeichnet, daß man
a1) ein heterocyclisches Derivat der folgenden Formel 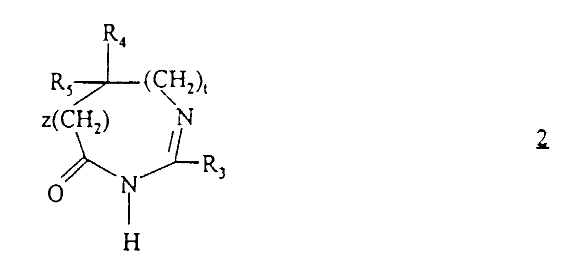 in der z, t, R₃, R₄ und R₅ die vorstehend für (I) angegebenen Bedeutungen haben, mit einem (Biphenyl-4-yl)-methylderivat der Formel 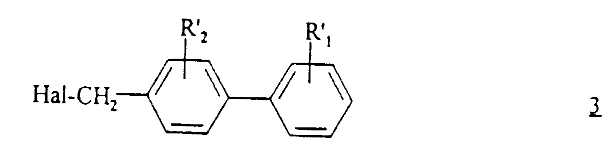 umsetzt, in der Hal ein Halogenatom bedeutet und R'₁ und R'₂ entweder die Bedeutung von R₁ bzw. R₂ haben oder eine Vorstufe für R₁ bzw. R₂ bedeuten;
bl) gegebenenfalls die erhaltene Verbindung der Formel 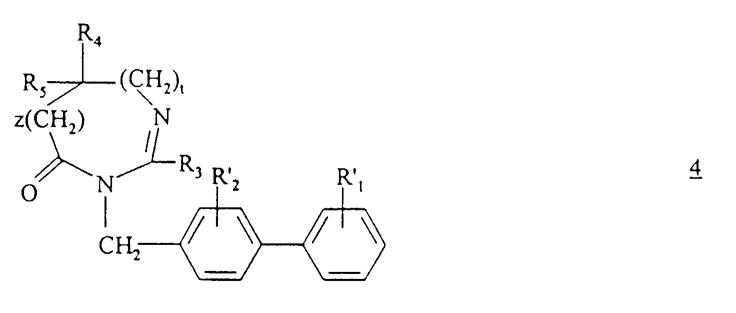 mit dem Lawesson-Reagenz [2,4-Bis- (4-methoxyphenyl)-1,3-dithia-2,4-diphosphetan-2,4-disulfid] behandelt;
c1) die in a1) oder b1) erhaltene Verbindung der Formel 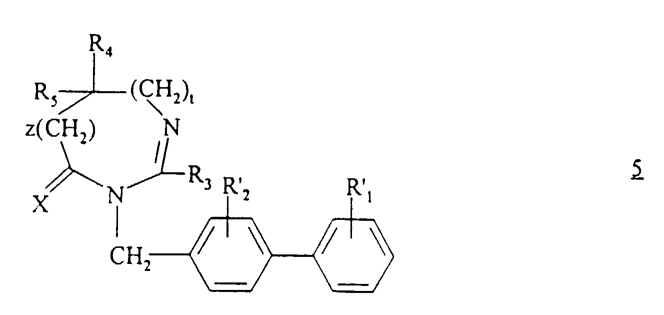 in der X ein Sauerstoff- oder Schwefelatom bedeutet, zur Herstellung der Verbindung (I) durch Umwandlung der Gruppen R'₁ und/oder R'₂ in die entsprechenden Gruppen R₁ und/oder R₂ behandelt, wobei die Verbindung gegebenenfalls anschließend in eines ihrer Salze übergeführt wird.

2. Verfahren zur Herstellung der Verbindungen der Formel (I) 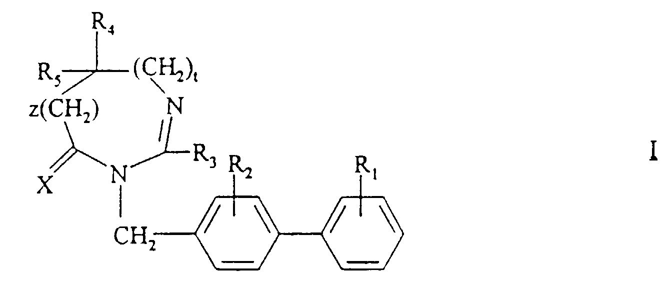 in der R₁, R₂, R₃, R₄, R₅, X, z und t die in Anspruch 1 definierten Bedeutungen haben, dadurch gekennzeichnet, daß man
a2) eine Aminosäure der Formel 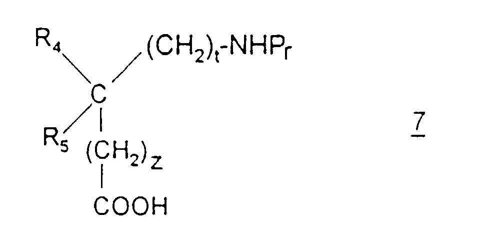 in der z, t, R₄ und R₅ die im Anspruch 1 für (I) angegebenen Bedeutungen haben und deren Aminfunktion durch die Gruppe Pr geschützt ist, mit einem (Biphenyl-4-yl)-methylaminderivat der Formel 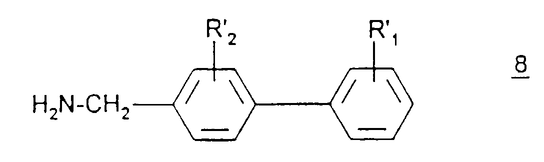 in der R'₁ und R'₂ entweder die Bedeutungen R₁ bzw. R₂ haben oder eine Vorstufe für R₁ bzw. R₂ bedeuten, umsetzt,
b2) nach Schutzgruppenentfernung am Amin die erhaltene Verbindung der Formel 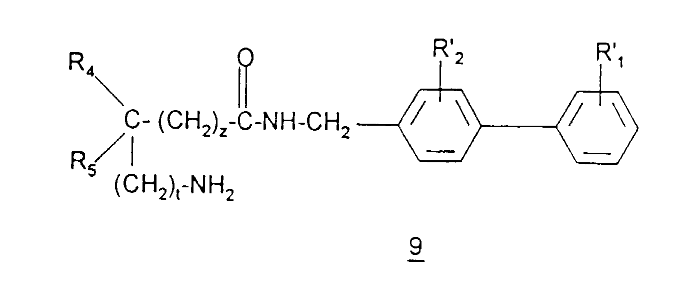 anschließend mit einem Alkylorthoester der Formel R₃C(OR)₃ (10), in der R₃ die im Anspruch 1 für (I) angegebene Bedeutung hat und R einen C₁-C₄-Alkylrest bedeutet, behandelt;
c2) gegebenenfalls die erhaltene Verbindung der Formel 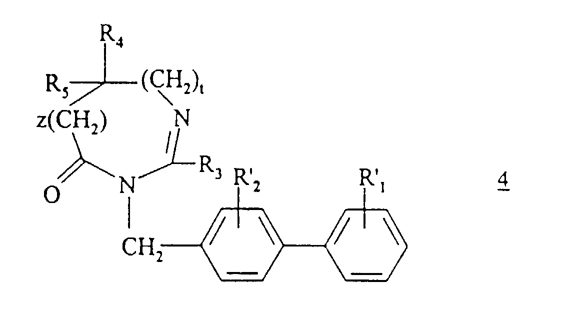 mit dem Lawesson-Reagenz [2,4-Bis-(4-methoxyphenyl)-1,3-dithia-2,4-diphosphetan-2,4-disulfid] behandelt; d2) die in b2) oder c2) erhaltene Verbindung der Formel 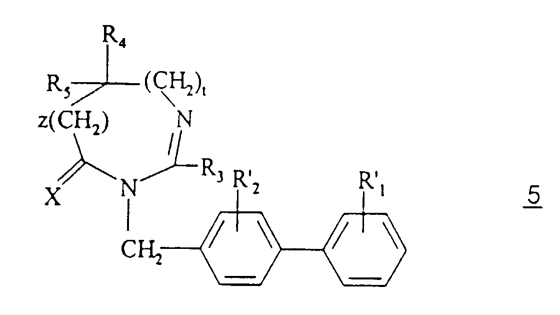 anschließend unter für die Herstellung der Verbindung (I) zweckmäßigen Bedingungen durch Umwandlung der Gruppen R'₂ und/oder R'₁ in die entsprechenden Gruppen R₂ und/oder R₁ behandelt, wobei die Verbindung anschließend gegebenenfalls in eines ihrer Salze übergeführt wird.

3. Verfahren zur Herstellung der Verbindungen der Formel (I) 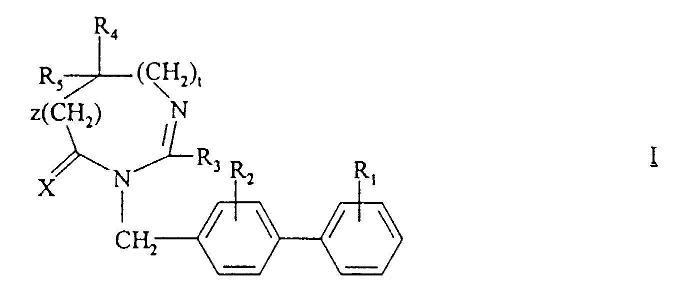 in der R₁, R₂, R₃, R₄, R₅ und X die in Anspruch 1 definierten Bedeutungen haben und z und t jeweils den Wert 0 haben, dadurch gekennzeichnet, daß man
a3) ein Imidazolderivat der Formel 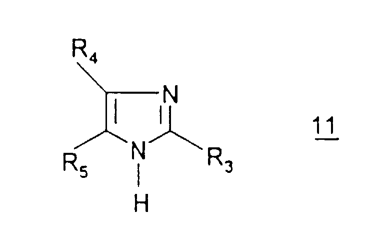 in der R₃, R₄ und R₅ die in Anspruch 1 für (I) angegebenen Bedeutungen haben, mit einem (Biphenyl-4-yl)-methylderivat der Formel 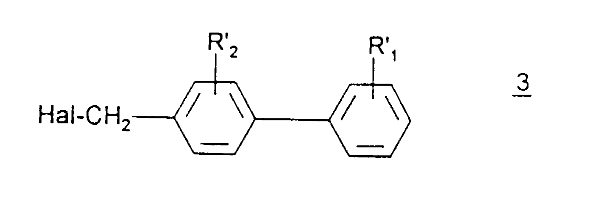 in der Hal ein Halogenatom bedeutet und R'₁ und R'₂ entweder die Bedeutung von R₁ bzw. R₂ haben oder eine Vorstufe für R₁ bzw. R₂ bedeuten, in Gegenwart von Sauerstoff und UV-Strahlung in basischem Milieu umsetzt;
b3) gegebenenfalls die erhaltene Verbindung der Formel 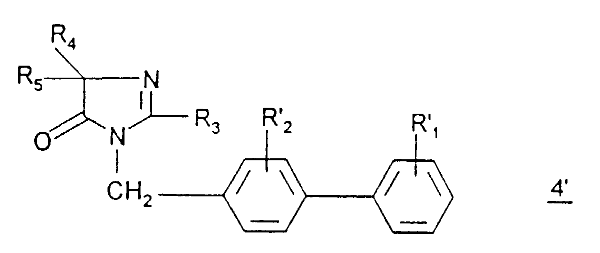 mit dem Lawesson-Reagenz [2,4-Bis-(4-methoxyphenyl)-1,3-dithia-2,4-diphosphetan-2,4-disulfid] behandelt;
c3) die in b3) oder c3) erhaltene Verbindung der Formel 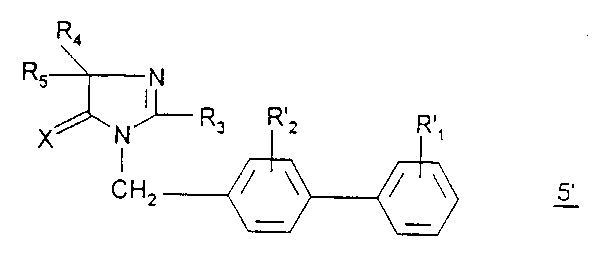 anschließend unter für die Herstellung der Verbindung (I) zweckmäßigen Bedingungen durch Umwandlung der Gruppen R'₁ und/oder R'₂ in die entsprechenden Gruppen R₁ und/oder R₂ behandelt, wobei die Verbindung anschließend gegebenenfalls in eines ihrer Salze übergeführt wird.

4. Verfahren nach einem der Ansprüche 1 bis 3, dadurch gekennzeichnet, daß in der Verbindung der Formel 3 oder 8 R'₂ Wasserstoff bedeutet und R'₁ eine veresterte Carboxygruppe bedeutet, die in den Stufen c1), d2) oder c3) in eine Carboxygruppe übergeführt wird.

5. Verfahren nach einem der Ansprüche 1 bis 3, dadurch gekennzeichnet, daß in der Verbindung der Formel 3 oder 8 R'₂ Wasserstoff bedeutet und R'₁ eine geschützte Tetrazolylgruppe oder eine Cyanogruppe bedeutet, die in den Stufen c1), d2) oder c3) in eine Tetrazolylgruppe übergeführt wird.

6. Verfahren nach einem der Ansprüche 1 bis 5, dadurch gekennzeichnet, daß in den Verbindungen der Formeln 2, 7 oder 11 R₄ und R₅ zusammen mit dem Kohlenstoffatom, an das sie gebunden sind, einen Cyclopentan- oder Cyclohexanrest bilden.

7. Verfahren nach einem der Ansprüche 1 bis 6, dadurch gekennzeichnet, daß in den Verbindungen der Formeln 2, 10 und 11 R₃ eine geradkettige C₁-C₆-Alkylgruppe bedeutet.

8. Verfahren nach einem der Ansprüche 1 bis 7, dadurch gekennzeichnet, daß es die Stufen a1) und c1); a2), b2) und d2); oder a3) und b3) umfaßt.

9. Verfahren nach einem der Ansprüche 1 oder 2, dadurch gekennzeichnet, daß in den Verbindungen der Formel 2 und 7 z und t jeweils den Wert 0 haben.

10. Verfahren nach Anspruch 1, dadurch gekennzeichnet, daß man verwendet: 1) eine Verbindung der Formel 2, in der R₃ eine n-Butylgruppe bedeutet, R₄ und R₅ zusammen mit dem Kohlenstoffatom, an das sie gebunden sind, eine Spirocyclopentangruppe bilden und z und t jeweils den Wert 0 haben; 2) eine Verbindung der Formel 3, in der R'₂ Wasserstoff bedeutet und R'₁ eine geschützte Tetrazolylgruppe bedeutet, wodurch man 2-n-Butyl-4-spirocyclopentan-1-[(2'-(5-tetrazolylbiphenyl-4-yl)-methyl]-2-imidazolin-5-on oder ein Salz davon mit Säuren oder Basen erhält.

11. Verfahren zur Herstellung einer Verbindung der Formel 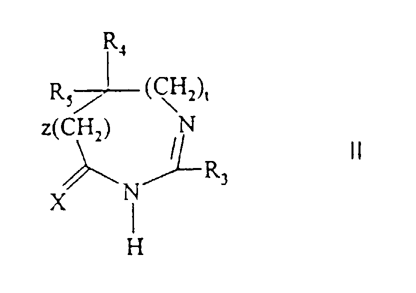 in der:
- R₃ Wasserstoff, unsubstituiertes oder durch ein oder mehr Halogenatome substituiertes C₁-C₆-Alkyl, C₂-C₆-Alkenyl, C₃-C₇-Cycloalkyl, Phenyl, Phenylalkyl mit C₁-C₃-Alkyl oder Phenylalkenyl mit C₂-C₃-Alkenyl bedeutet, wobei die Phenylgruppen unsubstituiert oder ein- oder mehrfach durch ein Halogenatom, C₁-C₄-Alkyl, C₁-C₄-Halogenalkyl, C₁-C₄-Polyhalogenalkyl, Hydroxyl oder C₁-C₄-Alkoxy substituiert sind;
- R₄ und R₅ jeweils unabhängig voneinander C₁-C₆-Alkyl, Phenyl oder Phenylalkyl mit C₁-C₃-Alkyl bedeuten, wobei die Alkyl-, Phenyl- und Phenylalkylgruppen unsubstituiert oder durch ein oder mehr Halogenatome oder durch eine unter C₁-C₄-Perfluoralkyl, Hydroxyl und C₁-C₄-Alkoxy ausgewählte Gruppe substituiert sind;
- oder R₄ und R₅ zusammen eine Gruppe der Formel =CR₇R₈ bilden, in der R₇ Wasserstoff, C₁-C₄-Alkyl oder Phenyl bedeutet und R₈ C₁-C₄-Alkyl oder Phenyl bedeutet;
- oder R₄ und R₅ miteinander zu einer Gruppe der Formel (CH₂)ₙ oder einer Gruppe der Formel (CH₂)ₚY(CH₂)_{q} gebunden sind, in der Y ein Sauerstoffatom, ein Schwefelatom, ein Kohlenstoffatom, das durch eine C₁-C₄-Alkylgruppe, Phenyl oder Phenylalkyl mit C₁-C₃-Alkyl substituiert ist, oder eine N-R₆-Gruppe bedeutet, in der R₆ Wasserstoff, C₁-C₄-Alkyl, Phenylalkyl mit C₁-C₃-Alkyl, C₁-C₄-Alkylcarbonyl, C₁-C₄-Halogenalkylcarbonyl, C₁-C₄-Polyhalogenalkylcarbonyl, Benzoyl, α-Aminoacyl oder eine N-Schutzgruppe bedeutet, oder R₄ und R₅ zusammen mit dem Kohlenstoffatom, an das sie gebunden sind, einen Indan- oder Adamantanrest bilden;
- p + q = m;
- n eine ganze Zahl von 2 bis 11 bedeutet;
- m eine ganze Zahl von 2 bis 5 bedeutet;
- X ein Sauerstoff- oder Schwefelatom bedeutet; und
- z und t 0 bedeuten oder eines dieser Symbole 0 und das andere 1 bedeutet;
mit der Einschränkung, daß
A/ wenn z und t 0 sind und X Sauerstoff bedeutet:
1) R₄ und R₅ eine andere Bedeutung haben als C₁-C₆-Alkyl, Phenyl oder Phenylalkyl mit C₁-C₃-Alkyl, wobei die Alkyl-, Phenyl- und Phenylalkylgruppen unsubstituiert oder durch ein oder mehr Halogenatome oder durch eine unter C₁-C₄-Perfluoralkyl, Hydroxyl und C₁-C₄-Alkoxy ausgewählte Gruppe substituiert sind; oder
2) R₄ und R₅ zusammen eine andere Bedeutung haben als die Gruppe der Formel (CH₂)ₚY(CH₂)_{q}, in der Y eine N-R₆-Gruppe bedeutet, in der R₆ Wasserstoff, C₁-C₄-Alkyl oder Phenylalkyl mit C₁-C₃-Alkyl bedeutet; oder
3) R₄ und R₅ zusammen eine andere Bedeutung haben als die Gruppe =CR₇R₈, wenn R₃ Wassserstoff, Phenyl, Methylphenyl oder Benzyl bedeutet;
4) n einen anderen Wert als 6 hat; oder
5) R₄ und R₅ zusammen eine andere Gruppe als (CH₂)ₙ bedeuten, in der n einen Wert von 3 bis 5 hat, wenn R₃ eine substituierte Phenylgruppe bedeutet;
B/ wenn z = 1 und R₃ Phenyl bedeutet, weisen R₄ und R₅ eine andere Bedeutung als Methyl auf;
C/ wenn z und t 0 sind und X Schwefel bedeutet:
1) R₄ und R₅ haben eine andere Bedeutung als Phenyl, wenn R₃ Methyl, Phenyl, Chlorphenyl, Methoxyphenyl oder Methylphenyl bedeutet; oder
2) R₄ und R₅ haben eine andere Bedeutung als C₁-C₃-Alkyl, wenn R₃ Methyl oder Phenyl bedeutet; oder
3) R₄ und R₅ haben eine andere Bedeutung als Methoxyphenyl oder Chlorphenyl, wenn R₃ Phenyl bedeutet; oder
4) R₄ und R₅ haben eine andere Bedeutung als Chlorphenyl, wenn R₃ Methyl bedeutet,
dadurch gekennzeichnet, daß man eine Verbindung der Formel 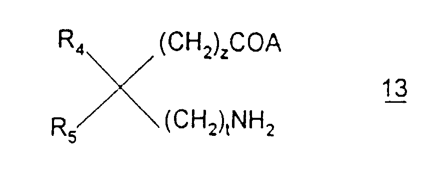 in der R₄ und R₅ die vorstehend für (II) angegebenen Bedeutungen haben und A eine OH-Gruppe, eine NH₂-Gruppe oder eine OR'-Gruppe bedeutet, worin R' Wasserstoff oder einen C₁-C₄-Alkylrest bedeutet, mit einer Verbindung der Formel umsetzt
R₃ - B 14
in der R₃ die in vorstehend für (II) angegebenen Bedeutungen hat und B
- eine Gruppe der Formel C(OR)₃
- eine Gruppe der Formel 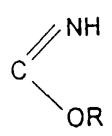 oder
- eine Gruppe der Formel COHal bedeutet,
wobei R C₁-C₄-Alkyl bedeutet und Hal ein Halogenatom und vorzugsweise Chlor bedeutet;
wobei anschließend die auf diese Weise erhaltene Verbindung gegebenenfalls mit dem Lawesson-Reagenz [2,4-Bis-(4-methoxyphenyl)-1,3-dithia-2,4-diphosphetan-2,4-disulfid] umgesetzt wird.

12. Verfahren zur Herstellung einer Verbindung nach Anspruch 11 der Formel 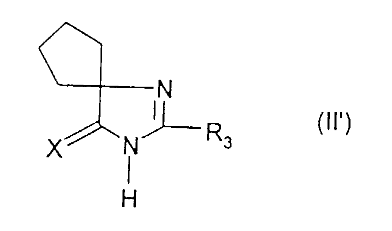 in der X ein Sauerstoff- oder Schwefelatom bedeutet und R₃ Wasserstoff, unsubstituiertes oder durch ein oder mehr Halogenatome substituiertes C₁-C₆-Alkyl, C₂-C₆-Alkenyl, C₃-C₇-Cycloalkyl, Phenyl, Phenylalkyl mit C₁-C₃-Alkyl oder Phenylalkenyl mit C₂-C₃-Alkenyl bedeutet, wobei die Phenylgruppen unsubstituiert oder ein- oder mehrfach durch ein Halogenatom, C₁-C₄-Alkyl, C₁-C₄-Halogenalkyl, C₁-C₄-Polyhalogenalkyl, Hydroxyl oder C₁-C₄-Alkoxy substituiert sind; mit der Bedingung, daß R₃ eine andere Bedeutung als substituiertes Phenyl hat, wenn X Sauerstoff bedeutet,
dadurch gekennzeichnet, daß man eine Verbindung der Formel 13, in der z und t jeweils den Wert 0 haben und R₄ und R₅ zusammen mit dem Kohlenstoffatom, an das sie gebunden sind, einen Spirocyclopentanrest bilden, und eine Verbindung der Formel 14, in der R₃ die vorstehend definierte Bedeutung hat, verwendet.

13. Verfahren nach Anspruch 12, dadurch gekennzeichnet, daß man eine Verbindung der Formel 13, in der z und t jeweils den Wert 0 haben und R₄ und R₅ zusammen mit dem Kohlenstoffatom, an das sie gebunden sind, einen Spirocyclopentanrest bilden, und eine Verbindung der Formel 14, in der R₃ eine n-Butylgruppe bedeutet, verwendet, wodurch man 2-n-Butyl-4-spirocyclopentan-2-imidazolin-5-on erhält.

14. Verfahren zur Herstellung des Hydrochlorids von 2-n-Butyl-4-spirocyclopentan-2-imidazolin-5-on, dadurch gekennzeichnet, daß man die im Verfahren nach Anspruch 13 erhaltene Verbindung mit konzentrierter Salzsäure versetzt.

15. Verfahren nach Anspruch 11 zur Herstellung einer Verbindung der Formel 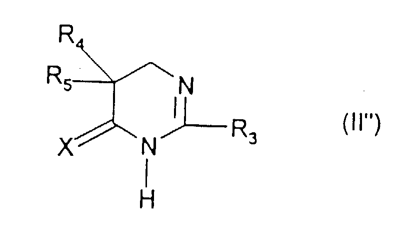 in der R₃, R₄, R₅ und X die vorstehend für (II) angegebenen Bedeutungen aufweisen.
dadurch gekennzeichnet, daß man eine Verbindung der Formel 13, in der t den Wert 1 und z den Wert 0 hat und R₄ und R₅ die in Anspruch 11 angegebenen Bedeutungen haben, und eine Verbindung der Formel 14, in der R₃ die in Anspruch 11 definierte Bedeutung hat, verwendet.

16. Verfahren nach Anspruch 11 zur Herstellung einer Verbindung der Formel 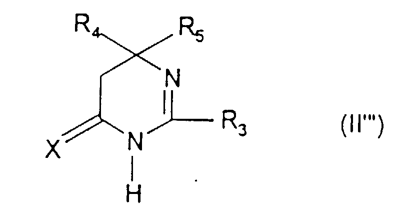 in der
- R₃ Wasserstoff, unsubstituiertes oder durch ein oder mehr Halogenatome substituiertes C₁-C₆-Alkyl, C₂-C₆-Alkenyl, C₃-C₇-Cycloalkyl, Phenyl, Phenylalkyl mit C₁-C₃-Alkyl oder Phenylalkenyl mit C₂-C₃-Alkenyl bedeutet, wobei die Phenylgruppen unsubstituiert oder ein- oder mehrfach durch ein Halogenatom, C₁-C₄-Alkyl, C₁-C₄-Halogenalkyl, C₁-C₄-Polyhalogenalkyl, Hydroxyl oder C₁-C₄-Alkoxy substituiert sind;
- R₄ und R₅ jeweils unabhängig voneinander C₁-C₆-Alkyl, Phenyl oder Phenylalkyl mit C₁-C₃-Alkyl bedeuten, wobei die Alkyl-, Phenyl- und Phenylalkylgruppen unsubstituiert oder durch ein oder mehr Halogenatome oder eine unter C₁-C₄-Perfluoralkyl, Hydroxyl und C₁-C₄-Alkoxy ausgewählte Gruppe substituiert sind;
- oder R₄ und R₅ zusammen eine Gruppe der Formel =CR₇R₈ bilden, in der R₇ Wasserstoff, C₁-C₄-Alkyl oder Phenyl bedeutet und R₈ C₁-C₄-Alkyl oder Phenyl bedeutet;
- oder R₄ und R₅ miteinander zu einer Gruppe der Formel (CH₂)ₙ oder einer Gruppe der Formel (CH₂)ₚY(CH₂)_{q} gebunden sind, in der Y ein Sauerstoffatom, ein Schwefelatom, ein Kohlenstoffatom, das durch eine C₁-C₄-Alkylgruppe, Phenyl oder Phenylalkyl mit C₁-C₃-Alkyl substituiert ist, oder eine N-R₆-Gruppe bedeutet, in der R₆ Wasserstoff, C₁-C₄-Alkyl, Phenylalkyl mit C₁-C₄-Alkyl, C₁-C₄-Alkylcarbonyl, C₁-C₄-Halogenalkylcarbonyl, C₁-C₄-Polyhalogenalkylcarbonyl, Benzoyl, α-Aminoacyl oder eine N-Schutzgruppe bedeutet, oder R₄ und R₅ zusammen mit dem Kohlenstoffatom, an das sie gebunden sind, einen Indan- oder Adamantanrest bilden;
- p + q = m;
- n eine ganze Zahl von 2 bis 11 bedeutet;
- m eine ganze Zahl von 2 bis 5 bedeutet;
- X ein Sauerstoff- oder Schwefelatom bedeutet;
mit der Einschränkung, daß R₃ eine andere Bedeutung als Phenyl hat, wenn R₄ und R₅ jeweils Methyl bedeuten,
dadurch gekennzeichnet, daß man eine Verbindung der Formel 13, in der z den Wert 1 und t den Wert 0 hat und R₄ und R₅ die vorstehend definierten Bedeutungen haben, und eine Verbindung der Formel 14, in der R₃ die vorstehend definierte Bedeutung hat, verwendet.

17. Verfahren zur Herstellung einer pharmazeutischen Zusammensetzung, dadurch gekennzeichnet, daß es im Vermischen einer nach dem Verfahren gemäß einem der Ansprüche 1 bis 10 erhaltenen Verbindung mit einem pharmazeutisch verträglichen Träger besteht.

18. Verfahren zur Herstellung einer pharmazeutischen Zusammensetzung, dadurch gekennzeichnet, daß es im Vereinigen einer nach dem Verfahren gemäß einem der Ansprüche 1 bis 10 erhaltenen Verbindung mit einer β-Blocker-Verbindung besteht.

19. Verfahren zur Herstellung einer pharmazeutischen Zusammensetzung, dadurch gekennzeichnet, daß es im Vereinigen einer nach dem Verfahren gemäß einem der Ansprüche 1 bis 10 erhaltenen Verbindung mit einem Diuretikum besteht.

20. Verfahren zur Herstellung einer pharmazeutischen Zusammensetzung, dadurch gekennzeichnet, daß es im Vereinigen einer nach dem Verfahren gemäß einem der Ansprüche 1 bis 10 erhaltenen Verbindung mit einem Nichtsteroid-Antiphlogistikum besteht.

21. Verfahren zur Herstellung einer pharmazeutischen Zusammensetzung, dadurch gekennzeichnet, daß es im Vereinigen einer nach dem Verfahren gemäß einem der Ansprüche 1 bis 10 erhaltenen Verbindung mit einem Calciumantagonisten besteht.

22. Verfahren zur Herstellung einer pharmazeutischen Zusammensetzung, dadurch gekennzeichnet, daß es im Vereinigen einer nach dem Verfahren gemäß einem der Ansprüche 1 bis 10 erhaltenen Verbindung mit einem Tranquilizer besteht.

## Patentansprüche (Patentansprüche für folgende(n) Vertragsstaat(en): GR)

1. Verbindung der Formel 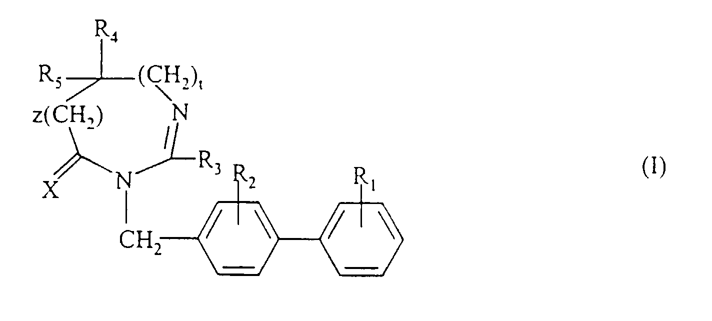 in der:
- R₁ und R₂ gleichartig oder verschieden sind und jeweils unabhängig voneinander Wasserstoff oder eine unter C₁-C₆-Alkyl, C₁-C₄-Alkoxy, Amino, Aminomethyl, Carboxy, Alkoxycarbonyl mit C₁-C₄-Alkoxy, Cyano, Tetrazolyl, Methyltetrazolyl, Methylsulfonylamino, Trifluormethylsulfonylamino, Trifluormethylsulfonylaminomethyl, N-Cyanocarbamoyl, N-Hydroxycarbamoyl, N-((4-Carboxy)-1,3-thiazol-2-yl)-carbamoyl, Ureido, 2-Cyanoguanidinocarbonyl, 2-Cyanoguanidinomethyl, Imidazol-1-ylcarbonyl und 3-Cyano-2-methylisothioureidomethyl ausgewählte Gruppe bedeutet, mit der Bedingung, daß mindestens einer der Substituenten R₁ oder R₂ eine andere Bedeutung als Wasserstoff hat;
- R₃ Wasserstoff, unsubstituiertes oder durch ein oder mehr Halogenatome substituiertes C₁-C₆-Alkyl, C₂-C₆-Alkenyl, C₃-C₇-Cycloalkyl, Phenyl, Phenylalkyl mit C₁-C₃-Alkyl oder Phenylalkenyl mit C₂-C₃-Alkenyl bedeutet, wobei die Phenylgruppen unsubstituiert oder ein- oder mehrfach durch ein Halogenatom, C₁-C₄-Alkyl, C₁-C₄-Halogenalkyl, C₁-C₄-Polyhalogenalkyl, Hydroxyl oder C₁-C₄-Alkoxy substituiert sind;
- R₄ und R₅ jeweils unabhängig voneinander C₁-C₆-Alkyl, Phenyl oder Phenylalkyl mit C₁-C₃-Alkyl bedeuten, wobei die Alkyl-, Phenyl- und Phenylalkylgruppen unsubstituiert oder durch ein oder mehr Halogenatome oder durch eine unter C₁-C₄-Perfluoralkyl, Hydroxyl und C₁-C₄-Alkoxy ausgewählte Gruppe substituiert sind;
- oder R₄ und R₅ zusammen eine Gruppe der Formel =CR₇R₈ bilden, in der R₇ Wasserstoff, C₁-C₄-Alkyl oder Phenyl bedeutet und R₈ C₁-C₄-Alkyl oder Phenyl bedeutet;
- oder R₄ und R₅ miteinander zu einer Gruppe der Formel (CH₂)ₙ oder eine Gruppe der Formel (CH₂)ₚY(CH₂)_{q} gebunden sind, in der Y ein Sauerstoffatom, ein Schwefelatom, ein Kohlenstoffatom, das durch eine C₁-C₄-Alkylgruppe, Phenyl oder Phenylalkyl mit C₁-C₃-Alkyl substituiert ist, oder eine N-R₆-Gruppe bedeutet, in der R₆ Wasserstoff, C₁-C₄-Alkyl, Phenylalkyl mit C₁-C₃-Alkyl, C₁-C₄-Alkylcarbonyl, C₁-C₄-Halogenalkylcarbonyl, C₁-C₄-Polyhalogenalkylcarbonyl, Benzoyl, α-Aminoacyl oder eine N-Schutzgruppe bedeutet, oder R₄ und R₅ zusammen mit dem Kohlenstoffatom, an das sie gebunden sind, einen Indan- oder Adamantanrest bilden;
- p + q = m;
- n eine ganze Zahl von 2 bis 11 bedeutet;
- m eine ganze Zahl von 2 bis 5 bedeutet;
- X ein Sauerstoff- oder Schwefelatom bedeutet; und
- z und t 0 bedeuten oder eines dieser Symbole 0 und das andere 1 bedeutet;
und Salze davon.

2. Verbindung nach Anspruch 1, dadurch gekennzeichnet, daß R₁ in ortho-Stellung vorliegt und eine Carboxy- oder Tetrazolylgruppe bedeutet und R₂ Wasserstoff bedeutet.

3. Verbindung nach einem der Ansprüche 1 und 2, dadurch gekennzeichnet, daß R₄ und R₅ zusammen mit dem Kohlenstoffatom, an das sie gebunden sind, einen Cyclopentan- oder Cyclohexanrest bilden.

4. Verbindung nach einem der Ansprüche 1 bis 3, dadurch gekennzeichnet, daß R₃ eine geradkettige C₁-C₆-Alkylgruppe bedeutet.

5. Verbindung nach einem der Ansprüche 1 bis 4, dadurch gekennzeichnet, daß X Sauerstoff bedeutet.

6. Verbindung nach einem der Ansprüche 1 bis 5, dadurch gekennzeichnet, daß z = t = 0.

7. Verbindung nach Anspruch 1, dadurch gekennzeichnet, daß es sich um 2-n-Butyl-4-spirocyclopentan-1-[(2'-(5-tetrazolyl)-biphenyl-4-yl)-methyl]-2-imidazolin-5-on oder ein Salz davon mit Säuren oder Basen handelt.

8. Verfahren zur Herstellung einer Verbindung (I) nach einem der Ansprüche 1 bis 7, dadurch gekennzeichnet, daß man
a1) ein heterocyclisches Derivat der folgenden Formel 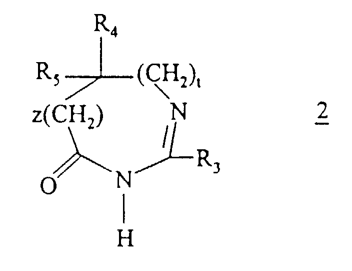 in der z, t, R₃, R₄ und R₅ die im Anspruch 1 für (I) angegebenen Bedeutungen haben, mit einem (Biphenyl-4-yl)-methylderivat der Formel 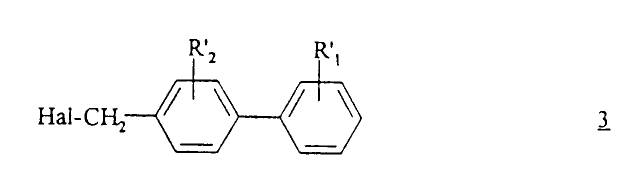 umsetzt, in der Hal ein Halogenatom bedeutet und R'₁ und R'₂ entweder die Bedeutung von R₁ bzw. R₂ haben oder eine Vorstufe für R₁ bzw. R₂ bedeuten;
b1) gegebenenfalls die erhaltene Verbindung der Formel 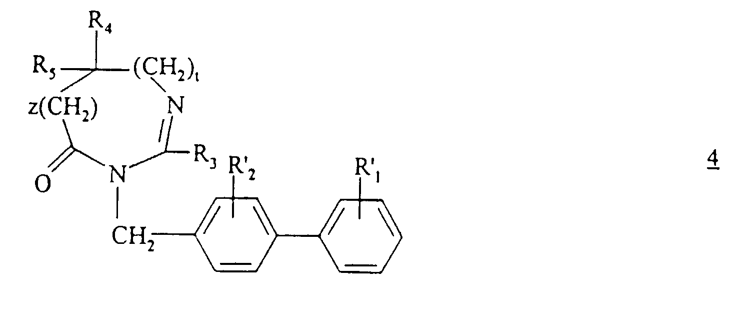 mit dem Lawesson-Reagenz [2,4-Bis-(4-methoxyphenyl)-1,3-dithia-2,4-diphosphetan-2,4-disulfid] behandelt;
c1) die in a1) oder b1) erhaltene Verbindung der Formel 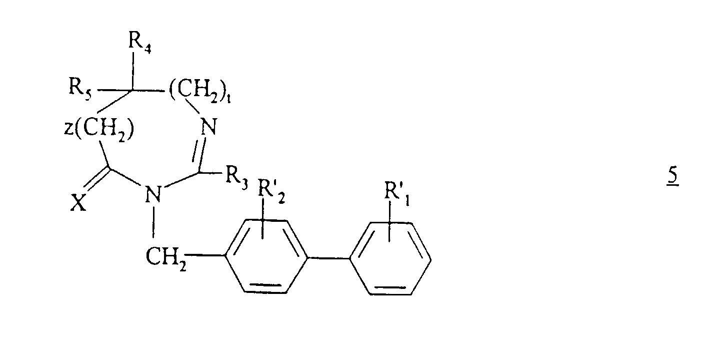 in der X ein Sauerstoff- oder Schwefelatom bedeutet, zur Herstellung der Verbindung (I) durch Umwandlung der Gruppen R'₁ und/oder R'₂ in die entsprechenden Gruppen R₁ und/oder R₂ behandelt, wobei die Verbindung gegebenenfalls anschließend in eines ihrer Salze übergeführt wird.

9. Verfahren zur Herstellung einer Verbindung (I) nach einem der Ansprüche 1 bis 7, dadurch gekennzeichnet, daß man
a2) eine Aminosäure der Formel 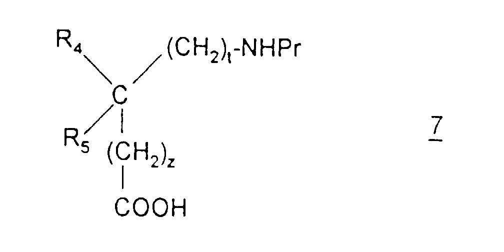 in der z, t, R₄ und R₅ die im Anspruch 1 für (I) angegebenen Bedeutungen haben und deren Aminfunktion durch die Gruppe Pr geschützt ist, mit einem (Biphenyl-4-yl)-methylaminderivat der Formel 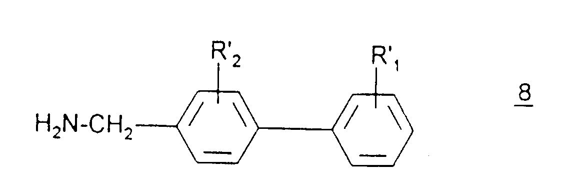 in der R'₁ und R'₂ entweder die Bedeutungen R₁ bzw. R₂ haben oder eine Vorstufe für R₁ bzw. R₂ bedeuten, umsetzt,
b2) nach Schutzgruppenentfernung am Amin die erhaltene Verbindung der Formel 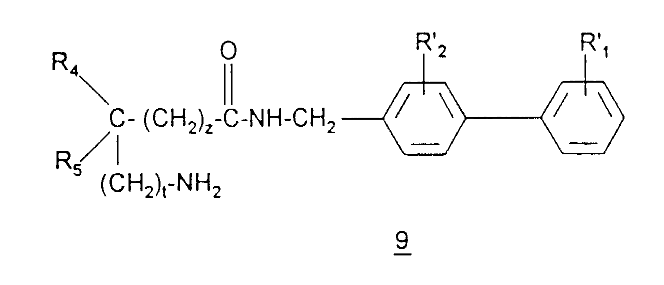 anschließend mit einem Alkylorthoester der Formel R₃C(OR)₃ (10), in der R₃ die im Anspruch 1 für (I) angegebene Bedeutung hat und R einen C₁-C₄-Alkylrest bedeutet, behandelt;
c2) gegebenenfalls die erhaltene Verbindung der Formel 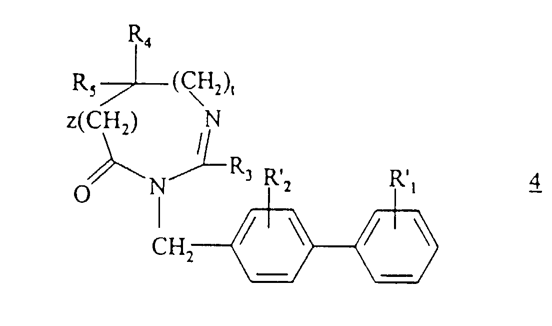 mit dem Lawesson-Reagenz [2,4-Bis-(4-methoxyphenyl)-1,3-dithia-2,4-diphosphetan-2,4-disulfid] behandelt;
d2) die in b2) oder c2) erhaltene Verbindung der Formel 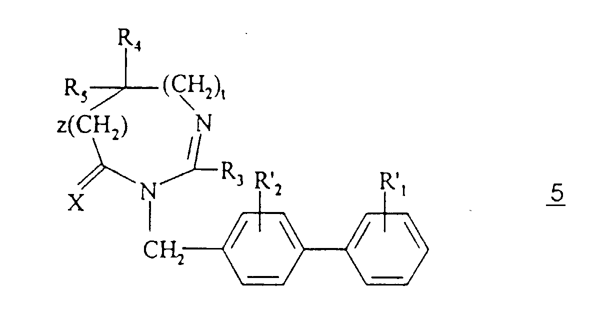 anschließend unter für die Herstellung der Verbindung (I) zweckmäßigen Bedingungen durch Umwandlung der Gruppen R'₂ und/oder R'₁ in die entsprechenden Gruppen R₂ und/oder R₁ behandelt, wobei die Verbindung anschließend gegebenenfalls in eines ihrer Salze übergeführt wird.

10. Verfahren zur Herstellung einer Verbindung (I) nach Anspruch 6, dadurch gekennzeichnet, daß man
a3) ein Imidazolderivat der Formel 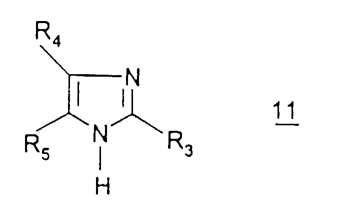 in der R₃, R₄ und R₅ die in Anspruch 1 für (I) angegebenen Bedeutungen haben, mit einem (Biphenyl-4-yl)-methylderivat der Formel 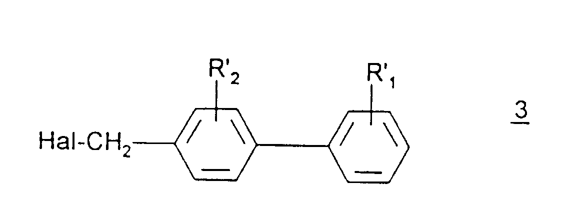 in der Hal ein Halogenatom bedeutet und R'₁ und R'₂ entweder die Bedeutung von R₁ bzw. R₂ haben oder eine Vorstufe für R₁ bzw. R₂ bedeuten, in Gegenwart von Sauerstoff und UV-Strahlung in basischem Milieu umsetzt;
b3) gegebenenfalls die erhaltene Verbindung der Formel 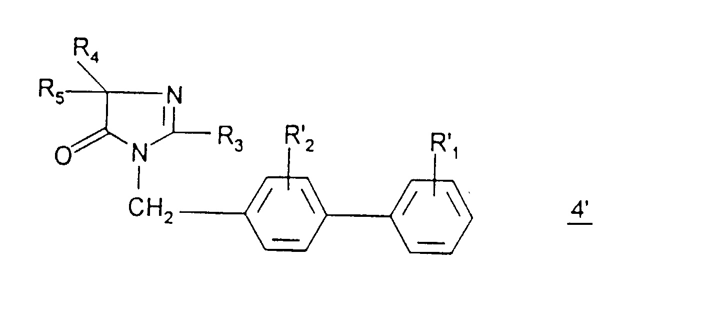 mit dem Lawesson-Reagenz [2,4-Bis-(4-methoxyphenyl)-1,3-dithia-2,4-diphosphetan-2,4-disulfid] behandelt;
c3) die in b3) oder c3) erhaltene Verbindung der Formel 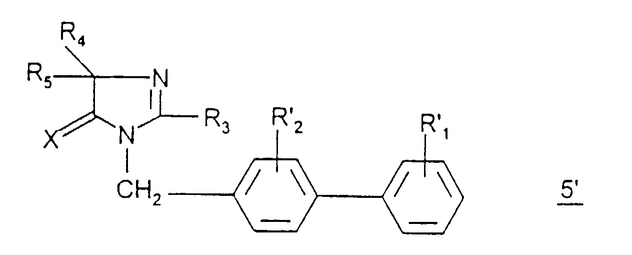 anschließend unter für die Herstellung der Verbindung (I) zweckmäßigen Bedingungen durch Umwandlung der Gruppen R'₁ und/oder R'₂ in die entsprechenden Gruppen R₁ und/oder R₂ behandelt, wobei die Verbindung anschließend gegebenenfalls in eines ihrer Salze übergeführt wird.

11. Verbindung der Formel 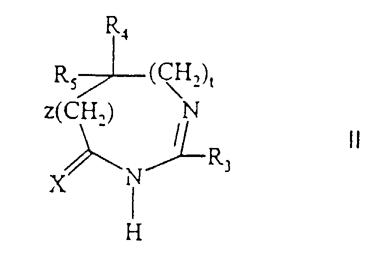 in der:
- R₃ Wasserstoff, unsubstituiertes oder durch ein oder mehr Halogenatome substituiertes C₁-C₆-Alkyl, C₂-C₆-Alkenyl, C₃-C₇-Cycloalkyl, Phenyl, Phenylalkyl mit C₁-C₃-Alkyl oder Phenylalkenyl mit C₂-C₃-Alkenyl bedeutet, wobei die Phenylgruppen unsubstituiert oder ein- oder mehrfach durch ein Halogenatom, C₁-C₄-Alkyl, C₁-C₄-Halogenalkyl, C₁-C₄-Polyhalogenalkyl, Hydroxyl oder C₁-C₄-Alkoxy substituiert sind;
- R₄ und R₅ jeweils unabhängig voneinander C₁-C₆-Alkyl, Phenyl oder Phenylalkyl mit C₁-C₃-Alkyl bedeuten, wobei die Alkyl-, Phenyl- und Phenylalkylgruppen unsubstituiert oder durch ein oder mehr Halogenatome oder durch eine unter C₁-C₄-Perfluoralkyl, Hydroxyl und C₁-C₄-Alkoxy ausgewählte Gruppe substituiert sind;
- oder R₄ und R₅ zusammen eine Gruppe der Formel =CR₇R₈ bilden, in der R₇ Wasserstoff, C₁-C₄-Alkyl oder Phenyl bedeutet und R₈ C₁-C₄-Alkyl oder Phenyl bedeutet;
- oder R₄ und R₅ miteinander zu einer Gruppe der Formel (CH₂)ₙ oder einer Gruppe der Formel (CH₂)ₚY(CH₂)_{q} gebunden sind, in der Y ein Sauerstoffatom, ein Schwefelatom, ein Kohlenstoffatom, das durch eine C₁-C₄-Alkylgruppe, Phenyl oder Phenylalkyl mit C₁-C₃-Alkyl substituiert ist, oder eine N-R₆-Gruppe bedeutet, in der R₆ Wasserstoff, C₁-C₄-Alkyl, Phenylalkyl mit C₁-C₃-Alkyl, C₁-C₄-Alkylcarbonyl, C₁-C₄-Halogenalkylcarbonyl, C₁-C₄-Polyhalogenalkylcarbonyl, Benzoyl, α-Aminoacyl oder eine N-Schutzgruppe bedeutet, oder R₄ und R₅ zusammen mit dem Kohlenstoffatom, an das sie gebunden sind, einen Indan- oder Adamantanrest bilden;
- p + q = m;
- n eine ganze Zahl von 2 bis 11 bedeutet;
- m eine ganze Zahl von 2 bis 5 bedeutet;
- X ein Sauerstoff- oder Schwefelatom bedeutet; und
- z und t 0 bedeuten oder eines dieser Symbole 0 und das andere 1 bedeutet;
mit der Einschränkung, daß
A/ wenn z und t 0 sind und X Sauerstoff bedeutet:
1) R₄ und R₅ eine andere Bedeutung haben als C₁-C₆-Alkyl, Phenyl oder Phenylalkyl mit C₁-C₃-Alkyl, wobei die Alkyl-, Phenyl- und Phenylalkylgruppen unsubstituiert oder durch ein oder mehr Halogenatome oder durch eine unter C₁-C₄-Perfluoralkyl, Hydroxyl und C₁-C₄-Alkoxy ausgewählte Gruppe substituiert sind; oder
2) R₄ und R₅ zusammen eine andere Bedeutung haben als die Gruppe der Formel (CH₂)ₚY(CH₂)_{q}, in der Y eine N-R₆-Gruppe bedeutet, in der R₆ Wasserstoff, C₁-C₄-Alkyl oder Phenylalkyl mit C₁-C₃-Alkyl bedeutet; oder
3) R₄ und R₅ zusammen eine andere Bedeutung haben als die Gruppe =CR₇R₈, wenn R₃ Wassserstoff, Phenyl, Methylphenyl oder Benzyl bedeutet;
4) n einen anderen Wert als 6 hat; oder
5) R₄ und R₅ zusammen eine andere Gruppe als (CH₂)ₙ bedeuten, in der n einen Wert von 3 bis 5 hat, wenn R₃ eine substituierte Phenylgruppe bedeutet;
B/ wenn z = 1 und R₃ Phenyl bedeutet, weisen R₄ und R₅ eine andere Bedeutung als Methyl auf;
C/ wenn z und t 0 sind und X Schwefel bedeutet:
1) R₄ und R₅ haben eine andere Bedeutung als Phenyl, wenn R₃ Methyl, Phenyl, Chlorphenyl, Methoxyphenyl oder Methylphenyl bedeutet; oder
2) R₄ und R₅ haben eine andere Bedeutung als C₁-C₃-Alkyl, wenn R₃ Methyl oder Phenyl bedeutet; oder
3) R₄ und R₅ haben eine andere Bedeutung als Methoxyphenyl oder Chlorphenyl, wenn R₃ Phenyl bedeutet; oder
4) R₄ und R₅ haben eine andere Bedeutung als Chlorphenyl, wenn R₃ Methyl bedeutet.

12. Verbindung nach Anspruch 11 der Formel 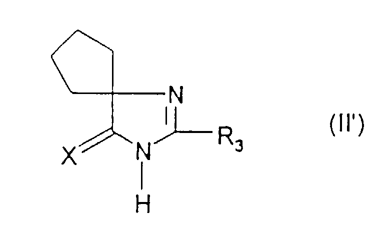 in der X ein Sauerstoff- oder Schwefelatom bedeutet und R₃ Wasserstoff, unsubstituiertes oder durch ein oder mehr Halogenatome substituiertes C₁-C₆-Alkyl, C₂-C₆-Alkenyl, C₃-C₇-Cycloalkyl, Phenyl, Phenylalkyl mit C₁-C₃-Alkyl oder Phenylalkenyl mit C₂-C₃-Alkenyl bedeutet, wobei die Phenylgruppen unsubstituiert oder ein- oder mehrfach durch ein Halogenatom, C₁-C₄-Alkyl, C₁-C₄-Halogenalkyl, C₁-C₄-Polyhalogenalkyl, Hydroxyl oder C₁-C₄-Alkoxy substituiert sind; mit der Bedingung, daß R₃ eine andere Bedeutung als substituiertes Phenyl hat, wenn X Sauerstoff bedeutet.

13. Verbindung nach Anspruch 12, dadurch gekennzeichnet, daß in der Formel II' X Sauerstoff bedeutet und R₃ n-Butyl bedeutet und daß es sich um 2-n-Butyl-4-spirocyclopentan-2-imidazolin-5-on handelt.

14. Hydrochlorid von 2-n-Butyl-4-spirocyclopentan-2-imidazolin-5-on.

15. Verbindung nach Anspruch 11 der Formel 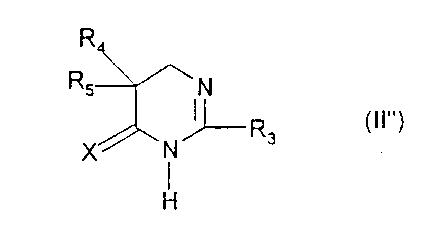 in der R₃, R₄, R₅ und X die vorstehend im Anspruch 11 für (II) angegebenen Bedeutungen aufweisen.

16. Verbindung nach Anspruch 11 der Formel 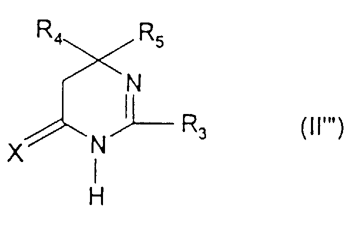 in der
- R₃ Wasserstoff, unsubstituiertes oder durch ein oder mehr Halogenatome substituiertes C₁-C₆-Alkyl, C₂-C₆-Alkenyl, C₃-C₇-Cycloalkyl, Phenyl, Phenylalkyl mit C₁-C₃-Alkyl oder Phenylalkenyl mit C₂-C₃-Alkenyl bedeutet, wobei die Phenylgruppen unsubstituiert oder ein- oder mehrfach durch ein Halogenatom, C₁-C₄-Alkyl, C₁-C₄-Halogenalkyl, C₁-C₄-Polyhalogenalkyl, Hydroxyl oder C₁-C₄-Alkoxy substituiert sind;
- R₄ und R₅ jeweils unabhängig voneinander C₁-C₆-Alkyl, Phenyl oder Phenylalkyl mit C₁-C₃-Alkyl bedeuten, wobei die Alkyl-, Phenyl- und Phenylalkylgruppen unsubstituiert oder durch ein oder mehr Halogenatome oder eine unter C₁-C₄-Perfluoralkyl, Hydroxyl und C₁-C₄-Alkoxy ausgewählte Gruppe substituiert sind;
- oder R₄ und R₅ zusammen eine Gruppe der Formel =CR₇R₈ bilden, in der R₇ Wasserstoff, C₁-C₄-Alkyl oder Phenyl bedeutet und R₈ C₁-C₄-Alkyl oder Phenyl bedeutet;
- oder R₄ und R₅ miteinander zu einer Gruppe der Formel (CH₂)ₙ oder einer Gruppe der Formel (CH₂)ₚY(CH₂)_{q} gebunden sind, in der Y ein Sauerstoffatom, ein Schwefelatom, ein Kohlenstoffatom, das durch eine C₁-C₄-Alkylgruppe, Phenyl oder Phenylalkyl mit C₁-C₃-Alkyl substituiert ist, oder eine N-R₆-Gruppe bedeutet, in der R₆ Wasserstoff, C₁-C₄-Alkyl, Phenylalkyl mit C₁-C₄-Alkyl, C₁-C₄-Alkylcarbonyl, C₁-C₄-Halogenalkylcarbonyl, C₁-C₄-Polyhalogenalkylcarbonyl, Benzoyl, α-Aminoacyl oder eine N-Schutzgruppe bedeutet, oder R₄ und R₅ zusammen mit dem Kohlenstoffatom, an das sie gebunden sind, einen Indan- oder Adamantanrest bilden;
- p + q = m;
- n eine ganze Zahl von 2 bis 11 bedeutet;
- m eine ganze Zahl von 2 bis 5 bedeutet;
- X ein Sauerstoff- oder Schwefelatom bedeutet;
mit der Einschränkung, daß R₃ eine andere Bedeutung als Phenyl hat, wenn R₄ und R₅ jeweils Methyl bedeuten.

17. Verfahren zur Herstellung einer Verbindung nach einem der Ansprüche 11 bis 16, dadurch gekennzeichnet, daß man eine Verbindung der Formel 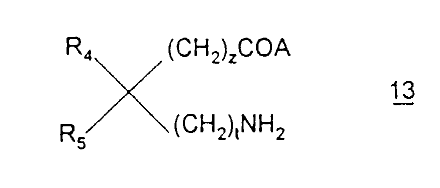 in der R₄ und R₅ die vorstehend im Anspruch 11 für (II) angegebenen Bedeutungen haben, A eine OH-Gruppe, eine NH₂-Gruppe oder eine OR'-Gruppe bedeutet, worin R' Wasserstoff oder einen C₁-C₄-Alkylrest bedeutet, mit einer Verbindung der Formel umsetzt
R₃ - B 14
in der R₃ die in den Ansprüchen 11 bis 16 angegebenen Bedeutungen hat und B
- eine Gruppe der Formel C(OR)₃
- eine Gruppe der Formel 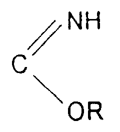 oder
- eine Gruppe der Formel COHal bedeutet,
wobei R C₁-C₄-Alkyl bedeutet und Hal ein Halogenatom und vorzugsweise Chlor bedeutet;
wobei anschließend die auf diese Weise erhaltene Verbindung gegebenenfalls mit dem Lawesson-Reagenz [2,4-Bis-(4-methoxyphenyl)-1,3-dithia-2,4-diphosphetan-2,4-disulfid] umgesetzt wird.

18. Verfahren zur Herstellung einer pharmazeutischen Zusammensetzung, dadurch gekennzeichnet, daß es im Vermischen einer Verbindung nach einem der Ansprüche 1 bis 7 mit einem pharmazeutisch verträglichen Träger besteht.

19. Verfahren zur Herstellung einer pharmazeutischen Zusammensetzung, dadurch gekennzeichnet, daß es im Vereinigen einer Verbindung nach einem der Ansprüche 1 bis 7 mit einer β-Blocker-Verbindung besteht.

20. Verfahren zur Herstellung einer pharmazeutischen Zusammensetzung, dadurch gekennzeichnet, daß es im Vereinigen einer Verbindung nach einem der Ansprüche 1 bis 7 mit einem Diuretikum besteht.

21. Verfahren zur Herstellung einer pharmazeutischen Zusammensetzung, dadurch gekennzeichnet, daß es im Vereinigen einer Verbindung nach einem der Ansprüche 1 bis 7 mit einem Nichtsteroid-Antiphlogistikum besteht.

22. Verfahren zur Herstellung einer pharmazeutischen Zusammensetzung, dadurch gekennzeichnet, daß es im Vereinigen einer Verbindung nach einem der Ansprüche 1 bis 7 mit einem Calciumantagonisten besteht.

23. Verfahren zur Herstellung einer pharmazeutischen Zusammensetzung, dadurch gekennzeichnet, daß es im Vereinigen einer Verbindung nach einem der Ansprüche 1 bis 7 mit einem Tranquilizer besteht.
